(19) Europäisches Patentamt
European Patent Office
Office européen des brevets

(11) EP 4 417 180 A2

(12) **EUROPÄISCHE PATENTANMELDUNG**

(43) Veröffentlichungstag:
**21.08.2024 Patentblatt 2024/34**

(21) Anmeldenummer: **24178368.7**

(22) Anmeldetag: **18.06.2019**

(51) Internationale Patentklassifikation (IPC):
***A61K 6/836*** *(2020.01)*

(52) Gemeinsame Patentklassifikation (CPC):
(C-Sets verfügbar)
**A61K 6/836** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.06.2018 DE 102018114690**

(62) Dokumentnummer(n) der früheren Anmeldung(en) nach Art. 76 EPÜ:
**19733690.2 / 3 810 064**

(71) Anmelder: **VOCO GmbH**
**27472 Cuxhaven (DE)**

(72) Erfinder:
- **Hahn, Christoph**
  **27639 Wurster Nordseeküste (DE)**
- **Lübbe, Gerrit**
  **27476 Cuxhaven (DE)**
- **Maletz, Reinhard**
  **27472 Cuxhaven (DE)**
- **Plaumann, Manfred Thomas**
  **27472 Cuxhaven (DE)**

(74) Vertreter: **Eisenführ Speiser**
**Patentanwälte Rechtsanwälte PartGmbB**
**Postfach 10 60 78**
**28060 Bremen (DE)**

Bemerkungen:
Diese Anmeldung ist am 28-05-2024 als Teilanmeldung zu der unter INID-Code 62 erwähnten Anmeldung eingereicht worden.

(54) **THERMOWIRKSAME DENTALE KOMPOSITZUSAMMENSETZUNG**

(57) Die vorliegende Erfindung betrifft dentale, lichthärtbare, einkomponentige Kompositzusammensetzungen umfassend (A) Monomere, (B) Füllstoffe, und (C) Initiatoren, deren Viskosität $\eta_{20}$ bei 20 °C größer als 400 Pa*s ist, vorzugsweise größer als 800 Pa*s und besonders bevorzugt größer als 1200 Pa*s ist und deren Viskosität $\eta_{50}$ bei 50 °C niedriger als 150 Pa*s ist, vorzugsweise niedriger als 120 Pa*s und besonders bevorzugt niedriger als 90 Pa*s und wobei der Quotient $\eta_{50}$ / $\eta_{20}$ aus der Viskosität der Kompositzusammensetzung bei 50°C und der Viskosität der Kompositzusammensetzung bei 20°C kleiner als 0,125, vorzugsweise kleiner als 0,1 ist. Die Erfindung ist vorzugsweise auf dentale Kompositzusammensetzungen gerichtet, die aus der Gruppe bestehend aus dentalen Füllungsmaterialien, Unterfüllungsmaterialien, Befestigungsmaterialien und Fissurenversiegler ausgewählt sind.

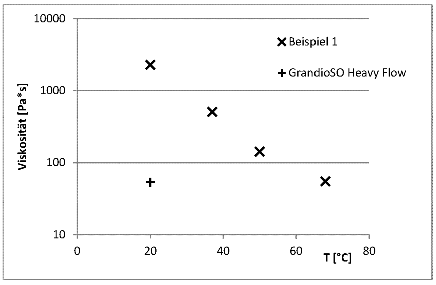


Fig. 1

EP 4 417 180 A2

(52) Gemeinsame Patentklassifikation (CPC): (Forts.)

C-Sets
**A61K 6/887, C08L 33/10**

## Beschreibung

Die vorliegende Erfindung betrifft dentale, lichthärtbare, einkomponentige Kompositzusammensetzungen umfassend (A) Monomere, (B) Füllstoffe, und (C) Initiatoren, deren Viskosität $\eta_{20}$ bei 20 °C größer als 400 Pa*s ist, vorzugsweise größer als 800 Pa*s und besonders bevorzugt größer als 1200 Pa*s ist und deren Viskosität $\eta_{50}$ bei 50 °C niedriger als 150 Pa*s ist, vorzugsweise niedriger als 120 Pa*s und besonders bevorzugt niedriger als 90 Pa*s und wobei der Quotient $\eta_{50}$ / $\eta_{20}$ aus der Viskosität der Kompositzusammensetzung bei 50°C und der Viskosität der Kompositzusammensetzung bei 20°C kleiner als 0,125, vorzugsweise kleiner als 0,1 ist.

Die Erfindung ist vorzugsweise auf dentale Kompositzusammensetzungen gerichtet, die aus der Gruppe bestehend aus dentalen Füllungsmaterialien, Unterfüllungsmaterialien, Befestigungsmaterialien und Fissurenversiegler ausgewählt sind. Die Erfindung betrifft ebenso gehärtete Kompositzusammensetzungen, die durch Lichthärtung der erfindungsgemäßen dentalen, lichthärtbaren, einkomponentigen Kompositzusammensetzungen erhalten werden. Die Erfindung zielt ferner auf dentale, lichthärtbare, einkomponentige Kompositzusammensetzungen zur Anwendung in einem dentalen therapeutischen Verfahren, vorzugsweise als ein auf 40 °C bis 80 °C vorab erwärmtes Dentalmaterial zum Füllen von Zähnen und/oder zum Befestigen von Kronen, Inlays, Onlays und Veneers und/oder zum Versiegeln von Fissuren. Darüberhinaus betrifft die Erfindung die Verwendung der dentalen, lichthärtbaren, einkomponentigen Kompositzusammensetzung zur Herstellung eines dentalen, vorzugsweise auf 40 °C bis 80 °C vorgewärmten, Erzeugnisses. Zudem umfasst die Erfindung ein Verfahren zum Vorbereiten einer dentalen Behandlung. In einer bestimmten Ausführungsform zielt die Erfindung auf eine spezielle Vorrichtung zur Applikation der erfindungsgemäßen Kompositzusammensetzung und auf ein Verfahren zur Herstellung der Kompositzusammensetzung. Die Erfindung ist in den beigefügten Anspüchen definiert.

Dentalkompositmaterialien sind Verbundwerkstoffe aus einem Kunststoff und anorganischen Füllkörpern. Herkömmlicherweise bestehen sie somit grundsätzlich aus verschiedenen Bausteinen: einer polymerisierbaren organischen Matrix, Füllstoffpartikeln und (üblicherweise) einem Mittel, das den Verbund zwischen dem (gehärteten) Polymer und den Füllstoffteilchen sicherstellt. Dentale Kompositmaterialien werden in Form härtbarer Zusammensetzungen eingesetzt, die nach ihrer Applikation polymerisiert werden.

Dentale, restaurative Materialien stellen eine spezielle Form der Kompositmaterialien dar, weil sie aufgrund ihrer extremen physikalischen und chemischen Belastung im überaus feindlichen Milieu des Mundes den höchsten Anforderungen ausgesetzt sind. Aufgrund ihres extremen Anforderungsprofils dienen diese Materialien oft als Basis zur Entwicklung nicht-dentaler Komposite, bzw. als Modell zum Einsatz im nicht-dentalen Bereich.

## Stand der Technik

Dentale, restaurative Kompositmaterialien werden seit fast 60 Jahren u.a. für die Füllungs- und Unterfüllungstherapie, als Befestigungsmaterialien sowie als Fissurenversiegelungsmassen eingesetzt. Dentalkomposite härten nach Einbringen in die Kavität, bzw. nach Aufbringen auf die Zahnoberfläche chemisch und/oder unter Zufuhr externer Energie in einer Polymerisationsreaktion aus.

Die organische polymerisationsfähige Komponente des dentalen Kompositmaterials wird in der Regel in einer radikalischen Reaktion vernetzt und enthält entsprechend ethylenisch ungesättigte, funktionelle Gruppen. Die Monomere und Oligomere umfassen die Mono-, Di- und/oder Polyacrylate und/oder Methacrylate, wie beispielsweise das Diglycidylmethacrylat des Bisphenol A ("Bis-GMA", 2,2-Bis[4-(2-hydroxy-3-methacryloxypropyloxy)phenyl]propan) und das Diurethandi(meth)acrylat aus 2,2,4-Trimethylhexamethylendiisocyanat und 2-Hydroxyethyl(meth)acrylat (UDMA). Wird im Folgenden von (Meth)acrylaten gesprochen, so sind im Kontext dieser Erfindung auch immer die analogen Acrylate gemeint. Kommerziell erhältliche Standardmischungen enthalten Bis-GMA, UDMA sowie Triethylenglykoldimethacrylat zur Absenkung der Viskosität.

Um die Harzmischung radikalisch härten zu können, wird der Masse ein Initiatorsystem beigegeben, das die radikalische Polymerisation beispielsweise nach Bestrahlung auslöst. Ein typisches System, das die radikalische Polymerisation der (Meth)acrylate startet, besteht aus einem Photoinitiator (Keton) und einem Beschleuniger (Amin). Als Keton kann typischerweise Campherchinon, als Amin die para-N,N-Dimethylaminobenzoesäure eingesetzt werden. Weitere photoaktive Bestandteile können der Mischung zugegeben werden. Andere bekannte Photoinitiatoren, die einzeln oder in Kombination mit dem System Campherchinon/Amin verwendet werden, sind Phosphinoxide, insbesondere das Phenyl-bis(2,4,6-trimethylbenzoyl)phosphinoxid und/oder das 2,4,6-Trimethylbenzoyldiphenylphosphinoxid. Ebenfalls ist der Einsatz von Boratsalzen als Photoinitiatoren bekannt. Weitere Photoinitiatoren können aus der Gruppe bestehend aus Benzoinalkylether, Benzoinalkylester, Benzilmonoketalen, Benzophenonen, Acetophenonen, Ketalen, Thioxanthonen und Titanocenen ausgewählt werden.

Wird die Zusammensetzung mit einer geeigneten Strahlungsquelle bei 470 nm belichtet, so vernetzt das Kompositmaterial photochemisch.

Die anorganischen Füllkörper des dentalen Kompositmaterials bestehen im Allgemeinen aus Quarz, Borsilikatglas, Lithiumaluminiumsilikat, Bariumaluminiumsilikat, Strontium-/Bariumglas, Zinkglas, Zirkoniumsilikat, pyrogene oder kolloidale Kieselsäure sowie nanoskaligen Metalloxiden.

Der Verbund der anorganischen Füllkörper mit der organischen Harzmatrix wird in der Regel durch den Einsatz von Kupplungsreagentien oder Haftvermittlern sichergestellt. Dieser Verfahrensschritt ist wesentlich für die spätere Eignung der Kompositmasse als Dentalmaterial. Hierbei wird der Füllstoff, meistens in Gegenwart schwacher Säuren, mit einem Silan behandelt, bevor er mit der flüssigen Harzkomponente vermengt wird.

Weitere Bestandteile eines Kompositwerkstoffes umfassen Farbstoffe, Pigmente, Stabilisatoren, Inhibitoren, Co-Initiatoren, Benetzungsmittel, etc.

Das Eigenschaftsprofil des resultierenden dentalen Verbundwerkstoffes wird in erster Linie von der anorganischen Phase bestimmt. Während der Youngs Modul für ein nichtgefülltes Harzsystem auf Bis-GMA-Basis 2.8 GPa beträgt, weist der Zahnschmelz einen Wert von 83 GPa und das Dentin einen Wert von 19 GPa auf. Durch Zugabe eines konventionellen, silylierten Füllstoffs zum Bis-GMA-Harz kann der Wert von 2.8 GPa deutlich verbessert werden. Wird der Füllstoff im Volumenverhältnis von 1 zu 1.25 dem Harz beigegeben, so kann der Youngs Modul auf einen Wert von 18 GPa angehoben werden. Für ein Verhältnis 1 zu 1 kann ein Wert von 30 GPa erreicht werden.

Füllstofftyp, Menge und Partikelverteilung bestimmen für eine gegebene Harzzusammensetzung die mechanischen, ästhetischen und rheologischen Kennzeichen eines dentalen Füllungskomposits wie Oberflächenhärte, Abriebbeständigkeit, Verschleißfestigkeit, Druckfestigkeit, Zugfestigkeit, Polymerisationsschrumpfung, Frakturresistenz und Wärmewechselbeständigkeit, ferner Polierbarkeit, Glanz, Opazität, Transluzenz und Farbstabilität sowie Fließverhalten, Standfestigkeit und Modellierbarkeit. Als Faustregel gilt: Je höher die Beladung des flüssigen Harzes mit silanisiertem Füllstoff, umso besser die mechanischen, physikalischen und chemischen Eigenschaften des ausgehärteten Formstoffs.

Vor dem Hintergrund der überragenden Bedeutung der anorganischen Phase für die Eigenschaften dentaler Kompositmaterialien läßt sich auch die traditionelle Einteilung dentaler Kompositwerkstoffe in drei verschiedenen Grundklassen verstehen.

Ein makrogefülltes Kompositmaterial ist eine mit relativ großen Partikeln (1-100 μm), hochgefüllte (bis 87 Gew.-%) Zusammensetzung. Während als Füllstoff früher Glaspulver mit mittleren Korngrößen von 30 - 50 μm diente, ist der Füllstoff heute meistens gemahlener Quarz oder auch eine Glaskeramik mit einer mittleren Teilchengröße von 8 - 12 μm. Makrogefüllte Komposite weisen die beste Verschleißfestigkeit auf, lassen sich jedoch aufgrund der Partikelgröße außerordentlich schlecht hochglanzpolieren. Während der Politur brechen die voluminösen Füllstoffpartikel aus der Füllung aus, es bleiben kleine Löcher zurück und die ausgebrochenen Füllstoffsplitter üben einen Schmirgeleffekt auf den restlichen Formstoff aus, so dass sich makrogefüllte Komposite nicht hochglanzpolieren lassen und ein grundsätzliches ästhetisches Defizit aufweisen.

Um der Forderung nach verbesserter Ästhetik nachzukommen, wurde die Gruppe der mikrogefüllten dentalen Kompositmaterialien entwickelt. Charakteristisches Kennzeichen dieser Gruppe ist die außergewöhnlich kleine Partikelgröße der Kompositfüllstoffe, die in erster Linie aus amorpher Kieselsäure bestehen und eine mittlere Teilchengröße von ca. 0.04 μm aufweisen. Diese kleine Partikelgröße bedingt eine extrem große Teilchenoberfläche, die ihrerseits infolge intensiver Wechselwirkungskräfte zwischen den Partikeloberflächen der Füllstoffbeladung des Kompositmaterials eine frühe Grenze setzt. In der Regel können mikrogefüllte Kompositmaterialien nicht über 50 Gew.-% mit Füllstoff versetzt werden, da das Material aufgrund zu hoher Viskosität dann nicht mehr verarbeitbar ist. Diese Kompositklasse ist hochglanzpolierbar, zeigt ausgezeichnete refraktive Eigenschaften und erfüllt alle Kriterien eines äußerst ästhetisch wirkenden dentalen Werkstoffes. Bedingt durch den geringen Füllstoffgehalt zeigen mikrogefüllte Werkstoffe verglichen mit den makrogefüllten dentalen Kompositen jedoch stark reduzierte mechanische Eigenschaften wie Abrieb, Zugfestigkeit, zu hohen Schrumpf, etc.

Durch den Versuch, die Hochglanzpolierbarkeit der mikrogefüllten Verbundwerkstoffe mit den guten mechanischen Eigenschaften der makrogefüllten Komposite zu kombinieren, entwickelte man die Klasse der sogenannten Hybridkomposite. Hierbei ist der eingesetzte Füllstoff eine Mischung aus konventionellem Glas mit einer Partikelgröße von 0.6 - 1.5 μm sowie aus nanoskaligen Teilchen von 0.01 - 0.05 μm. In der Regel beträgt der mengenmäßige Anteil der nanoskaligen Kieselsäureteilchen 7 - 15 Gew.-%. Der Gesamtfüllstoffgehalt kann bis zu 80 Gew.-% betragen. Aufgrund der großen Variation in den Partikelgrößen kann so eine äußerst kompakte Packungsdichte der Füllstoffteilchen erzielt werden, wobei kleinere Partikel in den Zwischenräumen der größeren Partikel zu liegen kommen.

Mit den Hybridkompositen wurden zu Beginn der 80er Jahre erste standfeste Dentalkomposite entwickelt. Ziel war es, eine möglichst hohe Füllstoffmenge in das System einzubringen, um beste mechanische Werte wie Oberflächenhärte, Abriebbeständigkeit, Verschleißfestigkeit, Druckfestigkeit, Zugfestigkeit, Polymerisationsschrumpfung, Frakturresistenz sowie Standfestigkeit und Modellierbarkeit zu erreichen. Die besten heutigen standfesten dentalen Füllungskomposite mit Spitzenwerten in ihren mechanischen Eigenschaften weisen einen Füllstoffanteil von bis zu 92 Gew.-% auf.

Anfang der 90er Jahre wurde alternativ versucht, die dentale Füllungstherapie durch extrem fließfähige Komposite zu verbessern. Im Gegensatz zu den höchstgefüllten standfesten Systemen sollten klinisch exzellente Füllungen

hier durch ein System erreicht werden, das leicht an den Kavitätenrändern anfließt und somit zu einer ausgesprochen starken Adaptation zwischen dem Dentalkomposit und dem Dentin führt. Hiermit sollte die Bildung von Sekundärkaries, die sich an den Randspalten zwischen der natürlichen Zahnhartsubstanz und dem Füllungskomposit bildet, verhindert werden. Die besten heutigen Flow-Systeme weisen einen Füllstoffanteil von ca. 80 Gew.-% auf.

In der dentalen Füllungstherapie gibt es somit zwei gegensätzliche Konzepte, zu optimalen Restaurationen zu gelangen. Einerseits gibt es die standfesten und stopfbaren Komposite, die aufgrund des hohen Füllstoffanteils einen geringen Polymerisationsschrumpf, hohe mechanische Werte der Oberflächenhärte, Abriebbeständigkeit, Verschleiß- und Druckfestigkeit aufweisen und andererseits existieren hoch fließfähige Komposite, die aufgrund ihrer rheologischen Eigenschaften eine ausgesprochen hohe marginale Adaptation an den Kavitätenwänden auszeichnen. So kann ein Zahnarzt heutzutage zunächst ein Flow-Material einsetzen, das eine gute Randspaltdichtigkeit gewährleistet, um dann mit einem standfesten Komposit den Großteil der Kavität zu füllen und so gute mechanische Eigenschaften der Restauration sicherzustellen. Nachteilig an dieser Methode ist der Einsatz zweier Verfahrensschritte mit unterschiedlichen Materialien.

Gegen Ende der 1990er Jahre gab es erste Versuche, durch Voraberwärmung des Komposits beide Konzepte zu vereinigen: Das standfeste, modellierbare Komposit sollte nicht mehr bei Raumtemperatur aus seiner Compule heraus in die Kavität appliziert werden, sondern zunächst in einer Heizeinheit vorgewärmt werden. Durch die Temperaturerhöhung sollte sich die Viskosität des Dentalkomposits verringern, das Ausdrücken des Composits vereinfacht werden und eine ausgezeichnete marginale Adaptation an den Kavitätenwänden stattfinden (J. Friedman, Thermally assisted polymerization of composite resins, Contemp. Esthet. Restor. Pract., 7, 46, 2003 und H.E. Strassler, R.D. Trushkowsky, Predictable restoration of Class 2 preparations with composite resin, Dent. Today, 23, 93 - 99, 2004). Man erkannte schnell, dass das Fließverhalten kommerzieller Komposite durch eine Erwärmung vorab verbessert werden konnte, allerdings waren die Ergebnisse je nach Komposittyp sehr unterschiedlich. Zudem wurde es nicht erreicht, dass die vorgewärmten Komposite eine so ausgeprägt reduzierte Viskosität aufwiesen im Vergleich zu den Flow-Systemen, die bei Raumtemperatur angewendet wurden (J.S. Blalock, R.G. Holmes, F.A. Rueggeberg, Effect of temperature on uncured composite film thickness, J. Prothet. Dent., 96, 424 - 432, 2006). Früh schon gab es auch in der Patentliteratur erste Druckschriften, die das Verfahren einer Voraberwärmung von dentalen Kompositen sowie entsprechende Geräte zur Erwärmung sowie zum Ausbringen der erwärmten Compulen unter Schutz stellten. Genannt seien hier die Patentschriften von J. Friedmann, beispielsweise die US 6,236,020 B1 (Verfahren), US 6,320,162 B1 (Gerät), US 6,312,254 B1 (Dispenser), US 6,616,448 B2 (Dispenser), US 7,015,423 B2 (Gerät) und US 7,097,452 B2 (Compule). Dieses zur Erwärmung dentaler Füllungskomposite nun kommerziell erhältliche Equipment kann vom Zahnarzt chair-side verwendet werden.

Ausgehend von den Patenten von J. Friedman zum Erwärmen dentaler Kompositmaterialien gab es seit Beginn der 2000er Jahre eine Vielzahl wissenschaftlicher Arbeiten zu diesem Thema. Im Vordergrund dieser Untersuchung stand hauptsächlich der Einfluss der erhöhten Temperatur auf die physikalischen Eigenschaften und die klinische Performance der Komposite. Häufig untersucht wurden Parameter wie Biegefestigkeit, E-Modul, Oberflächenhärte, Volumenschrumpfung, Schrumpfspannung und Umsetzungsgrad. Daneben wurden häufig marginale Adaptation und Microleakage sowie die Temperaturauswirkungen auf die Pulpa untersucht.

Ausführliche Untersuchungen gab es bezüglich der Polymerisationskinetik bei Lichthärtung unter den Bedingungen einer moderat erhöhten Temperatur. So fand man, dass der Doppelbindungsumsatz bei Lichthärtung und einer Temperatur von 54,5°C signifikant schneller und höher war im Vergleich zur Lichthärtung bei Raumtemperatur. Entsprechend geringer war das Ausmaß der "Post-Gel Phase". Man schlussfolgerte, dass aufgrund dieser Befunde die Eigenschaften des Polymerisats verbessert sein müssten (M. Trujillo, S.M. Newmann, J.W. Stansburry, Use of near-IR to monitor the influence of external heating on dental composite photopolymerization, Dent. Mater. 20, 766 - 777, 2004).

Andererseits sollte eine erhöhte Netzwerkdichte auch zu einem höheren Polymerisationsschrumpf führen und so einen negativen Einfluss auf die marginale Adaptation des Dentalkomposits ausüben (U. Lohbauer, S. Zinelis, C. Rahiotis, A. Petschelt, G. Eliades, The effect of resin composite pre-heating on monomer conversion and polymerization shrinkage, Dent. Mater. 25, 514 - 519, 2009).

Zusätzlich zu den Untersuchungen zum Polymerisationsschrumpf gab es ergänzende Studien zur Polymerisationsspannung, die mit steigendem Doppelbindungsumsatz ebenfalls zunahm (F.C. Calheiros, M. Daronch, F.A. Rueggeberg, R.R. Braga, Effect of temperature on composite polymerization stress and degree of conversion, Dent. Mater. 30, 613 - 618, 2014).

Die Ergebnisse dieser Studien wurden jedoch kritisch gesehen, da die obigen Untersuchungen alle isotherm durchgeführt wurden, denn diese Voraussetzungen entsprechen nicht der tatsächlichen klinischen Situation, in der der Zahnarzt die vorgewärmte Compule aus dem Temperiergerät entnimmt, in einen Dispenser einsetzt und das Material dann in die präparierte Kavität ausdrückt. Er bearbeitet und konturiert sodann das Material, so dass eine längere Zeit verstreicht, bevor das Komposit gehärtet wird. Es wurde geschätzt, dass ein Kompositmaterial, das auf 60°C erwärmt wurde, vom Zeitpunkt, an dem es aus dem Temperiergerät entnommen wird, innerhalb der nächsten 2 Minuten um 50% abkühlt und nach 5 Minuten 90% seiner Temperatur erniedrigt hat (J.S. Blalock, R.G. Holmes, F.A. Rueggeberg, Effect of temperature on uncured composite film thickness, J. Prothet. Dent., 96, 424 - 432, 2006).

Nachdem das Dentalkomposit appliziert ist, wird möglicherweise sehr schnell ein thermisches Gleichgewicht erreicht. Unter diesen, möglicherweise klinisch eher relevanten, nichtisothermen Verhältnissen wurde der Zusammenhang zwischen der marginalen Adaptation, dem Doppelbindungsumsatz und den mechanischen Eigenschaften ausgehärteter Dentalkomposite ein weiteres Mal untersucht. Man fand diesmal, dass es keinen Unterschied in den mechanischen Eigenschaften und dem Monomerumsatz zwischen vorgewärmten und nicht vorgewärmten Dentalkompositen gibt, dass jedoch eine verbesserte Adaptation des vorgewärmten Komposits an den Wänden der Kavität festgestellt wurde (N.R. Froes-Salgado, L.M. Silva, Y. Kawano, C. Francci, A. Reis, A.D. Loguercio, Composite pre-heating: Effects on marginal adaptation, degree of conversion and mechanical properties, Dent. Mater. 26, 908 - 9015, 2010).

Die Ergebnisse der wissenschaftlichen Untersuchungen sind somit - unter Berücksichtigung der klinischen Situation, die den Bedingungen in der Zahnarztpraxis bei Anwendung der Dentalpräparate weitestgehend entspricht - insgesamt positiv und in sich schlüssig.

Die temperaturabhängige Reduzierung der Viskosität wurde dagegen nur relativ selten und dabei meist nur indirekt untersucht. Diese indirekten Methoden basieren auf der erhöhten Fließfähigkeit des Komposits bei erhöhter Temperatur und damit niedrigerer Viskosität. Dazu wird eine kleine Menge Komposit zwischen zwei Glasplatten durch eine definierte Kraft verpresst. Anschließend wird entweder die Dicke als "Filmdicke" oder der Durchmesser als "Flow" bzw. "Fließfähigkeit" bestimmt. J. Friedman selbst fand eine Reduzierung der Filmdicke von 32% bei einer Temperatur von 60°C im Vergleich zu 22,2°C (EP 1 151 728 B1). J.S. Blalock fand für unterschiedliche Komposite eine Reduzierung der Filmdicke im Bereich von 4% bis 77% bei einer Erwärmung auf 60°C im Vergleich zu 23°C (J.S. Blalock et al., Effect of temperature on unpolymerized composite resin film thickness, J. Prosthet. Dent. 2006, 96 (6), 424-423). J. da Costa fand für unterschiedliche Komposite eine Reduzierung der Filmdicke von bis zu 40% bei einer Erwärmung auf 68°C im Vergleich zu 23°C (J. da Costa et al., Effect of heat on the flow of commercial composites, Am. J. Dent. 2009, 22 (2), 92-96). M. Goulart fand für unterschiedliche Komposite eine Reduzierung der Filmdicke von bis zu 24% bei einer Erwärmung auf 64°C im Vergleich zu 21°C (M. Goulart et al., Effect of pre-heating composites on film thickness, Journal of Research in Dentistry, 2013, 1 (4), 274-280). S. Deb fand für unterschiedliche Komposite eine Erhöhung der Fließfähigkeit von bis zu 55% bei einer Erwärmung auf 60°C im Vergleich zu 22°C (S. Deb et al., Pre-warming of dental composites, Dental Materials 2011, 27, e51-e59).

S. Lucey untersuchte den direkten Einfluss der Erwärmung auf die Viskosität. Es wurde eine Reduzierung der Viskosität von ca. 55% bei einer Erwärmung auf 60°C im Vergleich zu 24°C beobachtet (S. Lucey et al., Effect of preheating on the viscosity and microhardness of a resin composite, Journal of Oral Rehabilitation 2010, 37, 278-282).

Es fällt direkt ins Auge, dass all diese Studien an herkömmlichen Dentalkompositen, die für eine Applikation bei Raumtemperatur vorgesehen sind, durchgeführt wurden und die nicht auf den Vorgang einer Erwärmung vor Applikation speziell hin entwickelt wurden. Im Laufe der letzten Jahre gab es eine Unzahl an Patentschriften zu Temperiergeräten, mit denen Dentalkomposite vorab erwärmt und appliziert werden können. Unseres Wissens gibt es keine Studien oder Untersuchungen, die eigens auf die chemische Zusammensetzung eines Dentalkomposits zielt, das einer Erwärmung vor Applikation unterzogen wird.

Es war somit Aufgabe der Erfindung, ein Dentalkomposit bereitzustellen, das die guten mechanischen Eigenschaften hochgefüllter, somit standfester und modellierbarer Dentalkomposte wie hohe Biegefestigkeit (beispielsweise nach ISO 4049 ca. 130 MPa), hohe Oberflächenhärte (beispielsweise Mikro-Vickershärte von ca. 200 MHV), hohe Abriebbeständigkeit (beispielsweise ACTA-3-Medienabrasion, 200.000 Zyklen ca. 18 $\mu$m), hohe Verschleißfestigkeit, hohe Druckfestigkeit (beispielswesie analog ISO 9917 ca. 440 MPa), hohe Zugfestigkeit und hohe Frakturresistenz sowie geringe Polymerisationsschrumpfung (beispielsweise nach Bonded-Disc-Methode ca. 1,6%) und somit auch geringe Polymerisationsspannung (beispielsweise nach Bioman-Methode ca. 6,7 MPa) aufweist, zudem soll es die guten rheologischen Eigenschaften von Flow-Kompositen zeigen.

Kurz gesagt, die Methode aus dem Stand der Technik, d.h. die Versorgung einer Kavität mit zwei unterschiedlichen Füllungskompositen, einem Flow-Material und einem standfesten Material, soll so vereinfacht werden, dass nunmehr lediglich ein einziges Material zur Kavitätenversorgung ausreicht.

Die vorliegende Erfindung berücksichtigt insbesondere die folgenden Überlegungen:

Zum Erreichen eines hervorragenden Anfließens eines Dentalmaterials an Kavitätenrändern und somit zum Erreichen einer starken Adaptation zwischen dem Dentalmaterial und dem Dentin benötigt der Zahnarzt ein fließfähiges Dentalmaterial, welches bei der Behandlungstemperatur ausreichend niedrigviskos ist, also beispielsweise bei einer Temperatur im Bereich von 50° bis 60°C.

Für die Vorbereitung einer solchen Behandlung benötigt der Zahnarzt ein System aus Dentalmaterial und Applikationsvorrichtung, welches er bei einer niedrigeren (Vorbereitungs-) Temperatur einfach und sicher handhaben kann.

Es war daher eine weitere Aufgabe der vorliegenden Erfindung, ein Dentalmaterial bzw. eine Applikationsvorrichtung umfassend ein solches Dentalmaterial anzugeben, welches bei einer üblichen Vorbereitungstemperatur (üblich ist beispielsweise eine Temperatur von 20°C) sicher und einfach handhabbar ist und gleichzeitig bei einer üblichen Behandlungstemperatur (üblich ist beispielsweise eine Temperatur von 50°C) ein gutes Anfließverhalten und eine starke

Adaptation zwischen Dentalmaterial und Dentin ermöglicht.

Gemäß einem ersten Aspekt der vorliegenden Erfindung wird die gestellte Aufgabe gelöst durch eine dentale, lichthärtbare, einkomponentige Kompositzusammensetzung, wie sie in den Patentansprüchen definiert ist.

Gemäß einem weiteren Aspekt der vorliegenden Erfindung wird die Aufgabe gelöst durch eine Vorrichtung zur Applikation einer Kompositzusammensetzung, wie sie in den beigefügten Patentansprüchen definiert ist.

Die erfindungsgemäße dentale, lichthärtbare, einkomponentige Kompositzusammensetzung umfasst: (A) Monomere, (B) Füllstoffe, und (C) Initiatoren; sie ist dadurch gekennzeichnet, dass

- die Viskosität $\eta_{20}$ der Kompositzusammensetzung bei 20°C größer als 400 Pa*s ist und

- die Viskosität $\eta_{50}$ der Kompositzusammensetzung bei 50°C niedriger als 150 Pa*s ist und

- der Quotient $\eta_{50}$ / $\eta_{20}$ aus der Viskosität der Kompositzusammensetzung bei 50°C und der Viskosität der Kompositzusammensetzung bei 20°C kleiner als 0,125 ist,

Die erfindungsgemäße Vorrichtung zur Applikation einer Kompositzusammensetzung umfasst einen Hohlraum, der zumindest teilweise mit einer Menge einer erfindungsgemäßen Kompositzusammensetzung gefüllt ist
und
eine mit dem Hohlraum verbundene Applikationsspitze mit einer Austrittsöffnung für die Kompositzusammensetzung.

Im Stand der Technik war bislang zur Lösung entsprechender technischer Aufgaben ein anderer Lösungsweg beschritten worden. So vertreibt beispielsweise die VOCO GmbH, Cuxhaven, Deutschland Dentalmaterialien in einer nachlauffreien, nicht tropfenden Spritze, die mit einer speziellen Technologie ausgestattet ist (NDT-Technologie; NDT = Non-Dripping-Technology). Entsprechende Spritzen sind im Dokument EP 2 016 962 B offenbart. NDT-Spritzen und vergleichbare Spritzen anderer Hersteller lösen die technischen Probleme, die mit dem Einsatz dünnflüssiger Materialien einhergehen, auf technisch-ingenieurmäßigem Wege durch Ausgestaltung der Applikationsvorrichtung. Die vorliegende Erfindung wählt einen diametral anderen Lösungsansatz und konzentriert sich auf eine rheologisch vorteilhafte Ausgestaltung der einzusetzenden Kompositzusammensetzungen. Im vorliegenden Text wird detailliert dargelegt, wie der Fachmann unter Verwendung üblicher Bestandteile zu einer dentalen Kompositzusammensetzung gelangt, welche die erfindungsgemäßen unterschiedlichen Maßgaben hinsichtlich der Viskosität bei einerseits 20°C und andererseits 50°C erreicht. Die erfindungsgemäßen Kompositzusammensetzungen tragen durch ihre Eigenschaften dazu bei, dass bei einer Vorbereitungstemperatur von 20°C die Gefahr von Arbeitsplatzkontaminationen vermindert bzw. verhindert wird (vergleiche die Diskussion in EP 2 016 962 B). Insbesondere tragen die erfindungsgemäßen Kompositzusammensetzungen dazu bei, dass bei einer Verarbeitungstemperatur von 20°C bei Verwendung üblicher Applikationsvorrichtungen eine vorzeitige Abgabe der Kompositzusammensetzung erschwert wird, im Vergleich mit handelsüblichen, bereits bei 20°C besonders niedrigviskosen Kompositzusammensetzungen.

Erst durch das Erwärmen einer erfindungsgemäßen Kompositzusammensetzung von der Vorbereitungstemperatur (insbesondere 20°C) auf die Behandlungstemperatur (insbesondere 50°C) wird die Viskosität soweit reduziert, dass das Ausbringen der Kompositzusammensetzung aus einer Applikationsvorrichtung besonders leicht erfolgt und gleichzeitig die medizinisch-chemischen Vorgaben erreicht werden (Anfließverhalten; Adaptation). Bei der Behandlungstemperatur (insbesondere 50°C) wird die Applikationsvorrichtung regelmäßig von einem Zahnarzt verwendet, der besonders darin geschult ist, derartige Applikationsvorrichtungen (mit ihrem dann niedrigviskosen Inhalt) ruhig und sicher einzusetzen. Bei der Vorbereitungstemperatur (insbesondere 20°C) kann die erfindungsgemäße Applikationsvorrichtung (mit der darin befindlichen Kompositzusammensetzung) auch von weniger geschultem Personal (wie z. B. einer Zahnarzthelferin) benutzt werden, da das Risiko einer vorzeitigen Materialabgabe reduziert ist.

Eine erfindungsgemäße dentale, lichthärtbare, einkomponentige Kompositzusammensetzung weist vorzugsweise eine Viskosität $\eta_{20}$ bei 20°C von größer als 800 Pa*s, besonders bevorzugt von größer als 1200 Pa*s auf und/oder eine Viskosität $\eta_{50}$ bei 50°C von niedriger als 120 Pa*s, besonders bevorzugt vonniedriger als 90 Pa*s ist und/oder einen Quotient $\eta_{50}$ / $\eta_{20}$ aus der Viskosität der Kompositzusammensetzung bei 50°C und der Viskosität der Kompositzusammensetzung bei 20°C kleiner als 0,1 auf.

Eine erfindungsgemäße dentale, lichthärtbare, einkomponentige Kompositzusammensetzung weist vorzugsweise eine Viskosität $\eta_{37}$ bei 37°C von größer als 400 Pa*s auf. Solche Kompsitzusammensetzungen sind bei Intraoraltemperatur gut modellierbar.

Die Ausführungen zu den Viskositäten der erfindungsgemäßen, dentalen, lichthärtbaren, einkomponentigen Kompositzusammensetzungen treffen analog auf die erfindungsgemäßen Verwendungen der Kompositzusammensetzungen sowie die erfindungsgemäßen Verfahren zu. Insbesondere sind bevorzugte Viskositäten (bzw. deren Quotienten) von erfindungsgemäßen, dentalen, lichthärtbaren, einkomponentigen Kompositzusammensetzungen auch bevorzugte Viskositäten (bzw. deren Quotienten) bei der erfindungsgemäßen Verwendung dieser Kompsitzusammensetzungen

sowie in den erfindungsgemäßen Verfahren und umgekehrt.

Zur Festlegung der Grenzwerte für die Viskositäten siehe Abbildung 1 sowie weiter unten.

Insbesondere wird die Aufgabe gelöst durch eine dentale, lichthärtbare, einkomponentige Kompositzusammensetzung vorzugsweise zur Herstellung eines dentalen Füllungsmaterials, Unterfüllungsmaterials, Befestigungsmaterials oder Fissurenversieglers, umfassend:

(A) Monomere in einer Menge von 6 bis 35 Gew.-%, bezogen auf die Kompositzusammensetzung, vorzugsweise 10 bis 35 Gew.-%, besonders bevorzugt 10 bis 25 Gew.-%,
(B) Füllstoffe in einer Menge von 65 bis 93 Gew.-%, vorzugsweise 65 bis 89 Gew.-%, besonders bevorzugt 75 bis 89 Gew.-%, bezogen auf die Kompositzusammensetzung,
(C) Initiatoren in einer Menge von 0,001 bis 3 Gew.-%, bezogen auf die Menge der Kompositzusammensetzung,
(D) weitere Additive in einer Menge von 0,001 bis 5 Gew.-%, bezogen auf die Menge der Kompositzusammensetzung.

Die Ausführungen zu den erfindungsgemäß einsetzbaren Monomere (A) treffen sowohl auf den Einsatz der Monomere in den erfindungsgemäßen, dentalen, lichthärtbaren, einkomponentigen Kompositzusammensetzungen als auch auf den Einsatz in den erfindungsgemäßen Verfahren zu. Insbesondere sind bevorzugt in erfindungsgemäßen, dentalen, lichthärtbaren, einkomponentigen Kompositzusammensetzungen einsetzbare Monomere auch bevorzugt einsetzbar in den erfindungsgemäßen Verfahren und umgekehrt.

In einer besonderen Ausführungsform umfasst Bestandteil (A) der dentalen, lichthärtbaren, einkomponentigen Kompositzusammensetzung die Mischung von zumindest

(A-i) einer ersten Monomerensubstanz und
(A-ii) einer zweiten Monomerensubstanz,
wobei

- die Viskosität $\eta_{20}$ der zweiten Monomerensubstanz (A-ii) bei 20 °C größer ist als 100 Pa*s,
- die Viskosität $\eta_{20}$ der ersten Monomerensubstanz (A-i) bei 20 °C größer ist als 100 mPa*s,
- - die Viskosität der zweiten Monomerensubstanz (A-ii) bei 20 °C größer ist als die der ersten Monomerensubstanz (A-i) und
- das Massenverhältnis der ersten Monomerensubstanz (A-i) zur zweiten Monomerensubstanz (A-ii) im Bereich von 2:1 bis 1:10 liegt,
- wobei die zweite Monomerensubstanz (A-ii) vorzugsweise zumindest 40 Gew.-% 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI) enthält, wobei die Gew.-%-Angabe auf die Gesamtmasse der Monomere (A) bezogen ist.

Die Untersuchungen, welche die Erfinder im Rahmen der vorliegenden Erfindung durchgeführt haben, zeigen, dass bei Einsatz der Bestandteile (A) bis (D) in den angegebenen Mengen (vorzugsweise in den als bevorzugt angegebenen Mengenbereichen) besonders effizient die für die Erfindung charakteristischen Viskositätsmaßgaben eingestellt werden können. Der Fachmann wird sich bei der Konzeption erfindungsgemäßer Komposizusammensetzungen an den weiter unten angegebenen Beispielen **1 - 34** orientieren und - falls gewünscht - Variationen durchführen und dabei die Maßgaben betreffend die Viskosität bei 20°C bzw. 50°C beachten.

In den folgenden Ausführungsformen und Beispielen entspricht

(A1) den lichthärtbaren bi- oder tricyclischen Verbindungen $Q(Y_xZ_e)_b$,
(A2) dem Bis-GMA (2,2-Bis[4-(2-hydroxy-3-methacryloyloxypropoxy)phenyl]propan) und/oder den lichthärtbaren Derivaten des MDI (Diisocyanatodiphenylmethans) und/oder den lichthärtbare Derivate des TMXDI,
(A3) den lichthärtbaren Monomeren, die eine, zwei oder mehr ethylenische Gruppen aufweisende Substanzen sind, wie beispielsweise, ohne darauf beschränkt zu sein, die in der Dentalchemie üblicherweise eingesetzten (Meth)acrylatmonomere und die nicht (A1) und (A2) zuzuordnen sind,
(A4) den lichthärtbaren bi- oder tricyclischen Verbindungen $Q(Y_xZ_e)_b$, dem 7,7,9-Trimethyl-3,14-dioxa-4,13-dioxo-5,12-diazahexadecan-1,16-dioxydi(meth)acrylat (UDMA), dem 7,9,9-Trimethyl-3,14-dioxa-4,13-dioxo-5,12-diazahexadecan-1,16-dioxydi(meth)acrylat, dem 7,9-Dimethyl-3,14-dioxa-4,13-dioxo-5,12-diazahexadecan-1,16-dioxydi(meth)acrylat, dem 3,14-Dioxa-4,13-dioxo-5,12-diazahexadecan-1,16-dioxydi(meth)acrylat, dem 1,5,5-Trimethyl-1-[(2-methacryloyloxyethyl)carbamoylmethyl]-3-(2-methacryloyloxyethyl)carbamoylcyclohexan, dem 7,7,9,9-Tetramethyl-3,14-dioxa-4,13-dioxo-5,12-diazahexadecan-1,16-dioxydi(meth)acrylat, dem 2,7,7,9,15-Pentamethyl-3,14-dioxa-4,13-dioxo-5,12-diazahexadecan-1,16-dioxydi(meth)acrylat, dem 2,7,9,9,15-Pentamethyl-3,14-dioxa-4,13-dioxo-5,12-diazahexadecan-1,16-dioxydi(meth)acrylat, dem 2,7,9,15-Tetramethyl-3,14-dioxa-4,13-dioxo-5,12-diazahexadecan-1,16-dioxydi(meth)acrylat, dem 2,15-Dimethyl-3,14-dioxa-4,13-dioxo-5,12-diazahexadecan-1,16-dioxydi(meth)acrylat, dem 1,5,5-Trimethyl-1-[(1-methacryloyloxypropan-2-yl)carbamoylmethyl]-3-(1-methacryloyloxypropan-2-yl)carbamoylcyclohexan, dem 2,7,7,9,9,15-Hexamethyl-3,14-dioxa-4,13-dioxo-5,12-diazahexadecan-1,16-dioxydi(meth)acrylat, dem BisEMA (alkoxyliertes Bisphenol-A-di(meth)acrylat mit n = 2 - 6), den hydroxylgruppenhaltigen Poly(meth)acrylaten, den alkoxylierten hydroxylgruppenhaltigen Poly(meth)acrylaten und den lichthärtbaren kettenförmigen und/oder ringförmigen und/oder käfigförmigen Polysiloxanen,
(A5) den lichthärtbaren Monomeren (A3) ohne (A4).

In einer bevorzugten Ausgestaltung bestehen die Monomere (A) aus

(A1) 10 bis 60 Gew.-%, vorzugsweise 20 bis 50 Gew.-%, besonders bevorzugt 25 bis 40 Gew.-%, lichthärtbare bi- oder tricyclische Verbindungen $Q(Y_xZ_e)_b$, wobei gilt

Q bedeutet ein gesättigtes oder olefinisch ungesättigtes bi- oder tricyclisches Strukturelement, jeder Index b ist eine natürliche Zahl, ausgewählt aus der Gruppe der natürlichen Zahlen 1, 2, und 3,
jedes Z bedeutet eine lichthärtbare Gruppe,
jeder Index e ist eine natürliche Zahl, ausgewählt aus der Gruppe der natürlichen Zahlen 1, 2 und 3,
jedes Y bedeutet in der Struktur $Q(Y_xZ_e)_b$ bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und
jeder Index x ist 0 oder 1,

(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),
(A3) 0 bis 15 Gew.-%, vorzugsweise 0 bis 10 Gew.-%, besonders bevorzugt 0 bis 5 Gew.-% weitere radikalisch polymerisierbare Monomere, die nicht (A1) oder (A2) zuzuordnen sind, wobei die Gew.-%-Angaben von (A1), (A2) und (A3) auf die Gesamtmasse der Monomere (A) bezogen sind.

Die vorliegende Erfindung betrifft auch eine dentale, lichthärtbare, einkomponentige Kompositzusammensetzung umfassend:

(A) Monomere

(B) Füllstoffe und

(C) Initiatoren, wobei

die Monomere (A) bestehen aus

(A1) 10 bis 60 Gew.-%, vorzugsweise 20 bis 50 Gew.-%, besonders bevorzugt 25 bis 40 Gew.-%, lichthärtbare bi- oder tricyclische Verbindungen $Q(Y_xZ_e)_b$, wobei gilt

Q bedeutet ein gesättigtes oder olefinisch ungesättigtes bi- oder tricyclisches Strukturelement, jeder Index b ist eine natürliche Zahl, ausgewählt aus der Gruppe der natürlichen Zahlen 1, 2, und 3,
jedes Z bedeutet eine lichthärtbare Gruppe,
jeder Index e ist eine natürliche Zahl, ausgewählt aus der Gruppe der natürlichen Zahlen 1, 2 und 3,
jedes Y bedeutet in der Struktur $Q(Y_xZ_e)_b$ bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und
jeder Index x ist 0 oder 1,

(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),
(A3) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A1) oder (A2) zuzuordnen sind, wobei die Gew.-%-Angaben von (A1), (A2) und (A3) auf die Gesamtmasse der Monomere (A) bezogen sind.

(A1) umfasst die folgenden aliphatischen Bi- oder Tricyclen, deren unsubstituierte Strukturen beispielhaft die folgenden sind:

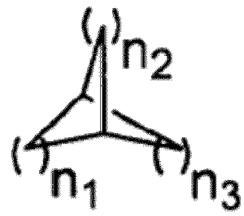

wobei $n_1$, $n_2$ und $n_3$ jeweils unabhängig voneinander eine natürliche Zahl von 1 bis 8 bedeuten können, vorzugsweise eine natürliche Zahl von 1 bis 4.

Exemplarisch genannt seien hier:

| | |
|---|---|
| Für $n_1 = n_2 = 1$; $n_3 = 2$ | Bicyclo[2.1.1]hexan |
| für $n_1 = 1$; $n_2 = n_3 = 2$ | Bicyclo[2.2.1]heptan |
| für $n_1 = n_2 = 1$; $n_3 = 3$ | Bicyclo[3.1.1]heptan |
| für $n_1 = n_2 = n_3 = 2$ | Bicyclo[2.2.2]octan |
| für $n_1 = n_2 = 1$; $n_3 = 4$ | Bicyclo[4.1.1]octan |
| für $n_1 = 1$; $n_2 = 2$; $n_3 = 3$ | Bicyclo[3.2.1]octan |
| für $n_1 = 1$; $n_2 = 2$; $n_3 = 4$ | Bicyclo[4.2.1]nonan |
| für $n_1 = n_2 = 2$; $n_3 = 4$ | Bicyclo[4.2.2]decan |

Nachfolgend werden exemplarisch einige disubstituierte Bicyclen gezeigt:

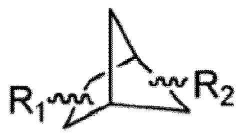
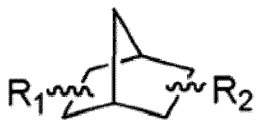
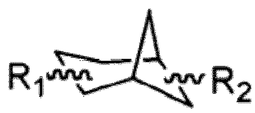
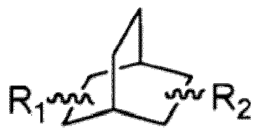
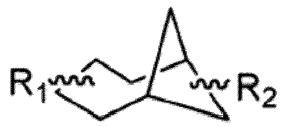
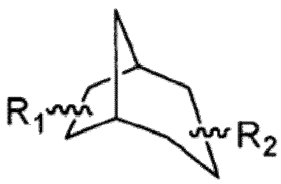
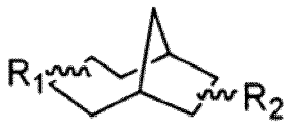
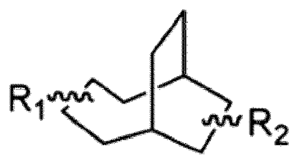

wobei R1 und R2 jeweils die sonstigen Reste der Verbindung bedeuten.

Beispiele für bicyclische Strukturelemente sind das Bicyclo[1.1.1]pentan-, das Bicyclo[2.1.1]hexan-, das Bicyclo[2.2.1]heptan-, das Bicyclo[3.1.1]heptan-, das Bicyclo[2.2.2]octan-, das Bicyclo[4.1.1]octan-, das Bicyclo[3.2.1]octan-, das Bicyclo[4.2.1]nonan-, das Bicyclo[3.3.1]nonan-, das Bicyclo[5.1.1]nonan-, das Bicyclo[3.2.2]nonan-, das Bicyclo[6.1.1]decan-, das Bicyclo[5.2.1]decan-, das Bicyclo[4.2.2]decan-, das Bicyclo[3.3.2]decan-, das Bicyclo[7.1.1]undecan-, das Bicyclo[6.2.1]undecan-, das Bicyclo[5.2.2]undecan-, das Bicyclo[4.3.2]undecan-, das Bicyclo[3.3.3]undecan-, das Bicyclo[8.1.1]dodecan-, das Bicyclo[7.2.1]dodecan-, das Bicyclo[6.2.2]dodecan-, das Bicyclo[5.3.2]dodecan-, das Bicyclo[4.3.3]dodecan-, das Bicyclo[4.4.2]dodecan-, das Bicyclo[5.4.1]dodecanstrukturelement sowie noch höhere Strukturelemente wie die entsprechenden Tridecane, Tetradecane, Pentadecane, etc.

Für unsubstituierte Tricyclen sind z.B. folgende Strukturen möglich:

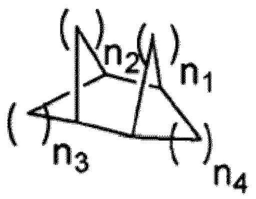

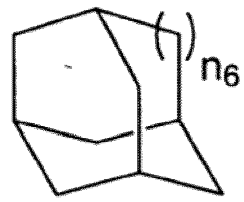

wobei $n_1$, $n_2$, $n_3$, $n_4$ bzw. $n_6$ jeweils unabhängig voneinander eine natürliche Zahl von 0 bis 5 bedeuten können.

Exemplarisch genannt seien:

Für $n_1 = 2$; $n_2 = 0$; $n_3 = 2$; $n_4 = 3$ Tricyclo[4.3.2.0$^{2,5}$]undecan
für $n_1 = 0$; $n_2 = 1$; $n_3 = 2$; $n_4 = 3$ Tricyclo[5.2.1.0$^{2,6}$]decan
für $n_1 = 0$; $n_2 = 2$; $n_3 = 2$; $n_4 = 3$ Tricyclo[5.2.2.0$^{2,6}$]undecan
für $n_1 = 2$; $n_2 = 0$; $n_3 = 2$; $n_4 = 2$ Tricyclo[4.2.2.0$^{2,5}$]decan
für $n_6 = 1$ Tricyclo[3.3.1.1$^{3,7}$]decan

Nachfolgend werden exemplarisch einige di- oder trisubstituierte Tricyclen gezeigt:

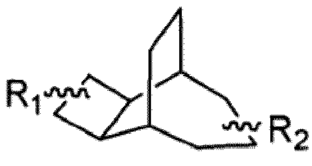

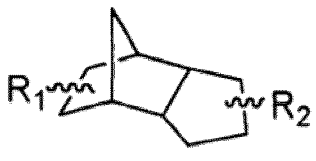

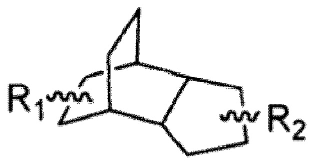

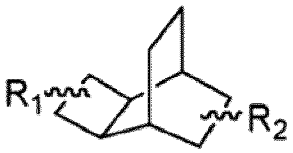

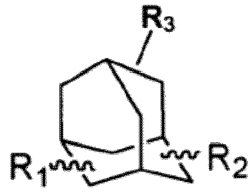

wobei R1, R2 und R3 jeweils die sonstigen Reste der Verbindung bedeuten.

Beispiele für tricyclische Strukturelemente sind das Tricyclo[3.2.1.0$^{2,6}$]octan-, das Tricyclo[4.2.1.0$^{2,6}$]nonan-, das Tricyclo[5.2.1.0$^{2,6}$]decan-, das Tricyclo[6.2.1.0$^{2,6}$]undecan-, das Tricyclo[7.2.1.0$^{2,6}$]dodecan-, oder das Tricyclo[4.2.1.1$^{2,5}$]decan-, das Tricyclo[4.3.1.1 $^{2,5}$]decan-, das Tricyclo[4.4.1.1$^{2,5}$]decan-, das Tricyclo[2.2.1.0$^{2,6}$]heptan-, das Tricyclo[2.2.2.0$^{2,6}$]octan-, das Tricyclo[3.2.2.0$^{2,6}$]nonan-, das Tricyclo[3.3.1.1$^{3,7}$]decan-, das Tricyclo[3.2.1.1$^{3,7}$]nonan-, das Tricyclo[4.2.2.2$^{2,5}$]dodecan-, das Tricyclo[4.3.2.2$^{2,5}$]tridecan-, das Tricyclo[4.4.2.2$^{2,5}$]tetradecan-, das Tricyclo[4.2.1.0$^{3,7}$]nonan-, das Tricyclo[4.4.1.1$^{1,5}$]dodecan-,
das Tricyclo[6.2.1.0$^{2,7}$]undecan-, das Tricyclo[5.2.2.0$^{2,6}$]undecan, das Tricyclo[6.2.2.0$^{2,7}$]dodecan-, Tricyclo[4.3.2.0$^{2,5}$]undecan-, das Tricyclo[4.2.2.0$^{2,5}$]decan- oder das Tricyclo[5.5.1.0$^{3,11}$]tridecanstrukturelement.

In einer bevorzugten Ausführungsform leitet sich die Struktur des polyalicyclischen Strukturelements von einem bicyclischen [a.c.d] Kohlenwasserstoff ab. Die Buchstaben a, c und d sind natürliche Zahlen und haben die Bedeutung der IUPAC-Nomenklatur. Die Summe von a, c und d liegt vorzugsweise im Bereich von 3 bis 13, bevorzugt im Bereich von 4 bis 7.

In einer weiteren bevorzugten Ausführungsform leitet sich die Struktur des polyalicyclischen Strukturelements von einem tricyclischen [a.c.d.f]-Kohlenwasserstoff ab. Die Summe von a, c, d und f liegt vorzugsweise im Bereich von 6 bis 12, bevorzugt im Bereich von 7 bis 9.

In einer bevorzugten Ausführungsform leitet sich die Struktur des polyalicyclischen Strukturelements von einem tricyclischen [a.2.1.0$^{2,(a+1)}$] Kohlenwasserstoff ab, wobei a jeweils die Zahl 3, 4, 5, 6 oder 7 bedeuten kann.

In einer weiteren bevorzugten Ausführungsform leitet sich die Struktur des polyalicyclischen Strukturelements von einem tricyclischen [ä.2.2.0$^{2,(a+1)}$] Kohlenwasserstoff ab, wobei a jeweils die Zahl 3, 4, 5, 6 oder 7 bedeuten kann.

In einer weiteren bevorzugten Ausführungsform leitet sich die Struktur des polyalicyclischen Strukturelements von einem tricyclischen [a.3.1.1] Kohlenwasserstoff ab, wobei a jeweils die Zahl 3, 4, 5, 6 oder 7 bedeuten kann.

Die lichthärtbare Gruppe Z bedeutet ein Strukturelement, das ausgewählt ist aus der Gruppe bestehend aus $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$ und -O-

$CH=CH_2$, wobei die (Meth)acrylate bevorzugt sind.

Das Verbindungselement Y bedeutet eine gerade oder verzweigte Alkylengruppe, wobei die Alkylengruppe durch Sauerstoffatome unterbrochen sein kann und dann Ether und/oder Polyalkylenglykole bildet.

Um zu den lichthärtbaren bi- oder tricyclischen Monomeren (A1) zu gelangen, geht man vorzugsweise von den entsprechenden, oben benannten, entsprechend alkoholsubstituierten polyalicyclischen Kohlenwasserstoffen, beziehungsweise von ihren alkoxylierten Varianten aus, die durch einfache Umsetzung mit (Meth)acrylsäure zu den Monomeren (A1) verestert werden.

Vorzugsweise verwendet man zur Veresterung die kommerziell erhältlichen Verbindungen Bicyclo[2.2.1]heptan-2,7-diol, Bis(hydroxymethyl)bicyclo[2.2.1]heptan, [5-(Hydroxymethyl)-6-bicyclo[2.2.1]hept-2-enyl]methanol, Tricyclo[3.3.1.1$^{3,7}$]decan-1,3-diethanol, [6-(Hydroxymethyl)-6-bicyclo[2.2.1]hept-2-enyl]methanol, Tricyclo[3.3.1.1$^{3,7}$]decan-1,3-diol, Bis(hydroxymethyl)tricyclo[5.2.1.0$^{2,6}$]decan, sowie die jeweils entsprechenden alkoxylierten Varianten, wobei das Bis(hydroxymethyl)tricyclo[5.2.1.0$^{2,6}$]decan und seine alkoxylierte Variante sowie das Bis(hydroxymethyl)bicyclo[2.2.1]heptan und seine alkoxylierte Variante am meisten bevorzugt sind.

Am Beispiel der am meisten bevorzugten Varianten werden die Synthesen der Ausgangssubstanzen beispielhaft im Detail erläutert:

Bis(hydroxymethyl)tricyclo[5.2.1.0$^{2,6}$]decan ist im Handel zu kaufen und kann beispielsweise als Dicidolgemisch der isomeren Verbindungen 3,8-Bis(hydroxymethyl)-tricyclo[5.2.1.0$^{2,6}$]decan und 4,8-Bis(hydroxymethyl)tricyclo[5.2.1.0$^{2,6}$]decan sowie 3,9-Bis(hydroxymethyl)tricyclo[5.2.1.0$^{2,6}$]decan und 4,9-Bis(hydroxymethyl)tricyclo[5.2.1.0$^{2,6}$]-decan bezogen werden.

Die Bis(hydroxymethyl)tricyclo[5.2.1.0$^{2,6}$]decane können aber auch, ausgehend von Dicycopentadien (Tricyclo[5.2.1.0$^{2,6}$]deca-3,8-dien), einfach synthetisiert werden. Dicyclopentadien ist präparativ ohne großen Aufwand durch Dimerisierung in einer Diels-Alder Reaktion zugänglich. Hydroformylierung von Dicyclopentadien ergibt dann das Bis(hydroxymethyl)tricyclo[5.2.1.0$^{2,6}$]decan. Je nach Synthesepfad können gezielt an unterschiedlichen Positionen substituierte Bis(hydroxymethyl)tricyclo[5.2.1.0$^{2,6}$]decane erhalten werden. So werden in den Druckschriften JP 7-206740, EP 1 112 995 B1 oder EP 0 049 631 B1 Vorschriften angegeben, wie das 8,9-Bis(hydroxymethyl)tricyclo[5.2.1.0$^{2,6}$]decan herstellbar ist. Die DE 103 52 260 B3 beschreibt dagegen Verfahren zur Herstellung von 3(4), 8(9)- Bis(hydroxymethyl)tricyclo[5.2.1.0$^{2,6}$]decan. Die Notation der Positionen der Hydroxymethylgruppen 3(4) und 8(9) bedeutet, 3 oder 4, 8 oder 9.

Das im Handel erhältliche und als Ausgangsverbindung zur Herstellung der am meisten bevorzugten Monomere (A1) einsetzbare 3(4),8(9)-Bis(hydroxymethyl)tricyclo[5.2.1.0$^{2,6}$]decan enthält somit Hydroxymethylgruppen sowohl an den Positionen 3 oder 4 als auch in den Stellungen 8 oder 9. Es ist nun möglich, durch Anlagerung von Alkoxiden, im Allgemeinen in Mengen von 1 bis 10 mol, insbesondere von Ethylenoxid, Propylenoxid, Butylenoxid, etc. in Gegenwart basischer Katalysatoren nach bekannten Verfahren die entsprechenden Polyetherpolyole zu synthetisieren. Die EP 0 023 686 B1 enthält hierzu genaue Herstellvorschriften.

Das 2,5(2,6)-Bis(hydroxymethyl)bicyclo[2.2.1]heptan ist kommerziell erhältlich oder kann aus Norbornadien (herstellbar aus Cyclopentadien und Ethin) durch Hydroformylierung und anschließender Reduktion des Diformylnorbornans zum Norbornandiol erhalten werden. Die Hydroformylierung kann sowohl klassisch als auch nicht-klassisch erfolgen.

Im klassischen Verfahren wird Norbornadien in einem organischen Lösungsmittel, beispielsweise Toluol, in einem Autoklaven unter Druck (100 atm) und hohen Temperaturen (100°C) in Gegenwart eines Katalysators mit dem Synthesegas $CO/H_2$ (1:1) umgesetzt. Nach einer Reaktionszeit von 90 Minuten kann kein Substrat mehr nachgewiesen werden und die Umsetzung ist mit Bildung der Dialdehyde mit hoher Selektivität abgeschlossen. Als Hauptkomponenten werden die zwei Isomere des exo-exo-Dialdehyds gebildet. Als Katalysatoren werden $[Pt(C_2H_4)(dppb)]/CH_3SO_3H$ verwendet. Die Abkürzung "dppb" bedeutet 1,4-Bis(diphenylphosphino)butan. Eine genaue Herstellvorschrift ist im Journal of Organometallic Chemistry, 447, 153 -157, 1993 in einer Arbeit mit dem Titel "Hydroformylation of norbornene and 2,5-norbornadiene catalysed by platinum-(0)-alkene compexes in the presence of methanesulfonic acid: determination oft he stereochemistry of the reaction" angegeben. Als Katalysatormetalle können auch Cobalt und Rhodium in Form ihrer Hydridocarbonylspezies $(HM(CO)_4)$, wie beispielsweise auf Basis von Hydridocobalttetracarbonyl $(HCo(CO)_4)$ eingesetzt werden.

Im nicht-klassischen Verfahren, d.h. in überkritischem Kohlendioxid, reagieren Wasserstoff und Kohlenmonoxid bei der katalytischen Umwandlung der Olefine zu den Aldehyden unter weit weniger drastischen Bedingungen im Vergleich zum klassischen Verfahren. Bei nur 20 bar und 100°C in Gegenwart von Rh/4-$H^2F^6$-TPP verläuft die Reaktion innerhalb von 30 Minuten fast quantitativ mit einem Anteil von 95% an Dialdehyden. Bei der Rhodium katalysierten Hydroformylierung in überkritischem Kohlendioxid wird zur Erhöhung der Löslichkeit des Rh-Katalysators in $CO_2$ der Triphenylphosphan Ligand mit Perfluoralkylgruppen derivatisiert, wobei der elektronische Einfluss auf das Metallzentrum mittels zweier $CH_2$-Gruppen, sogenannter spacer, auf ein Minimum reduziert ist. Das Akronym 4-$H^2F^6$-TPP bedeutet somit, dass in Position 4, also in para-Stellung des aromatischen Rings (vom P-Atom aus gesehen) zunächst 2 $CH_2$-Gruppenanschließen, gefolgt von 6 $CF_2$-Gruppen. Genaue Herstellvorschriften befinden sich in der Dissertation von H. Stemmer, 2001, Friedrich-Schiller-Universität Jena, mit dem Titel "Homogene Katalyse in überkritischem Kohlendioxid: Analogien und Unterschiede zu konventionellen Lösungsmitteln".

(A2) umfasst

1.) das Bis-GMA (2,2-Bis[4-(2-hydroxy-3-methacryloyloxypropoxy)phenyl]propan), das entweder durch Reaktion von Bisphenol A mit Glycidyl(meth)acrylat oder durch Reaktion des Diglycidylethers von Bisphenol A mit (Meth)acrylsäure erhalten werden kann. Vorteilhafterweise wird das Produkt auf letzterem Syntheseweg hergestellt. Hierzu wird der Ether zunächst durch Umsetzung von Epichlorhydrin (ECH, 1-Chlor-2,3-epoxypropan, hergestellt aus Allylchlorid (hergestellt aus Propen mit Chlor) mit Hypochloriger Säure und anschließender Behandlung mit NaOH) und Bisphenol A (BPA; 4,4'-Isopropylidenphenol, hergestellt aus Aceton und Phenol) erhalten. Weiterreaktion mit Methacrylsäure liefert das Bis-GMA.

Um zu einem Bis-GMA zu gelangen, das vorteilhafterweise zur Herstellung der erfindungsgemäßen, dentalen Kompositzusammensetzungen verwendet werden kann, sind einige Synthesedetails zu beachten. Bei der stöchiometrischen Umsetzung von BPA mit ECH (1:2) könnte man theoretisch die Bildung eines Produktes mit 2 terminalen Epoxidgruppen und keiner Hydroxylgruppe mit einem Molekulargewicht von 350 g/mol erwarten. In einem ersten Umsetzungsschritt reagiert die Epoxidgruppe des ECH mit dem Phenolation des BPA, das sich unter dem Einfluss des basischen Katalysators (NaOH) gebildet hat, unter Generierung des Chlorhydrinethers. In einem zweiten Schritt wird der Ether unter dem Einfluss der Base dehydrochloriert und der Monoglycidylether (MGEBA) entsteht. Die noch nicht abreagierte zweite phenolische Hydroxylgruppe könnte sich nun mit einem zweiten Molekül ECH umsetzen, die obigen Schritte wiederholen und den Diglycidylether von Bisphenol A (DGEBA) bilden. Dem gebildeten MGEBA stehen jetzt allerdings zwei konkurrierende Reaktionspfade offen, zum einen die bereits erwähnte Umsetzung mit ECH zum DGEBA, zum anderen die Reaktion des MGEBA mit weiterem BPA zu höhermolekularen Diphenolen, die sich ihrerseits weiter mit ECH umsetzen können. Zur Herstellung eines Bis-GMA, das vorteilhafterweise in einer erfindungsgemäßen, dentalen Kompositzusammensetzung Verwendung findet, wird die Reaktion zwischen BPA und ECH mit einem molaren Überschuss an ECH durchgeführt. Besonders bevorzugt wird mit einem großen molaren Überschuss gearbeitet, am besten so, dass das ECH die Rolle des Lösungsmittels übernimmt. Es ist somit dafür zu sorgen, dass sämtliche phenolische Hydroxylgruppen aufgebraucht werden und so die Bildung höherer Spezies möglichst unterdrückt wird.

DGEBA, das möglichst wenig oligomere Bestandteile aufweist, wird bei Temperaturen zwischen 100 und 150°C und Atmosphärendruck erwärmt bevor die Methacrylsäure in einem molaren Überschuss zudosiert wird. Sobald die Umsetzung zum Ester größer 96% beträgt, wird die Reaktion nach ca. 24 Stunden Reaktionszeit abgebrochen.

In der US 3, 066,112 wird die Synthese des Bis-GMA durch Reaktion von Bisphenol A mit Gycidyl(meth)acrylat angegeben.

2.) lichthärtbare Derivate des MDI (Diisocyanatodiphenylmethans), wie sie beispielsweise in den Beispielen der US 2006/0205902 A1 beschrieben sind.

Das MDI wird in einem ersten Schritt durch Umsetzung von Anilin mit Formaldehyd in Gegenwart von HCl zum Diaminodiphenylmethan und dann weiter in einer klassischen Phosgenierungsreaktion zum Diisocyanat unter Abspaltung von HCl synthetisiert. Reines MDI ist ein Feststoff. Aus diesem Grund verwendet man als Ausgangsprodukt für die Synthese der lichthärtbaren Derivate des MDI für die erfindungsgemäßen dentalen Kompositzusammensetzungen das nicht-destillierte Rohprodukt, wobei das zur Herstellung des MDI zum Einsatz kommende Amin das ungereinigte Anilin-Formaldehyd-Kondensat ist. Es bildet sich das entsprechend isomere flüssige Substanzgemisch der 4,4'-, 2,2'- und der 2,4'-MDI-Verbindungen. Bedingt durch den Kondensationscharakter der Formaldehyd-Anilin Reaktion kann das Reaktionsprodukt zudem auch Oligomere enthalten und daher höherfunktionell sein und ein komplexes Reaktionsgemisch darstellen.

MDI ist in unterschiedlichen Reinheiten und Zusammensetzungen auch kommerziell erhältlich, beispielsweise von "Covestro" unter dem Namen "Mondur", von "BASF" unter dem Namen "Lupranate" oder von "DOW" unter dem Namen "Isonate". Die bei Raumtemperatur flüssigen Varianten werden oft entweder als Mischungen der monomeren 4,4'-MDI und 2,4'-MDI Isomeren oder in Mischungen mit einem reduzierten Anteil an 2,2'-MDI Isomerem angeboten. Vorteilhafterweise werden zur Herstellung lichthärtbarer Derivate des MDI auch sogenannte "modifizierte MDI-Verbindungen" eingesetzt. Dem Fachmann ist die Herstellung dieser Varianten bekannt, sie sind auch kommerziell erhältlich. Hierzu zählen beispielsweise das carbodiimidmodifizierte MDI, das allophanatmodifizierte MDI, das biuretmodifizierte MDI sowie polymeres MDI, bzw. Kombinationen dieser MDI-Varianten (siehe hierzu auch die Patentschriften US 5,319,054 und US 5,440,003).

In den dem Dentalchemiker bekannten Isocyanatreaktionen können - ausgehend von flüssigem MDI - lichthärtbare MDI-Derivate für die erfindungsgemäßen dentalen Kompositzusammensetzungen synthetisiert werden (siehe hierzu zusätzlich auch WO 2018/071920 A1). Unter dem Begriff "lichthärtbare Derivate des MDI" werden insbesondere

die bevorzugten Verbindungen verstanden, die durch die Umsetzung von flüssigem MDI mit Alkoholen entstehen, wobei die Alkohole lichthärtbare Gruppen wie ungesättigte Kohlenwasserstoffgruppen tragen, beispielsweise $-CH=CH_2$, $-C(CH_3)=CH_2$, $-CH_2-CH=CH_2$, - $CH_2-C(CH_3)=CH_2$ und $-O-CH=CH_2$, vorzugsweise ungesättigte, aktivierte Kohlenwasserstoffgruppen tragen, beispielsweise $-O-(C=O)-CH=CH_2$ und $-O-(C=O)-C(CH_3)=CH_2$ oder $-(C=O)-CH=CH_2$, $-(C=O)-C(CH_3)=CH_2$. Die resultierenden Verbindungen sind Diurethane.

MDI kann beispielsweise mit primären Alkoholen wie 2-Hydroxyethyl(meth)acrylat (HEMA), 3-Hydroxypropyl(meth)acrylat (3-HPMA), 4-Hydroxybutyl(meth)acrylat (4-HBMA) oder mit sekundären Alkoholen wie Hydroxypropyl(meth)acrylat (HPMA), Hydroxybutyl(meth)acrylat (HBMA), Glycerindi(meth)acrylat (Gly-DMA), 2-Hydroxy-3-phenoxypropyl(meth)acrylat (HPPMA) zu lichthärtbaren MDI-Derivaten umgesetzt werden. Die Isocyanat-Alkohol Reaktion läuft in der Regel unkompliziert, schnell und quantitativ. Die Reaktion kann bei RT (Raumtemperatur) oder bei einer leicht über Raumtemperatur eingestellten Temperatur in Gegenwart einer sehr kleinen Katalysatormenge durchgeführt werden. Als Katalysatoren können tertiäre Amine, alkalische Substanzen und metallorganische Verbindungen verwendet werden. Zur Herstellung der lichthärtbaren Derivate des MDI werden vorzugsweise zinnorganische Verbindungen verwendet, beispielsweise Dibutylzinndilaurat, oder auch Verbindungen des zweiwertigen Zinns, wie Zinn-II-dioctoat. Alternativ können diese Reaktionen auch mit einem Amin, beispielsweise mit 1,4-Diazabicyclo[2.2.2]oxtan (DABCO), durchgeführt werden.

Zur Herstellung des MDI-HEMA Produkts wurde kommerziell erhältliches, flüssiges MDI mit einem Molekulargewicht von 250 g/mol mit kommerziell erhältlichem HEMA umgesetzt. Hierzu wurden die auf 60°C vorgewärmten Edukte im Molverhältnis 1 : 2 in einen zuvor ausgeheizten Reaktionsbehälter eingewogen und unter Rühren in dem bereits auf 60°C vorgewärmten Silikonbad zur Reaktion gebracht. Nach dem Vermengen der Edukte wurden wenige Tropfen Dibutylzinndilaurat der Mischung zugegeben. Das HEMA wurde zuvor mit BHT stabilisiert. Der Fortgang der Reaktion wurde mittels IR-Spektroskopie bestimmt. Die für das MDI charakteristische NCO-Gruppe absorbiert im Wellenzahlbereich von 2250 bis 2275 $cm^{-1}$. Die Bande besitzt eine hohe Intensität und wird durch Konjugation nicht beeinflusst. Nach 6 Stunden konnte keine Isocyanatbande mehr detektiert werden. Das MDI-HEMA Addukt wurde in einer Ausbeute von 86% als leicht gelbliches Öl erhalten.

3.) lichthärtbare Derivate des TMXDI, wie sie in N. Moszner et al. "Synthesis and polymerisation of new multifunctional urethane methacrylates", Die Angewandte Makromolekulare Chemie 265 (1999), 31 - 35, in N. Moszner et al. "A partially aromatic urethane dimethacrylate as a new substitute for Bis-GMA in restorative composites", Dental Materials, 24 2008, 694 - 699 sowie in der DE 198 03 979 A1 beschrieben sind.

Unter dem Begriff "lichthärtbare Derivate des TMXDI" werden Verbindungen verstanden, die durch die Umsetzung von TMXDI mit Alkoholen entstehen, wobei die Alkohole lichthärtbare Gruppen wie ungesättigte Kohlenwasserstoffgruppen tragen, beispielsweise $-CH=CH_2$, - $C(CH_3)=CH_2$, $-CH_2-CH=CH_2$, $-CH_2-C(CH_3)=CH_2$ und $-O-CH=CH_2$, vorzugsweise ungesättigte, aktivierte Kohlenwasserstoffgruppen tragen, beispielsweise $-O-(C=O)-CH=CH_2$ und $-O-(C=O)-C(CH_3)=CH_2$ oder $-(C=O)-CH=CH_2$, $-(C=O)-C(CH_3)=CH_2$. Die resultierenden Verbindungen sind Diurethane.

Die Umsetzungen der Isocyanate zu lichthärtbaren Derivaten des TMXDI erfolgen völlig analog zu den Reaktionen des MDI zu seinen lichthärtbaren Derivaten. In der Publikation von N. Moszner in der Angewandten Makromolekularen Chemie und in der Patentschrift ist die Umsetzung von HEMA und TMXDI zum Additionsprodukt TMXDI-HEMA explizit beschrieben. Diese Texte enthalten auch Synthesevorschriften zu weiteren Umsetzungen.

Das TMXDI wird durch Umsetzung der Isocyansäure mit dem m-Diisopropenylbenzol erhalten (siehe US 3,290,350). Die Verbindung ist auch kommerziell erhältlich.

(A3) sind lichthärtbare Monomere, die eine, zwei oder mehr ethylenische Gruppen aufweisende Substanzen sind, wie beispielsweise, ohne darauf beschränkt zu sein, die in der Dentalchemie üblicherweise eingesetzten (Meth)acrylatmonomere.

In der Patentliteratur ist eine Vielzahl von Verbindungen genannt, die allesamt Diester der Acryl- oder Methacrylsäure sind und sich zum Einsatz in einem erfindungsgemäßen lichthärtbaren Gemisch eignen.

Ein lichthärtbares Gemisch einer erfindungsgemäßen dentalen, Kompositzusammensetzung enthält beispielhaft ein oder mehrere Di(meth)acrylat-Monomere gewählt aus der Gruppe (A3), bestehend aus Ethylenglykoldi(meth)acrylat, alkoxyliertem Ethylenglykoldi(meth)acrylat, Diethylenglykoldi(meth)acrylat, 1,6-Hexandioldi(meth)acrylat, Triethylenglykoldi(meth)acrylat, 1,12-Dodecandioldi(meth)acrylat, 1,10-Decandioldi(meth)acrylat, Bisphenol-A-di(meth)acrylat, alkoxyliertem Bisphenol-A-di(meth)acrylat, Polyethylenglykoldi(meth)acrylat, Propylenglykoldi(meth)acrylat, Dipropylenglykoldi(meth)acrylat, Tripropylenglykoldi(meth)acrylat, Tetrapropylenglykoldi(meth)acrylat, Polypropylenglykoldi(meth)acrylat, 7,7,9-Trimethyl-3,14-dioxa-4,13-dioxo-5,12-diaza-hexadecan-1,16-dioxydi(meth)acrylat, Butandioldi(meth)acrylat, Propandioldi(meth)acrylat, Tetraethylenglykoldi(meth)acrylat, Neopentylglykoldi(meth)acrylat, alkoxyliertem Neopentyl-glykoldi(meth)acrylat, 2-Hydroxypropyl-1,3-di(meth)acrylat, 3-Hydroxypropyl-1,2-di(meth)acrylat, Pentaerythritoldi(meth)acrylat, alkoxyliertem Pentaerythritoldi(meth)acrylat, Pentaerythritoltri(meth)acrylat, alkoxyliertem Pentaerythritoltri(meth)acrylat, Pentaerythritol-tetra(meth)acrylat, alkoxyliertem Pentaerythritoltetra(meth)acrylat, Dipentaerythritoldi(meth)acrylat, alkoxyliertes Dipentaerythritoldi(meth)acrylat, Dipentaerythritoltri(meth)acrylat, alkoxyliertes Dipentaerythritoltri(meth)acrylat, Dipentaerythritoltetra(meth)acrylat, alkoxyliertes Dipentaerythritoltetra(meth)acrylat, Dipentaerythritolpenta(meth)acrylat, alkoxyliertes Dipentaerythritolpenta(meth)acrylat, Dipentaerythritolhexa(meth)acrylat, alkoxyliertes Dipentaerythritolhexa(meth)acrylat, Trimethylolpropantri(meth)acrylat, alkoxyliertem Trimethylolpropantri(meth)acrylat und Cyclohexandimethanoldi(meth)acrylat.

In einem bevorzugten erfindungsgemäßen lichthärtbaren Gemisch ist die Komponente (A3) in einem Anteil von kleiner 10 Gew.-% vorzugsweise von kleiner 5 Gew.-% bezogen auf die Gesamtmasse der Monomere (A) enthalten.

In einem besonders bevorzugten erfindungsgemäßen lichthärtbaren Gemisch fällt der optionale Bestandteil (A3) weg.

(A4) sind bestimmte lichthärtbare Monomeren, die als Partner für (A2) in thermowirksamen dentalen Zusammensetzungen ebenfalls infrage kommen, die der Dentalchemiker gut kennt und die in dentalen Zusammensetzungen gemeinhin Verwendung finden.

UDMA wird durch einfache Umsetzung von 2 mol Hydroxyethyl(meth)acrylat (HEMA) mit 2,4,4-Trimethylhexamethylendiisocyanat (TMDI) und/oder 2,2,4-Trimethylhexamethylendiisocyanat erhalten und ist, wie Bis-EMA oder wie die (alkoxylierten) hydroxylgruppenhaltigen Poly(meth)acrylaten, kommerziell erhältlich.

Weitere UDMA Varianten sind herstellbar durch Umsetzung von 2 mol HEMA mit 2,4-Dimethylhexamethylendiisocyanat (7,9 Dimethyl-3,14-dioxa-4,13-dioxo-5,12-diazahexadecan-1,16-dioxydi(meth)acrylat CAS-Nr: 865234-08-8), von 2 mol HEMA mit Hexamethylendiisocyanat (3,14-Dioxa-4,13-dioxo-5,12-diazahexadecan-1,16-dioxydi(meth)acrylat CAS-Nr.: 34100-36-2), von 2 mol HEMA mit Isophorondiisocyanat (1,5,5-Trimethyl-1-[(2-methacryloyloxyethyl)carbamoylmethyl]-3-(2-methacryloyloxyethyl)carbamoylcyclohexan CAS-Nr.: 42405-01-6), von 2 mol HEMA mit 2,2,4,4Tetramethylhexamethylendiisocyanat (7,7,9,9 Tetramethyl-3,14-dioxa-4,13-dioxo-5,12-diazahexadecan-1,16-dioxydi(meth)acrylat CAS-Nr.: 865234-10-2), von 2 mol Hydroxypropyl(meth)acrylat (HPMA) mit 2,2,4-Trimethylhexamethylendiisocyanat (2,7,7,9,15 Pentamethyl-3,14-dioxa-4,13-dioxo-5,12-diazahexadecan-1,16-dioxydi(meth)acrylat CAS-Nr.: 105883-40-7), von 2 mol Hydroxypropyl(meth)acrylat (HPMA) mit 2,4,4-Trimethylhexamethylendiisocyanat (2,7,9,9,15 Pentamethyl-3,14-dioxa-4,13-dioxo-5,12-diazahexadecan-1,16-dioxydi(meth)acrylat), von 2 mol HPMA mit 2,4-Dimethylhexamethylendiisocyanat (2,7,9,15 Tetramethyl-3,14-dioxa-4,13-dioxo-5,12-diazahexadecan-1,16-dioxydi(meth)acrylat), von 2 mol HPMA mit Hexamethylendiisocyanat (2,15 Dimethyl-3,14-dioxa-4,13-dioxo-5,12-diazahexadecan-1,16-dioxydi(meth)acrylat CAS-Nr.: 52723-94-1), von 2 mol HPMA mit Isophorondiisocyanat (1,5,5-Trimethyl-1-[(1-methacryloyloxypropan-2-yl)carbamoylmethyl]-3-(1-methacryloyloxypropan-2-yl)carbamoylcyclohexan CAS-Nr.: 76701-94-5), von 2 mol HPMA mit 2,2,4,4-Tetramethylhexamethylendiisocyanat (2,7,7,9,9,15 Hexamethyl-3,14-dioxa-4,13-dioxo-5,12-diazahexadecan-1,16-dioxydi(meth)acrylat), sowie die Varianten, die durch Umsetzung der oben genannten Diisocyanate mit Hydroxyethylacrylat (HEA) und Hydroxypropylacrylat (HPA) herstellbar sind.

Die lichthärtbaren Polysiloxane verfügen als Bindeglied zwischen anorganischer und organischer Chemie über besondere Materialeigenschaften. Lichthärtbare Polysiloxane sind als Bestandteile dentaler Kompositmaterialien unter dem Namen "Ormocere" (organically modified ceramics) allgemein bekannt. Beispielhaft zitiert seien hier die DE 44 16 857 C1, die DE 198 60 364 C2, die EP 1 874 847 B1, die EP 1 685 182 B1, die WO 2013/041723 A1, die WO 2013/053693 A1 oder die DE 10 2014 210 432. Da die lichthärtbaren Polysiloxane physiologisch inert sind, also keine nennenswerte Toxizität besitzen, sind sie speziell für Anwendungen in der Medizin bedeutsam. Grundlage für die kaum vorhandene Toxizität der Polysiloxane ist die geringe biologische Angreifbarkeit der Silizium-Kohlenstoffbindungen und die eingeschränkte Diffusionsfähigkeit der stark hydrophoben Polymerketten durch Zellmembranen, weshalb sie sich besonders zur Implantation (in Zähne) eignen sollten.

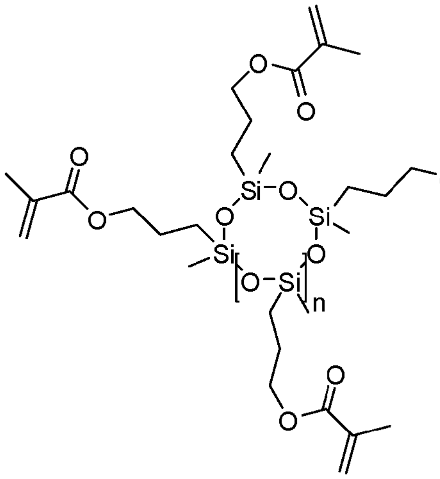

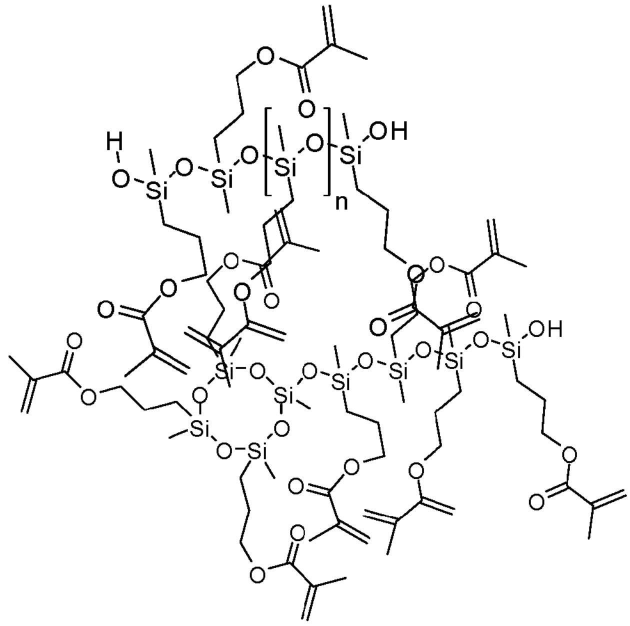

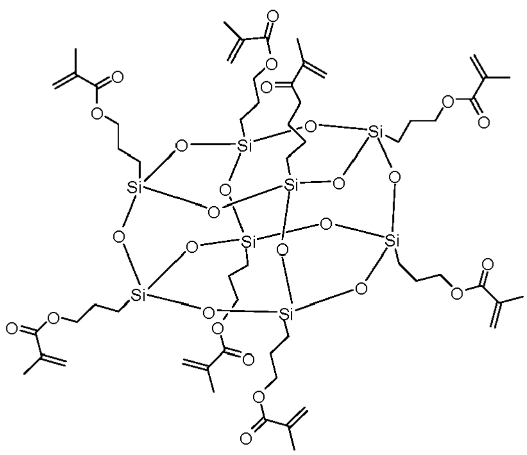

Mit radikalisch polymerisierbaren Gruppen substituierte kettenförmige und/oder zyklische und/oder in Käfigform vorliegende Polysiloxane mit mindestens 3 Siliziumatomen und/oder deren Mischformen können über den Sol-Gel Prozeß durch gezielte Hydrolyse und Kondensation von entsprechend funktionalisierten Derivaten von Alkoxiden des Siliziums oder von Halogensilanen synthetisiert werden. Diese Herstellverfahren sind in der Literatur vielfach beschrieben. In der Regel geht man bei einer solchen Synthese von einem Standardsilan wie beispielsweise dem Isocyanatopropyldiethoxysilan aus, das in einem ersten Schritt, ebenfalls in einer Standardreaktion, beispielsweise in einer Isocyanat-Alkohol Polyaddition, beispielsweise mit dem Glycerin-1,3-dimethacrylat zum entsprechenden Urethan umgesetzt wird. Die hierbei erhaltene Verbindung besteht auf der einen Seite aus dem Siliziumatom, das mit hydrolysier- und kondensierbaren Gruppen bestückt sind und das über einen sogenannten Abstandshalter, bestehend aus einer Alkylgruppe (hier eine Propylgruppe) und einer Urethangruppe als strukturelles Verbindungselement zu einem weiteren funktionellen Struktursegment, in diesem Fall zu zwei radikalisch polymerisierbare Methacrylatgruppen übergeht. Ein solch einfaches Syntheseverfahren kann in vielfacher Weise modifiziert werden, da die Reaktionsmöglichkeiten zwischen entsprechend funktionalisierten Silanen und geeigneten Reaktanden unbegrenzt erscheinen. Entsprechend groß sind die Synthesevorschläge in der Literatur. Die Ausgangsverbindung umfasst somit ein anorganisch kondensierbares Strukturelement, ein variabel gestaltbares Verbindungselement sowie ein radikalisch vernetzbares organisches Grundgerüst. In einer katalytisch gesteuerten Hydrolyse und Kondensation wird das Polysiloxan als ein anorganisches Kondensat, substituiert mit radikalisch polymerisierbaren Gruppen, gewonnen. Ob das Polykondensat in Form von Ketten, Ringen oder dreidimensionalen Käfigformen, bzw. in den entsprechenden Mischformen vorliegt, hängt von den genauen Bedingungen der Kondensation ab. Dazu zählen neben den Reaktionsbedingungen (pH-Wert, Menge an Lösungsmittel und Wasser, Art und Menge des Katalysators, Reaktionstemperatur, Art der Aufbereitung, etc.) auch die Strukturformen

des Ausgangssilans, wobei die Anzahl der Alkoxygruppen, die Anzahl der radikalisch polymerisierbaren Gruppen, die chemische Art des Verbindungselements sowie die Kettenlänge des Abstandshalters von Bedeutung sind. Angaben hierzu finden sich sowohl in der wissenschaftlichen Literatur wie in der Patentliteratur.

Das in dieser Anmeldung untersuchte lichthärtbare Polysiloxan wurde folgendermaßen synthetisiert:

100 g (0,42 mol) 3-Methacryloxypropyldimethoxymethylsilan werden in 400 ml Essigester gelöst. Es werden 10 ml 1N HCl-Lösung zugetropft und für 72 h bei 30°C gerührt. Es wird mit 2N NaOH-Lösung ausgeschüttelt, mit Wasser gewaschen und die organische Phase über Magnesiumsulfat getrocknet. Nach Zusatz von BHT wird zunächst bei 40 °C abrotiert und anschließend werden Lösungsmittelreste (z.B. Wasser- und Alkoholreste) unter Vakuum mittels einer Ölpumpe abgezogen, um die Alkohol- und Wasserreste zu entfernen. Es resultiert ein flüssiges Harz mit einer Viskosität von 3 Pa*s bei 25 °C.
$n_D^{20}$ = 1,466

Der Begriff "lichthärtbare Polysiloxane" beschreibt die mit lichthärtbaren Gruppen funktionalisierten Verbindungen von Alkoxiden des Siliziums oder von Halogensilanen, wobei die lichthärtbaren Gruppen ungesättigte Kohlenwasserstoffgruppen sind, beispielsweise - $CH=CH_2$, $-C(CH_3)=CH_2$, $-CH_2-CH=CH_2$, $-CH_2-C(CH_3)=CH_2$ und $-O-CH=CH_2$, vorzugsweise ungesättigte, aktivierte Kohlenwasserstoffgruppen sind, beispielsweise $-O-(C=O)-CH=CH_2$ und $-O-(C=O)-C(CH_3)=CH_2$ oder $-(C=O)-CH=CH_2$, $-(C=O)-C(CH_3)=CH_2$.

In einem bevorzugten erfindungsgemäßen lichthärtbaren Gemisch ist die Komponente (A5) in einem Anteil von kleiner 10 Gew.-% vorzugsweise von kleiner 5 Gew.-% bezogen auf die Gesamtmasse der Monomere (A) enthalten.

In einem besonders bevorzugten erfindungsgemäßen lichthärtbaren Gemisch fällt der optionale Bestandteil (A5) weg.

Die erfindungsgemäße, dentale, lichthärtbare, einkomponentige, Kompositzusammensetzung enthält Füllstoffe (B) in einer Menge von 65 bis 93 Gew.-%, bezogen auf die Kompositzusammensetzung.

Die Ausführungen zu den erfindungsgemäß einsetzbaren Füllstoffen (B) treffen sowohl auf den Einsatz der Füllstoffe in den erfindungsgemäßen, dentalen, lichthärtbaren, einkomponentigen Kompositzusammensetzungen als auch auf den Einsatz in den erfindungsgemäßen Verfahren zu. Insbesondere sind bevorzugt in erfindungsgemäßen, dentalen, lichthärtbaren, einkomponentigen Kompositzusammensetzungen einsetzbare Füllstoffe auch bevorzugt einsetzbar in den erfindungsgemäßen Verfahren und umgekehrt.

In einer bevorzugten Ausführungsform bestehen die Füllstoffe (B) aus und/oder sind herstellbar durch Vermischen von

(B1) 2 bis 25 Gew.-%, vorzugsweise 3 bis 20 Gew.-%, anorganischem Füllstoff mit einem $D_{50}$-Wert von 1 nm bis 200 nm und weiteren Füllstoffen, vorzugsweise
(B2) 40 bis 90 Gew.-%, vorzugsweise 50 bis 80 Gew.-%, anorganischem Füllstoff mit einem $D_{50}$-Wert von größer 1 µm bis 10 µm,
(B3) 8 bis 50 Gew.-%, vorzugsweise 15 bis 40 Gew.-%, anorganischem Füllstoff mit einem $D_{50}$-Wert von 0,4 µm bis 1,0 µm und
(B4) 0 bis 25 Gew.-%, vorzugsweise 0 bis 15 Gew.-%, weitere Füllstoffe, die nicht (B1), (B2) oder (B3) zuzuordnen sind,
wobei die Gew.-%-Angaben von (B1), (B2), (B3) und (B4) auf die Gesamtmasse der Füllstoffe (B) bezogen sind.

Die Füllstoffkomponente (B1) ist besonders wichtig, insbesondere um bei einem hohen Füllstoffgehalt von größer 75 Gew.-% die Viskositäten von $\eta_{20}$ und $\eta_{50}$ einzustellen.

(B1) sind nanoskalige Oxide oder Mischoxide im Größenbereich unterhalb von 200 nm, bevorzugt unterhalb von 100 nm und besonders bevorzugt unterhalb von 70 nm, die ausgewählt sind aus der Gruppe bestehend aus den Elementen Silizium, Titan, Yttrium, Barium, Zirkonium, Hafnium, Niob, Tantal, Wolfram, Wismut, Molybdän, Zinn, Zink, Ytterbium, Lanthan, Cer, Aluminium und deren Mischungen.

Besonders bevorzugt sind nanoskalige Teilchen von $SiO_2$, TiOz, ZrOz, ZnO, $SnO_2$ und $Al_2O_3$ und deren Mischungen.

(B1) können ebenfalls nanoskalige Sulfide, Selenide und Telluride von Metallen, Mischmetallen und deren Mischungen im Größenbereich unterhalb von 200 nm, bevorzugt unterhalb von 100 nm und besonders bevorzugt unterhalb von 70 nm sein.

Um eine gute Einbindung der Nanopartikel in die organische Phase des erfindungsgemäßen dentalen, lichthärtbaren, einkomponentigen Kompositmaterials zu gewährleisten, sind die Oberflächen der Nanopartikel organisch modifiziert, d.h. ihre Oberflächen weisen organische Strukturelemente auf.

Für eine organische Oberflächenmodifizierung sind vorzugsweise Verbindungen des allgemeinen Typs X-Sp-V geeignet, wobei "X" und "V" funktionelle Gruppen bedeuten, die durch einen Linker (Spacer, "Sp") miteinander verbunden sind.

Die funktionelle Gruppe "X" ist vorzugsweise so ausgewählt, dass sie unter Komplexbildung eine entsprechende Bindung mit der Oberfläche des Füllstoffpartikels eingehen kann. Geeignet sind beispielsweise Gruppen des Typs Silane, Phosphate, Phosphonate, Carboxylate, Dithiophosphate, Dithiophosphonate, Amine und Amide. Die Oberflächenanbindung der Verbindung für eine organische Oberflächenmodifizierung an die Füllstoffpartikel kann durch mehrfache Ausbildung einer funktionellen Gruppe (Polyphosphate, Polycarboxylate) verbessert werden.

Als Linker (Spacer, "Sp") eignen sich lineare oder verzweigte Alkylketten, Aromaten oder Kombinationen dieser Gruppen, die jeweils durch Heteroatome wie O, N, S oder P oder durch eine Urethangruppe unterbrochen sein können.

Die funktionelle Gruppe "V" vermittelt die Kompatibilität der Füllstoffpartikel mit der Gesamtmenge der lichthärtbaren Monomeren (A), beispielsweise durch Hydrophobierung. Bevorzugt sind lichthärtbare Gruppen; bevorzugt sind dabei lineare oder verzweigte Alkyl-, Arenyl- oder Alkenylgruppen, wobei letztere den Vorteil bieten, in die Polymerisation der härtbaren Monomeren miteinbezogen zu werden, was zu einer guten Einbindung der Partikel in das gehärtete Dentalmaterial führt. Besonders bevorzugt sind in diesem Zusammenhang (Meth)acrylatgruppen.

In einer bevorzugten Form handelt es sich bei den oxidischen nanoskaligen Füllstoffen um nanoskalige Kieselsäuren. Die Herstellung der nanoskaligen Kieselsäuren erfolgt auf bekannte Weise, z.B. durch Flammpyrolyse, Plasmaverfahren, Gasphasenkondensation, Kolloidtechniken, Präzipitationsverfahren, Sol-Gel Verfahren, etc.

Die nanoskaligen Kieselsäuren, die in erfindungsgemäßen, dentalen, lichthärtbaren, einkomponentigen, Kompositzusammensetzungen verwendet werden können, sind auch kommerziell, beispielsweise unter der Bezeichnung "NALCO COLLOIDAL SILICAS" (Nalco Chemical Co.), "Ludox colloidal silica" (Grace) oder "Highlink OG (Clariant) erhältlich.

Zur Oberflächenbehandlung der Kieselsäuren werden in besonders bevorzugter Weise Silane verwendet. Als Haftvermittler besonders geeignet ist das Methacryloxypropyltrimethoxysilan. Besonders bevorzugt eignen sich Verbindung der Formeln (1) oder (2) für die Silanisierung.

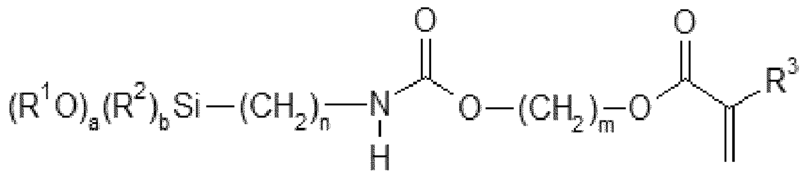

(1)

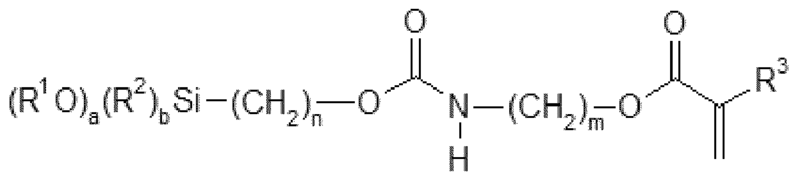

(2)

wobei $R^1$ eine C1- bis C4-Alkylgruppe bedeutet, und
$R^2$ eine C1- bis C8-Alkylgruppe bedeutet, und
$R^3$ ein Wasserstoffatom oder eine Methylgruppe bedeutet, und
a = 1, 2 oder 3, und
b = 3 - a, und
n = 1 bis 8, und
m = 1 bis 8 ist.

Das Verfahren zur Präparation silanisierter Füllstoffoberflächen besteht darin, eine Ethanol/Wasser Mischung (meistens 95/5 Vol%) zunächst mit Essigsäure auf einen pH-Wert von 4.5 - 5.5 einzustellen. Das Silan wird sodann in einer solchen Menge zugegeben, dass eine Lösungskonzentration von ca. 2% resultiert. Innerhalb von 5 Minuten sind die Alkoxysilylgruppen hydrolysiert und die Siloxanbildung setzt ein. Nun wird der zu behandelnde Füllstoff unter fortgesetztem Rühren der Lösung beigegeben. Innerhalb weniger Minuten wird das Silan vom Füllstoff adsorbiert und die Oberfläche der Füllkörper vom Haftvermittler beladen. Die Lösung wird abdekantiert und die Partikel werden zweimal mit Ethanol gewaschen. Abschließend werden die restlichen Silanolfunktionen für wenige Minuten bei 110°C und 24 Stunden bei Raumtemperatur kondensiert.

Das Silan agiert als oberflächenaktiver Stoff, der die Oberfläche des Füllstoffs mit der Harzmatrix kompatibilisiert und für einen festen Verbund zwischen dem organischen und dem anorganischen Material sorgt. Als besonders geeignetes Silan zum Aufbau eines Verbundes zwischen der anorganischen und der organischen Phase haben sich unter anderem das 3-Methacryloyloxypropyltrimethoxysilan sowie besonders bevorzugt die Silane der Formeln (1) und (2) erwiesen. Ein Teil der hydrolysierten Alkoxysilylgruppen des Silans reagieren direkt mit den Hydroxylgruppen auf der mineralischen Oberfläche des Füllstoffs, während der andere Teil untereinander kondensiert und so eine miteinander zusammenhängende Schicht des Kupplungsreagenzes auf der Füllstoffoberfläche ergibt. Im Verlauf der später stattfindenden radikalischen Polymerisation der dentalen Kompositmasse werden dann die Methacryloylfunktionen der an der Füllstoffoberfläche haftenden durchgängigen Schicht des Silans in die organische Harzphase mit einpolymerisiert und bilden so einen dauerhaften Verbund zwischen den hydrophilen Füllstoffen und der hydrophoben Harzmatrix.

Neben den nanoskaligen Oxiden und/oder Mischoxiden sowie den oben genannten Metallsalzen sind zur Komponente (B1) auch die röntgenopaken nanoskaligen Salze im Größenbereich unterhalb von 200 nm, bevorzugt unterhalb von 100 nm und besonders bevorzugt unterhalb von 70 nm der Seltenen Erden (Elemente 57 - 71), des Scandiums und des Yttriums zu zählen. Zu den bevorzugten Lanthanoiden gehören Lanthan, Cer, Samarium, Gadolinium, Dysprosium, Erbium und Ytterbium. Unter deren Salze sind die Fluoride bevorzugt, insbesondere nanoskaliges Ytterbiumfluorid ($YbF_3$).

Zu den röntgenopaken Salzen zählen auch bestimmte nanoskalige Salze der Erdalkalimetalle im Größenbereich unterhalb von 200 nm, bevorzugt unterhalb von 100 nm und besonders bevorzugt unterhalb von 70 nm, so beispielsweise die Salze des Bariums und des Strontiums. Bevorzugte Salze dieser Gruppe sind Fluoride, Phosphate und Sulfate, insbesondere nanoskaliges Bariumsulfat ($BaSO_4$) und nanoskaliges Strontiumfluorid ($SrF_2$).

Zur Oberflächenbehandlung der röntgenopaken nanoskaligen Salze im Größenbereich unterhalb von 200 nm, bevorzugt unterhalb von 100 nm und besonders bevorzugt unterhalb von 70 nm, speziell des Ytterbiumfluorids, des Strontiumfluorids sowie der Mischfluoride wie Strontium-dotierte Ytterbiumfluoridpartikel und des Bariumsulfats, werden in besonders bevorzugter Weise Phosphate, Phosphonate oder Carboxylate eingesetzt, wobei das Haftmittel über einen Spacer an eine (Meth)acrylatgruppe gebunden ist.

Von den röntgenopaken Salzen sind erfindungsgemäß besonders bevorzugt das Ytterbiumfluorid, das Strontiumfluorid, das Bariumsulfat sowie die Mischfluoride zwischen Ytterbiumfluorid und Strontiumfluorid, erfindungsgemäß ganz besonders bevorzugt sind das Strontiumfluorid-dotierte Ytterbiumfluorid und/oder das Ytterbiumfluorid dotierte Strontiumfluorid.

Die Nanopartikel (B1) sind bevorzugt nicht agglomeriert und nicht aggregiert. Sie liegen dann dispergiert in einem Medium vor, bevorzugt in monodisperser Form.

Als anorganische nicht-nanoskalige Füllstoffe (B2) und (B3) im Größenbereich über 200 nm, in dem Bereich, der im Folgenden "mikroskalig" genannt wird, können kompakte Gläser und unterschiedlich agglomerierte und aggregierte Kieselsäuren in verschiedenen Größen und Zuständen (monodispers, polydispers) zum Einsatz kommen.

Geeignete anorganische, mikroskalige Bestandteile (B2) und (B3) sind beispielsweise amorphe Materialien auf der Basis von Oxiden oder Mischoxiden aus $SiO_2$, $ZrO_2$ und/oder $TiO_2$ sowie Füllstoffe wie Quarz-Glaskeramik oder Glaspulver, Bariumsilikatgläser, Bariumfluorsilikatgläser, Strontiumsilikatgläser, Strontiumborsilikate, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie mikroskaliges Bariumsulfat oder Calciumfluorid sowie röntgenopake Füllstoffe wie mikroskaliges Ytterbiumfluorid.

Zum besseren Einbau in die Polymermatrix können auch die mikroskaligen Füllstoffe organisch oberflächenmodifiziert sein. Beispielhaft genannt sei die Oberflächenbehandlung der Füllstoffe mit einem Silan. Als Haftvermittler eignet sich hier besonders das Methacryloxypropyltrimethoxysilan. Besonders bevorzugt werden auch hier die Silane der Formeln (1) und (2) eingesetzt.

Innerhalb einer erfindungsgemäßen lichthärtbaren, einkomponentigen, dentalen Zusammensetzung bewirken die Mikropartikel eine weitgehende gleichmäßige Füllung des Volumens, wobei die verbleibenden Hohlräume zwischen den Mikropartikeln durch die oben beschriebenen Nanopartikel (Komponente (B1)) zumindest teilweise gefüllt werden. Unter Mikropartikeln werden in Zusammenhang mit der vorliegenden Erfindung Partikel mit einer mittleren Partikelgröße von über 200 nm bis 10 $\mu$m verstanden. Vorzugsweise ist die mittlere Partikelgröße kleiner als 5 $\mu$m. Es hat sich gezeigt, dass die mit den Mikropartikeln bereits erreichbare Volumenfüllung der lichthärtbaren, einkomponentigen, dentalen Zusammensetzung umso vollständiger und gleichmäßiger ist, je kleiner die Mikropartikel sind.

Dadurch wird sowohl die Schrumpfung der dentalen Zusammensetzung als auch ihre Empfindlichkeit gegen Abrasion vermindert.

Die Mikropartikel der jeweiligen Komponenten (B2) und (B3) können eine monomodale oder polymodale, beispielsweise eine bimodale Partikelgrößenverteilung aufweisen. Die Gesamtmikropartikelfraktion mit einer bimodalen oder multimodalen Partikelgrößenverteilung ist erfindungsgemäß bevorzugt (bei einer dann monomodalen Partikelgrößenverteilung der jeweiligen Mikropartikelkomponenten (B2) und (B3)), da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit

möglich die Bereiche zwischen den Partikeln der Fraktionen mit der größeren Partikelgröße ausfüllen werden. Die noch verbleibenden Hohlräume werden wie oben beschrieben durch Nanopartikel gefüllt.

Ganz besonders bevorzugt werden somit in einer erfindungsgemäßen lichthärtbaren, einkomponentigen, dentalen Zusammensetzung eine Mikropartikelgesamtkomponente ((B2) plus (B3)) eingesetzt, welche zwei oder mehr Fraktionen von Mikropartikeln enthält, wobei sich die mittleren Partikelgrößen der Fraktionen unterscheiden.

Vorzugsweise enthält die mikroskalige Gesamtkomponente ((B2) plus (B3)) zumindest zwei Mikropartikel-Fraktionen, wobei deren mittlere Partikelgrößen um mindestens 0,5 $\mu$m, bevorzugt um mindestens 0,7 $\mu$m, voneinander abweichen. In manchen Ausgestaltungen beträgt die Differenz der mittleren Partikelgrößen der Mikropartikel-Fraktionen mindestens 1,0 $\mu$m.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Besonders bevorzugt umfasst eine erfindungsgemäße, lichthärtbare, einkomponentige, dentale Zusammensetzung eine Gesamtmikrokomponente ((B2) plus (B3)), welche eine erste Mikropartikelfraktionen (B2), die jeweils eine mittlere Partikelgröße im Bereich von 1 $\mu$m bis 10 $\mu$m, vorzugsweise von 1,2 $\mu$m bis 5 $\mu$m und besonders bevorzugt von 1,5 $\mu$m bis 4,0 $\mu$m besitzen, und eine zweite Mikropartikelfraktionen (B3), die jeweils eine mittlere Partikelgröße im Bereich von 0,4 $\mu$m bis 1 $\mu$m, vorzugsweise von 0,5 $\mu$m bis 0,9 $\mu$m und besonders bevorzugt von 0,6 $\mu$m bis 0,8 $\mu$m besitzen.

Bevorzugt liegt das Verhältnis der Gesamtmasse von (B2) zu (B3) im Bereich von 1 : 1 bis 12 : 1, vorzugsweise im Bereich von 1,5 : 1 bis 8 : 1.

Bevorzugt liegt das Verhältnis der mittleren Korngröße von (B2) zur mittleren Korngröße von (B3) im Bereich von 1,5 : 1 bis 10 : 1, vorzugsweise im Bereich von 2 : 1 bis 5 : 1.

In einer besonders bevorzugten erfindungsgemäßen lichthärtbaren, einkomponentigen, dentalen Zusammensetzung umfasst die Komponente (B) eine erste Mikropartikelfraktion (B2), die jeweils eine mittlere Partikelgröße im Bereich von 1 $\mu$m bis 10 $\mu$m, vorzugsweise 1,2 $\mu$m bis 5 $\mu$m und besonders bevorzugt 1,5 $\mu$m bis 4,0 $\mu$m besitzt, und eine zweite Mikropartikelfraktionen (B3), die jeweils eine mittlere Partikelgröße im Bereich von 0,4 $\mu$m bis 1 $\mu$m, vorzugsweise von 0,5 $\mu$m bis 0,9 $\mu$m und besonders bevorzugt von 0,6 $\mu$m bis 0,8 $\mu$m, besitzt; wobei das Verhältnis der Gesamtmasse der ersten Mikropartikelfraktionen zur Gesamtmasse der zweiten Mikropartikelfraktionen im Bereich von 1 : 1 bis 12 : 1, vorzugsweise 1,5 : 1 bis 8 : 1 und/oder das Verhältnis der mittleren Korngröße der ersten Mikropartikelfraktion (B2) zur mittleren Korngröße der zweiten Mikropartikelfraktion (B3) im Bereich von 1,5 : 1 bis 10 : 1, vorzugsweise 2 : 1 bis 5 : 1 liegt.

In einer besonders bevorzugten erfindungsgemäßen lichthärtbaren, einkomponentigen, dentalen Zusammensetzung wird zumindest ein Teil der Mikropartikel der Komponenten (B2) und (B3) durch organisch oberflächenmodifizierte Partikel, vorzugsweise silanisierte Partikel gebildet und/oder es wird zumindest ein Teil der Mikropartikel der Komponenten (B2) und (B3) durch Dentalglas-Partikel gebildet; vorzugsweise sind zumindest ein Teil der Mikropartikel der Komponenten (B2) und (B3) organisch oberflächenmodifizierte Dentalglas-Partikel, vorzugsweise silanisierte Dentalglas-Partikel.

Neben den Komponenten (B1), (B2) und (B3) kann die lichthärtbare, einkomponentige, dentale Zusammensetzung zusätzlich zu der Mischung von Füllstoffpartikeln weitere Füllstoffe als Komponente (B4) umfassen.

So können z.B. verstärkend wirkende Füllstoff-Materialien, wie Glasfasern, Polyamid- oder Kohlenstofffasern eingesetzt werden. Eine erfindungsgemäße lichthärtbare, einkomponentige, dentale Zusammensetzung kann auch feinteilige und/oder grobteilige anorganische Füllstoffe in Partikelgrößen enthalten, die unterschiedlich zu denen von (B2) und (B3) sind, sowie Splitter- oder Perlpolymerisate aufweisen, wobei die Perlpolymerisate Homo- oder Copolymere organischer härtbarer Monomere sein können.

Eine erfindungsgemäße lichthärtbare, einkomponentige, dentale Zusammensetzung kann auch einen mikroskaligen röntgenopaken Füllstoff aufweisen. Bevorzugt enthält die erfindungsgemäße Zusammensetzung dann mikroskaliges $YbF_3$ und/oder $BaSO_4$.

Qualitative und quantitative Charakterisierung der Füllstoffpartikel:
Die nachfolgend beschriebenen Schritte in der qualitativen und quantitativen Charakterisierung der Füllstoffpartikel (insbesondere von nanoskaligen Füllstoffpartikeln) sind dem Fachmann gut bekannt und in der Literatur umfassend beschrieben.

Harz-/Füllstofftrennung:
In einem ersten Schritt werden 1 g einer erfindungsgemäßen lichthärtbaren, einkomponentigen, dentale Zusammensetzung (nachfolgend auch Kompositmaterial genannt) in 10 ml Aceton resuspendiert und die erhaltene Suspension anschließend mit einer Zentrifuge für 10 min bei 5000 U/min zentrifugiert. Der Überstand (nachfolgend Harzphase genannt) wird in ein Sammelglas abdekantiert und der Rückstand in 5 ml Aceton aufgeschlämmt. Es wird erneut für 10 min bei 5000 U/min zentrifugiert, dekantiert und der Rückstand in 5 ml Aceton erneut aufgeschlämmt. Die Schritte

Zentrifugieren, Dekantieren und Aufschlämmen werden noch zweimal unter identischen Bedingungen wiederholt. Die von den Harzphasen getrennte Gesamtmenge an Rückständen wird getrocknet und die Gesamtmenge an Harzphasen am Rotationsverdampfer von Aceton befreit.

Nach Durchführung des ersten Schrittes umfasst die getrocknete Gesamtmenge an Rückständen regelmäßig solche Füllstoffpartikel, die eine Partikelgröße von 200 nm oder größer 200 nm besitzen (nachfolgend makroskopische Füllstoffpartikel genannt). Die von Aceton befreite Gesamtmenge an Harzphasen (nachfolgend Harzanteil genannt) umfasst neben polymerisierbaren Monomeren außerdem regelmäßig Füllstoffpartikel mit einer Partikelgröße von ca. 200 nm oder insbesondere kleiner 200 nm (nachfolgend nanoskalige Teilchen genannt). Dieses Verfahren stellt somit sicher, dass das dentale Kompositmaterial durch die Zentrifugation in (i) einen Anteil makroskopischer Füllstoffpartikel, wobei speziell die dentalen Gläser im Größenordnungsbereich von größer 200 nm bis in den hohen Mikrometerbereich betroffen sind, und (ii) einen Harzanteil umfassend nanoskaligen Teilchen vollständig aufgetrennt wird.

Die mittlere Partikelgröße $d_{50}$ der erfindungsgemäß einzusetzenden makroskopischen Füllstoffpartikel der Füllstoffkomponenten (B2), (B3) und (B4) einer erfindungsgemäßen Zusammensetzung wird mittels Lichtstreuung (Laserbeugung) bestimmt, vorzugsweise mit einem Partikelgrößenmessgerät Beckman Coulter LS 13320.

Die nanoskaligen Teilchen, die sich in dem Harzanteil befinden, können beispielsweise sowohl nicht-aggregierte und/oder nicht agglomerierte, beispielsweise auch röntgenopake Partikel sein, beispielsweise $YbF_3$ oder $BaSO_4$ mit Partikelgrößen in einem Bereich von ca. 3 nm bis 200 nm, vorzugsweise von 5 nm bis 200 nm, besonders bevorzugt von 7 nm bis 100 nm und ganz besonders bevorzugt von 7 nm bis 70 nm aufweisen, als auch nicht-röntgenopake Kieselsäuren, die beispielsweise als pyrogene Kieselsäuren in Form von Aggregaten und/oder Agglomeraten mit einer Partikelgröße in einem Bereich von ca. 150 nm bis ca. 200 nm vorliegen oder auch Kieselsäuren, die nach dem Sol-Gel Verfahren (oder auch aus Wasserglas) synthetisiert werden und die ebenfalls nicht-aggregiert und/oder nichtagglomeriert vorliegen und Partikelgrößen in einem Bereich von ca. 3 nm bis 200 nm, vorzugsweise von 5 nm bis 200 nm, besonders bevorzugt von 7 nm bis 100 nm und ganz besonders bevorzugt von 7 nm bis 70 nm aufweisen.

Der Gesamtmassenanteil anorganischer Partikel in dem Harzanteil wird durch Differenzwiegung nach Veraschung eines entsprechenden Harzanteils gravimetrisch bestimmt.

TEM in Kombination mit EELS:
In einem zweiten Schritt werden die Füllstoffpartikel in dem Harzanteil einer qualitativen und quantitativen Charakterisierung unterzogen. Hierzu wird TEM (Transmissionselektronenmikroskopie) in Verbindung mit EELS (Elektronenenergieverlustspektroskopie) eingesetzt.

Mittels TEM werden die Partikelgrößen der einzelnen Partikel sowie deren Anzahl ermittelt; eine Elementarbestimmung einzelner Partikel erfolgt mittels EELS.

Zur Durchführung der kombinierten TEM/EELS-Charakterisierung wird in einem ersten Schritt durch Verdünnung mit härtbarem Harz die Konzentration der nanoskaligen Teilchen im Harzanteil zunächst reduziert. Dadurch wird weitestgehend ausgeschlossen, dass eine "Überlagerung" von nanoskaligen Teilchen in den späteren Bildern beobachtet wird. Eine solche "Überlagerung" würde die Partikelcharakterisierung verfälschen. Eigene Untersuchungen haben gezeigt, dass die optimale Partikelkonzentration (d.h. der Volumenanteil der Füllstoffpartikel) für solche Untersuchungen bei 1 Vol.-% liegt, bezogen auf die Gesamtmasse der verdünnten Probe.

Aus den durch Verdünnung mit härtbarem Harz gewonnenen verdünnten Harzanteilen werden in einem zweiten Schritt durch Aushärtung Stäbchen hergestellt. Aus diesen Stäbchen werden anschließend mit einem Ultradiamantmesser (beispielsweise Ultramikrotom ULTRCAT UCT, LEICA, Wetzlar) mehrere 300 nm dünne Ultradünnschnitte angefertigt. Die Ultradünnschnitte werden zur Stabilisierung auf Kupfer-TEM-Grids transferiert. Es resultieren Dünnschnittpräparate. Diese Dünnschnittpräparate werden dann mit 120 kV Beschleunigungsspannung in einem TEM mittels Hellfeld-Abbildungen untersucht.

Eine TEM-Untersuchung an den vorstehend beschriebenen Dünnschichtpräparaten erlaubt es, nicht-aggregierte und nicht-agglomerierte nanoskalige Partikel von aggregierten und/oder agglomerierten Partikeln (z.B. Kieselsäuren, wie beispielsweise Aerosilen), zu unterscheiden (zur Identifizierung der chemischen Zusammensetzung siehe die nachfolgenden Ausführungen).

Sofern hochauflösende Abbildungen untersucht werden sollen, können Ultradünnschnitte mit Schichtdicken kleiner 100 nm hergestellt und untersucht werden.

In einem dritten Schritt werden die Füllstoffpartikel in den Ultradünnschnitten bzw. Dünnschnittpräparaten mittels EELS-Punktanalysen chemisch charakterisiert, so dass die chemische Zusammensetzung einzelner Partikel bekannt wird (zur Bestimmung der Oberflächenmodifikation von Partikeln siehe nachfolgende Punkte).

Die volumen- oder gewichtsbezogenen Anteile von (ggf. auch mehrerer) Partikelfraktionen werden in einem vierten Schritt wie folgt aus einer TEM Aufnahme ermittelt: Der im Mikroskop betrachtete Bildausschnitt einer TEM-Aufnahme stellt eine Fläche dar, deren Kantenlängen a und b mittels der Legende bestimmt werden. Multipliziert mit der Dicke c des Ultradünnschnittes ergibt sich ein Gesamtvolumen $V_{Gesamt}$ für den in der TEM betrachteten Bereich. Dieses Gesamtvolumen $V_{Gesamt}$ ist die Summe aus dem Harzvolumen $V_{Harz}$ und dem Volumen aller Partikel $V_{Partikel}$ innerhalb dieses Volumens (das Volumen aller Partikel umfasst ggf. mehrere Gruppen von Partikeln, z.B. sortiert nach

verschiedenen Kriterien wie z.B. der Größe). Es gilt $V_{Gesamt} = a * b * c = V_{Harz} + V_{Partikel}$.

Das Volumen einzelner Partikel (und damit das Volumen aller Partikel in dem betrachteten Volumen) ist rechnerisch zugänglich über das Kugelvolumen der einzelnen Partikel. Hierzu wird in der TEM-Aufnahme der Durchmesser bzw. Radius eines entsprechenden Partikels bestimmt. Das daraus errechnete Kugelvolumen, multipliziert mit der Dichte des entsprechenden Materials, aus dem das Partikel besteht (Material identifizierbar mittels EELS), ergibt die Masse des Partikels. Das Harzvolumen, zugänglich aus dem Gesamtvolumen minus dem Partikelvolumen, multipliziert mit der Harzdichte, ergibt die Harzmasse. Die Harzdichte ergibt sich weitestgehend aus der Dichte des zur Verdünnung eingesetzten Harzes und ggf. der Dichte des verdünnten Harzanteils (letztere kann ggf. bei der Berechnung der Harzdichte vernachlässigt werden, wenn der Anteil des verdünnten Harzes vernachlässigbar ist). Der Anteil der Partikel (oder einer Gruppe von Partikeln) in Gewichtsprozent errechnet sich aus $mp*100/(m_{Partikel}+m_{Harz})$, wobei $m_P$ die Masse der betrachteten Partikelfraktion in dem betrachteten Volumen, $m_{Partikel}$ die Masse aller Partikel im betrachteten Volumen und $m_{Harz}$ die Masse des Harzes in dem betrachteten Volumen bedeutet. Bei der abschließenden Berechnung des Gewichtsanteils der zu betrachtenden Partikelfraktion wird der Verdünnungsfaktor entsprechend berücksichtigt.

Bestimmung organischer Oberflächenmodifikationen:

Vorabbetrachtung:

Viele bekannte röntgenopake Füllstoffmaterialien (wie z.B. Ytterbiumfluorid oder Bariumsulfat) weisen den Nachteil auf, dass sie nur schwer in die Matrix (Harzmatrix) aus polymerisierbaren Monomeren (die sogenannte organische Harzphase) einzuarbeiten sind, weil sie keine hinreichenden chemischen Bindungen (Anbindungsmöglichkeiten) mit den hydrophoben Gruppen des Mediums eingehen. Glasartige Füllstoffe lassen sich beispielsweise mit Hilfe der Silanisierung über Si-OH Gruppen hervorragend in die Harzmatrix dentaler Kompositmaterialien einarbeiten. Bei Ytterbiumfluorid und Bariumsulfat sind solche Gruppen auf den Oberflächen nicht vorhanden; sie sind somit nicht silanisierbar und führen in einem gehärteten Dentalmaterial zu einer unzureichenden physikalischen und chemischen Resistenz (siehe hierzu WO 2005/011621 A1, Seite 2 unten).

Die in einer erfindungsgemäßen lichthärtbaren, einkomponentigen, dentalen Zusammensetzung eingesetzten röntgenopaken nanoskaligen Teilchen werden somit auf ihren Oberflächen keine Silane aufweisen. Vielmehr erfolgt die Verknüpfung über Stickstoff-, Sauerstoff-, Schwefel- und/oder Phosphoratome (siehe hierzu wiederum WO 2005/011621 A1 sowie unsere Ausführungen weiter oben im Text).

Abtrennung polymerisierbarer Monomere von nanoskaligen Teilchen:

"Cross-Flow"-Verfahren:

Die Abtrennung polymerisierbarer Monomere von nanoskaligen Teilchen erfolgt beispielsweise in einem dem Fachmann bekannten "Cross-Flow"-Verfahren mittels Ultrafiltrationsmembranen.

Hierbei wird ein Harzanteil enthaltend nanoskalige Teilchen, polymerisierbare Monomere und ggf. ein geeignetes Verdünnungsmittel aus einem Behältnis mittels einer Pumpe in einen Kreislauf aus bestimmten Membranen gepumpt, wobei die polymerisierbaren Monomere die Poren der Membranen passieren und als Filtrat separiert werden, während die nanoskaligen Teilchen innerhalb des Kreislaufs (und damit im Behältnis) verbleiben.

Für diesen Trennungsgang ist beispielsweise das System "Vivaflow 50" von "Sartorius Stedim Biotech GmbH, Göttingen" geeignet. Pumpenantrieb (7554-95) und Pumpenkopf entstammen der Reihe "Masterflex US" von "Cole-Palmer Instrument Co.", Illinois, USA. Der Betrieb der Pumpe wird während der Filtration auf 2,5 bar eingestellt. Zwei Trennmembranen des Typs "50,000 MWCO (PES)" werden hintereinander geschaltet. Der MWCO (Molecular Weight Cut Off) gibt hierbei die Trenngrenze an, d.h. die Größe der Moleküle, die noch effizient die Membran passieren können. Dieser Wert wird in Dalton angegeben. Die erhaltenen Fraktionen werden anschließend wie unten beschrieben, untersucht.

Sedimentations-Feld-Fluss-Fraktionierung (SF3):

Besser noch als das "Cross-Flow"-Verfahren ist die Durchführung einer Sedimentations-Feld-Fluss-Fraktionierung (SF3). Dabei lassen sich insbesondere unterschiedliche Partikelfraktionen voneinander und zusätzlich vom Harzanteil trennen. Voraussetzung hierbei ist, dass sich die unterschiedlichen Partikelfraktionen ausreichend in Größe und/oder Dichte voneinander unterscheiden.

Entsprechende Geräte, die eine hierfür notwendige Trennsäule enthalten, sind bei der Firma Postnova Analytics GmbH, Landsberg, erhältlich. Das die Trennsäule enthaltende Modul trägt die Bezeichnung CF2000 Centrifugal FFF und wird durch die weiteren Module PN7140 (Eluent Organizer), PN1130 (Isocratic Pump), PN5300 (Autosampler), PN3621 MALS (21-Multi-Angle Light Scattering Detector) und PN8050 (Fraction Collector) ergänzt. In dieser Kombination

erlaubt die Centrifugal FFF-Anlage nicht nur die analytische, sondern auch die präparative Trennung von Partikelfraktionen. Die erhaltenen Fraktionen werden anschließen wie unten beschrieben, untersucht.

Charakterisierung der Oberflächenmodifikation:

Eine wie oben hergestellte und anschließend von Lösungsmitteln befreite Probe, die nanoskalige Teilchen in Form eines Pulvers enthält, wird anschließend mittels spektroskopischer Verfahren untersucht (z.B. mittels $^{1}$H-NMR, $^{13}$C-NMR, $^{15}$N-NMR, $^{29}$Si-NMR und $^{31}$P-NMR sowie IR).

Signale, die sich nicht einem Silan, beispielsweise dem gamma-Methacryloxypropylsilylrest, zuordnen lassen, werden organischen Oberflächenmodifikationen zugeordnet, die nicht auf Silanen basieren, z.B. Oberflächenmodifikationen mittels organischer Verbindungen auf Oberflächen von Ytterbiumfluorid bzw. Bariumsulfatpartikeln.

Die Anteile organisch oberflächenmodifizierter Partikel bzw. nicht organisch oberflächenmodifizierter Partikel können regelmäßig auch durch Auswertung der Intensitäten entsprechender Schwingungsbanden im IR-Spektrum bestimmt werden. Hierzu werden üblicherweise Referenz-Schwingungsbanden (Referenzkurven) organisch oberflächenmodifizierter bzw. nicht organisch oberflächenmodifizierter Partikel mit den entsprechenden chemischen Zusammensetzungen herangezogen.

Charakterisierung mittels Bildanalyse und Ramanspektroskopie:

Dem Fachmann sind zusätzliche Verfahren bzw. Kopplungen von Verfahren bekannt, die eine qualitative und quantitative Charakterisierung der Füllstoffpartikel erlauben. Insoweit sei beispielsweise verwiesen auf den Artikel "Chemische Identität einzelner Partikel" von Deborah Huck-Jones und Renate Hessemann in "Nachrichten aus der Chemie", Volume 62, September 2014, Seiten 886 und 887. Die darin offenbarte Kombination von Bildanalyse und Ramanspektroskopie eignet sich regelmäßig auch zur Charakterisierung der Füllstoffpartikel im Rahmen der vorliegenden Erfindung. Dies gilt insbesondere für Proben, die nach der oben beschriebenen Harzfüllstofftrennung erhalten werden. Eine geeignete Bildanalyse ist z.B. auch die oben im Text beschriebene TEM-Analyse.

Bestandteil (C) - Initiatoren und/oder Katalysatoren für die radikalische Polymerisation

Eine erfindungsgemäße lichthärtbare, einkomponentige, dentale Zusammensetzung enthält Initiatoren und/oder Katalysatoren für die radikalische Polymerisation, wobei Bestandteil (C) einen oder mehrere Lichthärtungsinitiatoren umfasst oder aus diesen besteht.

Die Ausführungen zu den erfindungsgemäß einsetzbaren Initiatoren treffen sowohl auf den Einsatz der Initiatoren in den erfindungsgemäßen, dentalen, lichthärtbaren, einkomponentigen Kompositzusammensetzungen als auch auf den Einsatz in den erfindungsgemäßen Verfahren zu. Insbesondere sind bevorzugt in erfindungsgemäßen, dentalen, lichthärtbaren, einkomponentigen Kompositzusammensetzungen einsetzbare Initiatoren auch bevorzugt einsetzbar in den erfindungsgemäßen Verfahren und umgekehrt.

Beispiele für einen Lichthärtungsinitiator schließen Substanzen ein, die nur photosensibilisierend wirken, sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acylgermanium-Verbindungen, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1, oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer erfindungsgemäßen lichthärtbaren, einkomponentigen, dentalen Zusammensetzung bewirken können.

Das Absorptionsmaximum von Campherchinon (CQ) liegt bei ca. 470 nm und somit im Bereich des blauen Lichts. Campherchinon (CQ) zählt zu den Plz-Initiatoren und wird regelmäßig zusammen mit einem Coinitiator eingesetzt.

Vorzugsweise enthält ein erfindungsgemäßes Kompositmaterial die Kombination eines alpha-Diketons und eines aromatischen tertiären Amins, bevorzugt ist die Kombination von Campherchinon (CQ) und Ethyl-*p*-*N*,*N*-dimethylaminobenzoat (DABE).

Ebenfalls bevorzugt ist die weitere Kombination des Systems "alpha-Diketon/aromatisches tertiäres Amin" mit

einem Phosphinoxid, insbesondere mit dem Phenyl-bis(2,4,6-trimethylbenzoyl)phosphinoxid und/oder dem 2,4,6-Trimethylbenzoyldiphenylphosphinoxid.

Bezüglich der Strukturen geeigneter Phosphinoxide zum Einsatz in einer erfindungsgemäßen lichthärtbaren, einkomponentigen, dentalen Zusammensetzung wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsinitiatorsystem in einer erfindungsgemäßen, lichthärtbaren, einkomponentigen, dentalen Zusammensetzung.

Die EP 1 905 415 beschreibt polymerisierbare Dentalzusammensetzungen mit Acylgermanium-Verbindungen als Initiatoren.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

<u>Bestandteil (D)</u> - weitere übliche Additive

Eine erfindungsgemäße, lichthärtbare, einkomponentige, dentale Zusammensetzung umfasst in manchen Fällen ein oder mehrere weitere(s) Additiv(e).

Die Ausführungen zu den erfindungsgemäß einsetzbaren Additiven treffen sowohl auf den Einsatz der Additive in den erfindungsgemäßen, dentalen, lichthärtbaren, einkomponentigen Kompositzusammensetzungen als auch auf den Einsatz in den erfindungsgemäßen Verfahren zu. Insbesondere sind bevorzugt in erfindungsgemäßen, dentalen, lichthärtbaren, einkomponentigen Kompositzusammensetzungen einsetzbare Additive auch bevorzugt einsetzbar in den erfindungsgemäßen Verfahren und umgekehrt.

Diese Additive können verschiedene Funktionen haben. Übliche Additive für den Einsatz in dentalen Werkstoffen sind dem Fachmann bekannt, je nach gewünschter Funktion wird er das oder die geeigneten Additive auswählen. Beispielhaft sind im Folgenden typische Additive und ihre Funktionen beschrieben.

Lichthärtbare, einkomponentige, dentale Zusammensetzungen, wie sie erfindungsgemäß bevorzugt sind, enthalten vorzugsweise einen oder mehrere Inhibitor(en), auch Stabilisator(en) genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren, einkomponentigen, dentalen Zusammensetzung. Gängige Inhibitoren sind Phenolderivate wie Hydrochinonmonomethylether (HQME) oder 2,6-Di-tert.-butyl-4-methylphenol (BHT). Weitere Inhibitoren wie tert.-Butylhydroxyanisol (BHA), 2,2-Diphenyl-1-picryl-hydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben. Alternative Inhibitoren sind in der DE 101 19 831 A1 oder in der EP 1 563 821 A1 angegeben.

Eine erfindungsgemäß bevorzugte lichthärtbare, einkomponentige, dentale Zusammensetzung umfasst somit als Additiv ein oder mehrere Polymerisationsinhibitoren zur Erhöhung der Lagerstabilität der Zusammensetzung, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

Eine erfindungsgemäße, lichthärtbare, einkomponentige, dentale Zusammensetzung kann als Additiv eine oder mehrere fluoridabgebende Substanzen, dabei vorzugsweise Natriumfluorid und/oder Aminfluoride umfassen.

UV-Absorber, die beispielsweise durch ihre konjugierten Doppelbindungssysteme und aromatischen Ringe befähigt sind, UV Strahlung zu absorbieren, sind in manchen Fällen Bestandteil einer erfindungsgemäßen, lichthärtbaren, einkomponentigen, dentalen Zusammensetzung. Beispiele an UV-Absorbern sind 2-Hydroxy-4-methoxybenzophenon, Salicylsäurephenylester 3-(2'-Hydroxy-5'-methylphenyl)benzotriazol oder Diethyl-2,5-dihydroxyterephthalat.

Da die Zähne möglichst naturgetreu wiederherzustellen sind, ist es notwendig, erfindungsgemäße, lichthärtbare, einkomponentige, dentale Zusammensetzungen in den unterschiedlichsten Farbtönen bereitzustellen. Man benutzt zu diesem Zweck in der Regel anorganische Farbstoffe sowie organische Pigmente in sehr geringen Mengen, die somit in bevorzugten Ausgestaltungen als Additiv eingesetzt werden.

Weitere optionale Additive sind Aromastoffe, dentale Arzneimittel, organische Polymere und Oligomere, vorzugsweise Weichmacher, Mikrobizide, vorzugsweise Bakterizide, grenzflächenaktive Substanzen, vorzugsweise Tenside, Konservierungsmittel oder Molekulargewichtsregler.

Die Erfindung umfasst ebenfalls eine gehärtete Kompositzusammensetzung, die durch Lichthärten einer erfindungsgemäßen, dentalen, lichthärtbaren, einkomponentigen Kompositzusammensetzung erhalten wird.

Die Erfindung umfasst auch eine erfindungsgemäße, dentale, lichthärtbare, einkomponentige Kompositzusammensetzung zur Anwendung in einem dentalen Therapieverfahren, vorzugsweise zur Anwendung in einem dentalen Therapieverfahren mit folgenden Schritten:

- Erwärmen der Kompositzusammensetzung auf eine Temperatur von 40 °C oder mehr, vorzugsweise eine Temperatur im Bereich von 40 °C bis 80 °C, vorzugsweise in einem Ofen und/oder durch Bestrahlung, vorzugsweise mit IR-Strahlen,
- Kontaktieren der auf eine Temperatur von 40 °C oder mehr, vorzugsweise eine Temperatur im Bereich von 40 °C bis 80 °C, erwärmten Kompositzusammensetzung mit einem zu behandelnden Zahn eines Patienten, vorzugsweise als dentales Füllungsmaterial, Unterfüllungsmaterial, Befestigungsmaterial und Fissurenversiegler.

Die Erfindung ist darüberhinaus auf die Verwendung einer erfindungsgemäßen Kompositzusammensetzung zur Herstellung eines dentalen Erzeugnisses gerichtet, wobei die Herstellung nicht am menschlichen oder tierischen Körper erfolgt.

In einer besonderen Ausführungsform betrifft die Erfindung ebenfalls eine Vorrichtung zur Applikation einer erfindungsgemäßen Kompositzusammensetzung, umfassend

- einen Hohlraum, der zumindest teilweise mit einer Menge einer Kompositzusammensetzung gefüllt ist und
- eine mit dem Hohlraum verbundene Applikationsspitze mit einer Austrittsöffnung für die Kompositzusammensetzung.

In einer weiteren besonderen Ausführungsform betrifft die Erfindung auch eine Vorrichtung wie oben beschrieben, wobei die Austrittsöffnung eine Austrittsquerschnittsfläche im Bereich von 0,2 bis 3,0 $mm^2$ besitzt, vorzugsweise eine Austrittsquerschnittsfläche von nicht mehr als 2,0 $mm^2$, besonders bevorzugt eine Austrittsquerschnittsfläche von nicht mehr als 1,5 $mm^2$, besitzt und

wobei die Applikationsspitze der Vorrichtung eine vorzugsweise aus Metall oder Kunststoff bestehende Kanüle ist und/oder
die Vorrichtung ausgewählt ist aus der Gruppe bestehend aus Compulen und Spritzen.

Bei großen Austrittsquerschnittsflächen der erfindungsgemäßen Vorrichtungen erlaubt es die erfindungsgemäße Kompositzusammensetzung aufgrund ihrer ausreichend hohen Viskosität bei Raumtemperatur die gefüllte Vorrichtung tropfsicher zu Lagern und zu Handhaben und so eine Kontamination von Arbeitsflächen zu vermeiden. Gleichzeitig erlaubt es die bei Erwärmung (z.B. auf 40 °C bis 80°C) ausreichend niedrige Viskosität aber auch die Vorrichtung mit einer Applikationsspitze mit einer möglichst kleinen Austrittsquerschnittsfläche auszugestalten, um eine punktgenaue Applikation bei der Behandlung zu gewährleisten.

Die Erfindung ist zudem auf ein Verfahren zur Herstellung einer erfindungsgemäßen Kompositzusammensetzung oder zur Herstellung einer Vorrichtung zur Applikation einer erfindungsgemäßen Kompositzusammensetzung gerichtet, mit folgendem Schritt: Vermischen der Bestandteile

(A) Monomere in einer Menge von 6 bis 35 Gew.-%, bezogen auf die Gesamtmenge der Kompositzusammensetzung, vorzugsweise 10 bis 35 Gew.-%, besonders bevorzugt 10 bis 25 Gew.-%,
(B) Füllstoffe in einer Menge von 65 bis 93 Gew.-%, vorzugsweise 65 bis 89 Gew.-%, besonders bevorzugt 75 bis 89 Gew.-%, bezogen auf die Gesamtmenge der Kompositzusammensetzung,
(C) Initiatoren in einer Menge von 0,001 bis 3 Gew.-% bezogen auf die Gesamtmenge der Kompositzusammensetzung,
(D) weitere Additive in einer Menge von 0,001 bis 5 Gew.-% bezogen auf die Gesamtmenge der Kompositzusammensetzung,

zu der Kompositzusammensetzung,
wobei die Bestandteile so ausgewählt sind, dass die Viskosität $\eta_{20}$ der Kompositzusammensetzung bei 20°C größer als 400 Pa*s ist und die Viskosität $\eta_{50}$ der Kompositzusammensetzung bei 50°C niedriger als 150 Pa*s ist und wobei der Quotient $\eta_{50}$ / $\eta_{20}$ aus der Viskosität der Kompositzusammensetzung bei 50°C und der Viskosität der Kompositzusammensetzung bei 20°C kleiner als 0,125 ist, vorzugsweise kleiner als 0,1.

Bevorzugt ist ein solches erfindungsgemäßes Verfahren, wobei
Bestandteil (A) hergestellt wird mit den folgenden Schritten:

Bereitstellen von zumindest

(A-i) einer ersten Monomerensubstanz und
(A-ii) einer zweiten Monomerensubstanz,
wobei

- die Viskosität $\eta_{20}$ der zweiten Monomerensubstanz (A-ii) bei 20 °C größer ist als 100 Pa*s,
- die Viskosität $\eta_{20}$ der ersten Monomerensubstanz (A-i) bei 20 °C größer ist als 100 mPa*s,
- die Viskosität der zweiten Monomerensubstanz (A-ii) bei 20 °C größer ist als die der ersten Monomerensubstanz (A-i)

und

- das Massenverhältnis der ersten Monomerensubstanz (A-i) zur zweiten Monomerensubstanz (A-ii) im Bereich von 2:1 bis 1:10 liegt,

wobei die zweite Monomerensubstanz (A-ii) vorzugsweise zumindest 40 Gew.-% 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI) enthält, wobei die Gew.-%-Angabe auf die Gesamtmasse der Monomere (A) bezogen ist
und
Mischen der bereitgestellten Monomerensubstanzen (A-i) und (A-ii), vor dem und/oder beim Mischen mit den Bestandteilen (B), (C) und (D)
und/oder wobei
Bestandteil (B) hergestellt wird durch Vermischen von
(B1) 2 bis 25 Gew.-%, vorzugsweise 3 bis 20 Gew.-%, anorganischem Füllstoff mit einem $D_{50}$-Wert von 1 nm bis 200 nm,
und weiteren Füllstoffbestandteilen, vorzugsweise (B2) 40 bis 90 Gew.-%, vorzugsweise 50 bis 80 Gew.-%, anorganischem Füllstoff mit einem $D_{50}$-Wert von größer 1 $\mu$m bis 10 $\mu$m, (B3) 8 bis 50 Gew.-%, vorzugsweise 15 bis 40 Gew.-%, anorganischem Füllstoff in einer Größe mit einem $D_{50}$-Wert von 0,4 $\mu$m bis 1,0 $\mu$m und
(B4) 0 bis 25 Gew.-%, vorzugsweise 0 bis 15 Gew.-%, weitere Füllstoffe, die nicht (B1), (B2) oder (B3) zuzuordnen sind,
wobei die Gew.-%-Angaben von (B1), (B2), (B3) und (B4) auf die Gesamtmasse der Füllstoffe (B) bezogen sind.

Besonders bevorzugt ist ein erfindungsgemäßes Verfahren mit folgendem zusätzlichen Schritt zur Herstellung der Vorrichtung zur Applikation einer Kompositzusammensetzung:
Einfüllen der hergestellten erfindungsgemäßen Kompositzusammensetzung in einen Hohlraum einer zuvor nicht gefüllten Vorrichtung zur Applikation einer Kompositzusammensetzung.

Die Erfindung umfasst zudem ein Verfahren zum Vorbereiten einer dentalen Behandlung eines Patienten, mit folgendem Schritt:
Erwärmen einer erfindungsgemäßen Kompositzusammensetzung oder einer erfindungsgemäßen Vorrichtung enthaltend eine erfindungsgemäße Kompositzusammensetzung auf eine Temperatur von 40 °C oder mehr, vorzugsweise eine Temperatur im Bereich von 40 °C bis 80 °C, vorzugsweise in einem Ofen und/oder durch Bestrahlung, vorzugsweise mit IR-Strahlen.

Die Erfindung umfasst auch ein Verfahren zum Behandeln eines Zahnes, mit folgenden Schritten:

- Herstellen oder Bereitstellen einer erfindungsgemäßen Kompositzusammensetzung,
- Erwärmen der erfindungsgemäßen Kompositzusammensetzung auf eine Temperatur von 40 °C oder mehr, vorzugsweise eine Temperatur im Bereich von 40 °C bis 80 °C, vorzugsweise in einem Ofen und/oder durch Bestrahlung, vorzugsweise mit IR-Strahlen,
- Kontaktieren der auf eine Temperatur von 40 °C oder mehr, vorzugsweise eine Temperatur im Bereich von 40 °C bis 80 °C, erwärmten erfindungsgemäßen Kompositzusammensetzung mit einem zu behandelnden Zahn eines Patienten, vorzugsweise als dentales Füllungsmaterial, Unterfüllungsmaterial, Befestigungsmaterial und Fissurenversiegler.

Die Erfindung wird im Folgenden näher beschrieben.

Beispiele:

Abkürzungen:

| | |
|---|---|
| TEGDMA: | Triethylenglycoldi methacrylat |
| GDMA: | Glycerin-1,3-dimethacrylat |
| DODMA: | 1,12-Dodecandioldimethacrylat |
| TCDDMA: | Bis(methacryloyloxymethyl)tricyclo[5.2.1.0$^{2,6}$]decan |
| TCD-2EO-DMA: | Bis(methacryloyl-2-oxyethyloxymethyl)tricyclo[5.2.1.0$^{2,6}$]decan |
| BCHDMA: | Bis(hydroxymethyl)bicyclo[2.2.1]heptan |
| UDMA: | 7,7,9-Trimethyl-4,13-dioxo-3,14-dioxa-5,12-diazahexadecan-1,16-dioxydimethacrylat |
| BisEMA: | Ethoxyliertes Bisphenol A-Dimethacrylat mit durchschnittlich 2,6 Ethylenoxid-einheiten |
| BisGMA: | 2,2-Bis[4-(2-hydroxy-3-methacryloxypropyloxy)phenyl]propan) |
| MDI-HEMA: | Additionsprodukt von Diisocyanatodiphenylmethan (MDI) mit HEMA (Hydroxyethylmethacrylat) |
| TMXDI-HEMA: | Additionsprodukt von Tetramethylxylylendiisocyanat (TMXDI) mit HEMA (Hydroxyethylmethacrylat) |
| Polysiloxan 1: | Kondensationsprodukt aus 3-Methacryloxypropyldimethoxymethylsilan (Synthese siehe weiter oben) |
| Dentalglas 1: | Barium-Aluminium-Borosilikat-Glas (D50 0,8 μm / D25 0,5 μm / D75 1,0 μm), silanisiert mit γ-Methacryloxypropyl-trimethohxysilan |
| Dentalglas 2: | Barium-Aluminium-Borosilikat-Glas (D50 2,7 μm / D25 1,4 μm / D75 6,1 μm) , silanisiert mit γ-Methacryloxypropyl-trimethohxysilan |
| Dentalglas 3: | Barium-Aluminium-Borosilikat-Glas (D50 0,8 μm / D25 0,5 μm / D75 1,0 μm), silanisiert mit 2-(Methacryloyloxy)ethyl[3-(triethoxysilyl)propyl]-carbamat |
| Dentalglas 4: | Barium-Aluminium-Borosilikat-Glas (D50 2,7 μm / D25 1,4 μm / D75 6,1 μm), silanisiert mit 2-(Methacryloyloxy)ethyl[3-(triethoxysilyl)propyl]-carbamat |
| Nano-$SiO_2$ 1: | nicht agglomerierte, nicht aggregierte Kieselsäure (D50 40 nm), silanisiert mit γ-Methacryloxypropyl-trimethohxysilan |
| Nano-$SiO_2$ 2: | nicht agglomerierte, nicht aggregierte Kieselsäure (D50 40 nm), silanisiert mit 2-(Methacryloyloxy)ethyl[3-(triethoxysilyl)propyl]carbamat |
| pyrogen.-$SiO_2$: | Aerosil R709 |

Herstellung der Harze:

Die entsprechenden Methacrylatmonomere wurden bei Raumtemperatur mit Hilfe eines KPG-Rührers homogenisiert. Dabei wurden die Initiatoren und Inhibitoren bereits direkt im Harz gelöst.

Herstellung der Komposite

Zur Herstellung der Komposite wurde jeweils die Monomermischungen mit den darin gelösten Initiatoren und Inhibitoren vorgelegt und schrittweise die Füllstoffe hinzugegeben und im Hauschildmischer homogenisiert. Anschließend wurde das fertige Komposit bei Raumtemperatur unter Vakuum (-0,85 bar) entlüftet.

Rheometer-Messung:

Gemessen wurde standardmäßig an einem Rheometer der Firma Anton Paar des Typs Physica MCR 301 mit einer 12 mm-Messplatte (Platte/Platte), 1 mm Spaltabstand und 450 mg Substanz.

Die Methode zur Messung der Viskositäten der Komposite bei 20 °C und bei 50 °C umfasst drei aufeinanderfolgende Abschnitte. Vor der Messung wird die Platte auf eine Temperatur von 20 °C temperiert. In Phase I der Messung wird für fünf Minuten bei 20 °C, einer Deformation von 1% und einer Oszillationsfrequenz von 300 rad/s gemessen. Der letzte Punkt bei der Temperatur, bei der die Viskosität des Komposits ihren Endwert erreicht hat, wird dabei zur Auswertung herangezogen. In Phase II wird ebenfalls für fünf Minuten bei den gleichen Deformations- und Oszillationsbedingungen bei einer Temperatur von 50 °C gemessen und dabei analog zu Phase I der letzte Punkt zur Auswertung herangezogen. Phase III entspricht wieder Phase I, in der dann die Viskosität des Komposits sich langsam wieder dem ursprünglichen Wert annähert.

Analog wurden für einige Beispiele die Viskositäten bei 37 °C und 68 °C betimmt, wobei in den drei Abschnitten bei Temperaturen von 37 °C, 68 °C und 37 °C statt 20 °C, 50 °C und 20 °C gemessen wurde.

Biegefestigkeit (BF): Die Biegefestigkeiten wurden entsprechend ISO 4049:2009 bestimmt. Die Kompositpasten wurden in Formen der Abmessung 25 mm x 2 mm x 2 mm appliziert und mit einer Celalux 2-Lampe (VOCO GmbH) abschnittsweise für jeweils 40 Sekunden lichtgehärtet. Die Bestimmung der Biegefestigkeit erfolgt bei einer Vorschubgeschwindigkeit von 0,75 mm/min an einer Zwick-Universalprüfmaschine (Zwick GmbH & Co. KG, Ulm).

Zur Festlegung der Grenzwerte:

Figur 1 zeigt beispielhaft die Temperaturabhängigkeit der Viskosität für Beispiel 1. Ein Material mit einer Viskosität von mehr als 400 Pa*s (bei Raumtemperatur) ist standfest. Auch bei einer Temperatur von 37°C (Intraoraltemperatur) ist das Material noch eben modellierbar. Bei 50°C wird eine Viskosität von unter 150 Pa*s erreicht und das Material beginnt zu fließen. In der zahnärztlichen Praxis werden die Komposite je nach Modell und Gerätetyp der Heizeinheit auf eine Temperatur von ca. 70°C vorgewärmt. Die erfindungsgemäße Zusammensetzung aus Beispiel 1 erreicht bei 68°C eine Viskosität von 54,8 Pa*s und ist damit äußerst fließfähig, da hier eine Viskosität erreicht wird, die der des kommerziell erhältlichen, fließfähigen Komposits GrandioSO Heavy Flow entspricht. Dieses Flow-Komposit dient als Referenz zum Nachweis der Fließfähigkeit vormals standfester Kompositmaterialien. Ist gewährleistet, dass die Komposite bei 50°C eine Viskosität von unter 150 Pa*s aufweisen, dann entspricht ihre Fließfähigkeit bei der Voraberwärmung genau dem eines auf Fließfähigkeit bei Raumtemperatur hin entwickelten Materials.

Die Thermowirksamkeit bzw. der Thermoeffekt einer dentalen Kompositzusammensetzung lässt sich anschaulich durch folghende Beziehung ausdrücken:

$$Thermoeffekt = \frac{\eta(20°C) - \eta(50°C)}{\eta(20°C)} \times 100\%$$

Ein Quotient $\eta_{50}$ / $\eta_{20}$ von 0,125 entspricht somit einem Thermoeffekt von 87,5% und ein Quotient $\eta_{50}$ / $\eta_{20}$ von 0,1 entspricht einem Thermoeffekt von 90%. Der Thermoeffekt einer dentalen Kompositzusammensetzung ist also umso größer je größer die Differenz seiner Viskositäten bei den beiden festgelegten Temperaturen ist. Diese Werte spiegeln die Eigenschaft der erfindungsgemäßen Kompositzusammensetzung wider, bei der Vorbereitungstemperatur eine möglichst hohe Viskosität und bei der Behandlungstemperatur eine möglichst niedrige Viskosität zu erreichen. Dies bedeutet für die dentale Kompositzusammensetzung:

1.) einen sauberen, sicheren, kontaminationsfreien Angang vor der Applikation,
2.) eine punktgenaue Applikation mit einem schnellen, vollständigen Anfließen an den Kavitätenrändern,
3.) Wechsel der fließfähigen Phase zurück zur Standfestigkeit und damit eine ausgezeichnete Modellierbarkeit für den Zahnarzt.

Eine ausreichende Standfestigkeit liegt also insbesondere dann vor, wenn sowohl die Viskosität $\eta_{20}$ bei 20 °C als auch die Viskosität $\eta_{37}$ bei 37 °C größer als 400 Pa*s sind. Denn dann lassen sich die erfindungsgemäßen Kompositzusammensetzungen sowohl bei Raumtemperatur sauber und kontaminationsfrei handhaben als auch bei Intraoraltemperatur gut modellieren.

Tabelle 1 enthält beispielhaft die numerischen Werte zur Thermowirksamkeit kommerziell erhältlicher Dentalkomposite.

Keine kommerziell erhältlichen Dentalkomposite weisen einen ausreichenden Thermoeffekt sowie die erforderlichen Werte der Viskosität vor und nach dem Erwärmen auf, so dass es bisher keine standfesten Produkte im Markt gibt, deren Viskosität bei Erwärmung überproportional abnimmt, sie also gut im erwärmten Zustand an den präparierten Kavitätenrändern - den Flow-Kompositen vergleichbar - anfließen, um bei zunehmender Abkühlung wieder in den standfesten Zustand überzugehen, gut modellierbar zu sein und dann nach Lichthärtung die überragenden mechanischen Werte hochgefüllter Dentalkomposite aufzuweisen.

Dentale Zusammensetzungen mit lichthärtbaren Bi- oder Tricyclen sind aus dem Stand der Technik bekannt.

Die Offenlegungsschrift DE 28 16 823 beschreibt härtbare Dentalmassen für Zahnfüllungen, Zahnprothesen und als Versiegelungsmassen, die Di(meth)acrylsäureester des Bis(hydroxymethyl)tricyclo[5.2.1.0$^{2,6}$]decans enthalten. In Beispiel 9 wird ein zweikomponentiges Paste/Paste System aus dem Diacrylsäureester des Bis(hydroxymethyl)tricyclo[5.2.1.0$^{2,6}$]decans mit dem propoxylierten Dimethacrylat des Bisphenol A beschrieben. In den Beispielen 12 und 13

werden lichthärtbare, einkomponentige Kompositzusammensetzungen offenbart, die als organische Matrix Diacrylsäureester des Bis(hydroxymethyl)tricyclo[5.2.1.0$^{2,6}$]decans aufweisen. In Beispiel 15 wird eine lichthärtbare Überzugsmasse auf Basis des Dimethacrylats des Bis(hydroxymethyl)tricyclo[5.2.1.0$^{2,6}$]decans in Kombination mit dem Hexandioldimethacrylat offenbart.

Die Offenlegungsschrift DE 29 31 926 beschreibt die (Meth)acrylate des alkoxyliertem Bis(hydroxymethyl)tricyclo[5.2.1.0$^{2,6}$]decans als geeignetes Bindemittel für härtbare Dentalmassen. Diese tricyclischen Systeme seien als Verdünnermonomere für hochviskose dentale Standardmonomere wie Bis-GMA (Bisphenol-A-Glycidyl-Methacrylat) geeignet. Zur Herstellung gemischter Bindemittel können die (Meth)acrylate des alkoxylierten Bis(hydroxymethyl)tricyclo[5.2.1.0$^{2,6}$]decans zusammen mit Ethylenglykoldi(meth)acrylat, Triethylenglykoldi(meth)acrylat, Trimethylolpropantri(meth)acrylat, Pentaerythrit-tetraacrylat, Butandiol-1,3-di(meth)acrylat und Hexandiol-1,6-di(meth)acrylat eingesetzt werden.

In Beispiel 14 wird eine zweikomponentige chemisch härtbare Dentalkompositzusammensetzung offenbart, deren Harzmatrix aus 70 Teilen Bis(hydroxymethyl)tricyclo[5.2.1.0$^{2,6}$]decans und 30 Teilen Bis-GMA besteht. Beispiel 19 gibt eine einkomponentige, strahlungshärtbare Kompositzusammensetzung an, die als Harzmatrix das propoxylierte Dimethacrylat des Bis(hydroxymethyl)tricyclo[5.2.1.0$^{2,6}$]decans aufweist.

Die Offenlegungsschrift DE 29 31 925 zielt - wie die oben zitierte DE 29 31 926 - auf (Meth)acrylsäureester von Ethergruppen enthaltenden tricyclischen Decandiolen. Beansprucht werden die alkoxylierten Tricyclen für eine Verwendung zur Herstellung von Klebe- und Dichtmassen sowie zur Herstellung von Zahnreparaturmaterialien.

Auch die US 4,131,729 gibt dentale, lichthärtbare Kompositzusammensetzungen der (Meth)acrylsäureester von Bis(hydroxymethyl)tricyclo[5.2.1.0$^{2,6}$]decan an (Beispiele 12, 13 und 15). Das tricyclische System kann allein oder in Kombination mit anderen Monomeren eingesetzt werden. Im Text heißt es "Bekannte bifunktionelle Monomere, die besonders geeignet sind für neue Kombinationen mit den erfindungsgemäßen Monomeren sind die Di(meth)acrylate von Hexandiol, Bis-(p-hydroxyethoxy)-phenylpropan, oder Bis-[p-(gamma-hydroxypropoxy)-phenyl]-propan."

Die DE 2 200 021, betitelt "Acrylsäureester von OH-gruppenhaltigen tricyclischen Decanolen", beansprucht Tricyclodecanderivate zur Verwendung in härtbaren Klebstoffen zur Herstellung von warmfesten Verklebungen.

Weitere (Meth)acrylsäureester von tricyclischen Decanolen werden in den Schriften DE 24 06 557, DE 35 22 005, DE 35 22 006 und DE 37 03 120 beschrieben.

Die DE 10 2005 021 332 A1 beansprucht dentale Kompositmaterialien mit geringer Schrumpfkraft. Die Harzmatrix der härtbaren Zusammensetzung umfasst Bis-GMA oder das tricyclische Derivat TCD-di-HEMA oder das tricyclische Derivat TCD-di-HEA in einer Menge von 60 - 80 Gew.-%, 10 bis 18 Gew.-% UDMA (Urethandimethacrylat) und der Rest TEDMA und/oder multifunktionelle Vernetzer, wobei die Gew.-% Angaben auf die Harzphase bezogen sind.

Die DE 10 2007 034 457 A1 beansprucht dentale Kompositmaterialien mit niedriger Schrumpfspannung und hoher Biegefestigkeit. Die Harzmatrix der härtbaren Zusammensetzung enthält Bis-GMA und ein Mitglied der Gruppe TCD-di-HEMA und TCD-di-HEA in einer Menge von 60 - 80 Gew.-%, 10 - 18 Gew.-% UDMA und als Rest TEDMA und/oder multifunktionelle Vernetzer, wobei die Gew.-% Angaben auf die Harzphase bezogen sind.

Die EP 2 436 366 B1 ist auf ein Kompositmaterial gerichtet, das ein Monomer mit einem polyalicyclischen Strukturelement umfasst und als Versiegelungsmaterial eingesetzt wird. In den Beispielen 3 und 4 (Tabelle 1) wird das Bis(methacryloyloxymethyl)-tricyclo[5.2.1.0$^{2,6}$]decan zusammen mit Bis-GMA eingesetzt. Allerdings liegt der Gesamtfüllstoffgehalt weit unter 70 Gew.-% bezogen auf die Kompositzusammensetzung und der Anteil des Tricyclodecanderivats übersteigt den Anteil des Bis-GMA bei weitem.

Tabelle 1:

| **kommerzielles Komposit** | η(20°C) [*Pa · s*] | η(50°C) [*Pa · s*] | $\frac{\eta(50°C)}{\eta(20°C)}$ | $\frac{\eta(20°C) - \eta(50°C)}{\eta(20°C)} \times 100\%$ |
|---|---|---|---|---|
| Filtek Supreme XTE (3M Espe) | 2045 | 869 | 0.42 | 57.5% |
| Clearfil Majesty Posterior (Kuraray) | 3147 | 2135 | 0.68 | 32.2% |
| Ceram X (Dentsply) | 1422 | 368 | 0.26 | 74.1% |
| Sonic Fill (Kerr) | 419 | 257 | 0.61 | 38.7% |
| Sonic Fill 2 (Kerr) | 1019 | 683 | 0.67 | 33.0% |

(fortgesetzt)

| **kommerzielles Komposit** | η(20°C) [*Pa* · *s*] | η(50°C) [*Pa* · *s*] | $\frac{\eta(50°C)}{\eta(20°C)}$ | $\frac{\eta(20°C) - \eta(50°C)}{\eta(20°C)} \times 100\%$ |
|---|---|---|---|---|
| Ecosite Universal Bulk Fill (DMG) | 460 | 221 | 0.48 | 51.9% |
| Esthet-X (Dentsply) | 923 | 250 | 0.27 | 73.2% |
| GrandioSO Heavy Flow (VOCO) | 53.3 | n.b. | n.b. | n.b. |

Tabelle 2:

| Beispiel | | | 1 | 2 | 3 |
|---|---|---|---|---|---|
| (A) Monomere | (A1) | TCDDMA | 4.23 | 4.63 | 6.04 |
| | (A2) | BisGMA | 16.90 | 10.87 | 9.11 |
| | (A3) | UDMA | | | |
| (B) Füllstoffe | (B1) | Nano-$SiO_2$ 1 | 6.28 | 14.76 | 14.56 |
| | (B2) | Dentalglas 2 | 60.33 | 58.02 | 58.49 |
| | (B3) | Dentalglas 1 | 12.04 | 11.59 | 11.68 |
| (C) Initiatoren | (C1) | CQ | 0.06 | 0.04 | 0.04 |
| | (C2) | DABE | 0.10 | 0.07 | 0.07 |
| (D) Sonstiges | (D1) | BHT | 0.06 | 0.02 | 0.01 |
| | | Gesamt | 100.00 | 100.00 | 100.00 |
| Viskosität η(20°C) [*Pa* · *s*] | | | 2290.2 | 1974.1 | 1405.9 |
| Viskosität η(37°C) [Pa · *s*] | | | 505.8 | 473.3 | 449.7 |
| Viskosität η(50°C) [*Pa* · *s*] | | | 142.3 | 134.1 | 136.7 |
| Viskosität η(68°C) [*Pa* · *s*] | | | 54.8 | 54.1 | 53.5 |
| $\frac{\eta(50°C)}{\eta(20°C)}$ | | | 0.06 | 0.07 | 0.10 |
| Thermoeffekt $\frac{\eta(20°C)-\eta(50°C)}{\eta(20°C)} \times 100\%$ | | | 93.8% | 93.2% | 90.3% |
| Biegefestigkeit [MPa] | | | 155.4 | 149.6 | 154.8 |

Tabelle 3:

| Beispiel | | | 4 | 5 | 6 |
|---|---|---|---|---|---|
| (A) Monomere | (A1) | TCDDMA | 6.34 | 5.21 | 4.56 |
| | (A2) | BisGMA | 14.79 | 12.17 | 10.71 |
| | (A3) | UDMA | | | |

(fortgesetzt)

| Beispiel | | | 4 | 5 | 6 |
|---|---|---|---|---|---|
| (B) Füllstoffe | (B1) | Nano-$SiO_2$ 1 | 6.28 | 9.26 | 16.67 |
| | (B2) | Dentalglas 2 | 60.33 | 61.02 | 58.24 |
| | (B3) | Dentalglas 1 | 12.04 | 12.19 | 9.69 |
| (C) Initiatoren | (C1) | CQ | 0.06 | 0.05 | 0.05 |
| | (C2) | DABE | 0.10 | 0.08 | 0.07 |
| (D) Sonstiges | (D1) | BHT | 0.06 | 0.02 | 0.01 |
| | | Gesamt | 100.00 | 100.00 | 100.00 |
| Viskosität $\eta$(20°C) [*Pa · s*] | | | 1502.3 | 1253.4 | 1817.7 |
| Viskosität $\eta$(50°C) [*Pa · s*] | | | 123.9 | 134.5 | 142.3 |
| $\frac{\eta(50°C)}{\eta(20°C)}$ | | | 0.08 | 0.11 | 0.08 |
| Thermoeffekt $\frac{\eta(20°C)-\eta(50°C)}{\eta(20°C)} \times 100\%$ | | | 91.8% | 89.3% | 92.2% |
| Biegefestigkeit [MPa] | | | 141.0 | 134.9 | 152.4 |

Tabelle 4:

| Beispiel | | | 7 | 8 | 9 |
|---|---|---|---|---|---|
| (A) Monomere | (A1) | TCDDMA | 7.38 | 5.74 | 8.55 |
| | (A2) | BisGMA | 17.23 | 13.39 | 8.11 |
| | (A3) | UDMA | | 2.00 | |
| (B) Füllstoffe | (B1) | Nano-$SiO_2$ 1 | 3.00 | 6.28 | 14.16 |
| | (B2) | Dentalglas 2 | 60.17 | 60.33 | 57.55 |
| | (B3) | Dentalglas 1 | 12.01 | 12.04 | 11.51 |
| (C) Initiatoren | (C1) | CQ | 0.07 | 0.06 | 0.04 |
| | (C2) | DABE | 0.11 | 0.10 | 0.07 |
| (D) Sonstiges | (D1) | BHT | 0.03 | 0.06 | 0.02 |
| | | Gesamt | 100.00 | 100.00 | 100.00 |
| Viskosität $\eta$(20°C) [*Pa · s*] | | | 1387.2 | 1465.2 | 1203.1 |
| Viskosität $\eta$(50°C) [*Pa · s*] | | | 112.8 | 118.1 | 145.4 |
| $\frac{\eta(50°C)}{\eta(20°C)}$ | | | 0.08 | 0.08 | 0.12 |
| Thermoeffekt $\frac{\eta(20°C)-\eta(50°C)}{\eta(20°C)} \times 100\%$ | | | 91.9% | 91.9% | 87.9% |
| Biegefestigkeit [MPa] | | | 128.2 | 135.0 | 123.7 |

Tabelle 5:

| Beispiel | | | 10 | 11 | 12 |
|---|---|---|---|---|---|
| (A) Monomere | (A1) | TCDDMA | 7.51 | | |
| | | TCD-2EO-DMA | | 4.63 | |
| | | BDHDMA | | | 4.63 |
| | (A2) | BisGMA | 17.43 | 10.87 | 10.87 |
| | (A3) | UDMA | | | |
| (B) Füllstoffe | (B1) | pyrogen.-$SiO_2$ | 2.20 | | |
| | | Nano-$SiO_2$ 1 | | 14.76 | 14.76 |
| | (B2) | Dentalglas 2 | 60.57 | 58.02 | 58.02 |
| | (B3) | Dentalglas 1 | 12.11 | 11.59 | 11.59 |
| (C) Initiatoren | (C1) | CQ | 0.06 | 0.04 | 0.04 |
| | (C2) | DABE | 0.10 | 0.07 | 0.07 |
| (D) Sonstiges | (D1) | BHT | 0.02 | 0.02 | 0.02 |
| | | Gesamt | 100.00 | 100.00 | 100.00 |
| Viskosität $\eta$(20°C) [*Pa · s*] | | | 1488.6 | 1966.3 | 1908.4 |
| Viskosität $\eta$(50°C) [*Pa · s*] | | | 143.6 | 139.8 | 142.1 |
| $\frac{\eta(50°C)}{\eta(20°C)}$ | | | 0.10 | 0.07 | 0.07 |
| Thermoeffekt $\frac{\eta(20°C)-\eta(50°C)}{\eta(20°C)} \times 100\%$ | | | 90.4% | 92.9% | 92.6% |
| Biegefestigkeit [MPa] | | | 127.0 | 142.1 | 135.6 |

Tabelle 6:

| Beispiel | | | 13 | 14 | 15 |
|---|---|---|---|---|---|
| (A) Monomere | (A1) | TCDDMA | 4.63 | 6.20 | 5.03 |
| | (A2) | BisGMA | 10.87 | | |
| | | MDI-HEMA | | 9.30 | |
| | | TMXDI-HEMA | | | 10.47 |
| | (A3) | UDMA | | | |
| (B) Füllstoffe | (B1) | Nano-$SiO_2$ 1 | | 14.76 | 14.76 |
| | | Nano-$SiO_2$ 2 | 14.76 | | |
| | (B2) | Dentalglas 2 | | 58.02 | 58.02 |
| | | Dentalglas 4 | 58.02 | | |
| | (B3) | Dentalglas 1 | | 11.59 | 11.59 |
| | | Dentalglas 3 | 11.59 | | |
| (C) Initiatoren | (C1) | CQ | 0.04 | 0.04 | 0.04 |
| | (C2) | DABE | 0.07 | 0.07 | 0.07 |

(fortgesetzt)

| Beispiel | | | 13 | 14 | 15 |
|---|---|---|---|---|---|
| (D) Sonstiges | (D1) | BHT | 0.02 | 0.02 | 0.02 |
| | | Gesamt | 100.00 | 100.00 | 100.00 |
| Viskosität η(20°C) [*Pa* · *s*] | | | 2344.5 | 1641.1 | 1588.7 |
| Viskosität η(50°C) [*Pa* · *s*] | | | 117.3 | 148.7 | 147.3 |
| $\frac{\eta(50°C)}{\eta(20°C)}$ | | | 0.05 | 0.09 | 0.09 |
| Thermoeffekt $\frac{\eta(20°C)-\eta(50°C)}{\eta(20°C)} \times 100\%$ | | | 95.0% | 90.9% | 90.7% |
| Biegefestigkeit [MPa] | | | 153.1 | 128.4 | 123.5 |

Tabelle 7:

| Beispiel | | | 16 | | |
|---|---|---|---|---|---|
| (A) Monomere | (A1) | TCDDMA | 7.21 | | |
| | (A2) | BisGMA | 16.81 | | |
| | (A3) | UDMA | | | |
| (B) Füllstoffe | (B1) | Nano-$SiO_2$ 1 | 6.28 | | |
| | (B2) | Dentalglas 2 | 57.92 | | |
| | (B3) | Dentalglas 1 | 11.56 | | |
| (C) Initiatoren | (C1) | CQ | 0.06 | | |
| | (C2) | DABE | 0.10 | | |
| (D) Sonstiges | (D1) | BHT | 0.06 | | |
| | | Gesamt | 100.00 | | |
| Viskosität η(20°C) [*Pa* · *s*] | | | 502.2 | | |
| Viskosität η(50°C) [*Pa* · *s*] | | | 62.5 | | |
| $\frac{\eta(50°C)}{\eta(20°C)}$ | | | 0.124 | | |
| Thermoeffekt $\frac{\eta(20°C)-\eta(50°C)}{\eta(20°C)} \times 100\%$ | | | 87.6% | | |
| Biegefestigkeit [MPa] | | | 127.2 | | |

Tabelle 8:

| Beispiel | | | 17 | 18 | 19 |
|---|---|---|---|---|---|
| (A) Monomere | (A4) | UDMA | 3.84 | 4.48 | 5.17 |
| | (A2) | BisGMA | 11.01 | 11.28 | 11.64 |
| | (A5) | TEGDMA | 2.62 | 2.15 | 1.66 |

(fortgesetzt)

| Beispiel | | | 17 | 18 | 19 |
|---|---|---|---|---|---|
| (B) Füllstoffe | (B1) | Nano-$SiO_2$ 1 | 10.00 | 10.00 | 10.00 |
| | (B2) | Dentalglas 2 | 60.33 | 59.97 | 59.50 |
| | (B3) | Dentalglas 1 | 12.07 | 11.99 | 11.90 |
| (C) Initiatoren | (C1) | CQ | 0.04 | 0.04 | 0.04 |
| | (C2) | DABE | 0.07 | 0.07 | 0.07 |
| (D) Sonstiges | (D1) | BHT | 0.02 | 0.02 | 0.02 |
| | | Gesamt | 100.00 | 100.00 | 100.00 |
| Viskosität $\eta$(20°C) [$Pa \cdot s$] | | | 708 | 1069 | 1211 |
| Viskosität $\eta$(37°C) [$Pa \cdot s$] | | | 322 | 402 | 431 |
| Viskosität $\eta$(50°C) [$Pa \cdot s$] | | | 128 | 133 | 145 |
| Viskosität $\eta$(68°C) [$Pa \cdot s$] | | | 51 | 52 | 54 |
| $\frac{\eta(50°C)}{\eta(20°C)}$ | | | 0.181 | 0.124 | 0.120 |
| Thermoeffekt $\frac{\eta(20°C)-\eta(50°C)}{\eta(20°C)} \times 100\%$ | | | 81.9% | 87.6% | 88.0% |
| Biegefestigkeit [MPa] | | | 136.4 | 137.2 | 137.9 |

Tabelle 9:

| Beispiel | | | 20 | 21 | 22 |
|---|---|---|---|---|---|
| (A) Monomere | (A4) | BisEMA | 5.83 | 5.22 | 4.66 |
| | (A2) | BisGMA | 10.82 | 12.18 | 13.99 |
| | (A5) | TEGDMA | | | |
| (B) Füllstoffe | (B1) | Nano-$SiO_2$ 1 | 9.00 | 9.00 | 9.00 |
| | (B2) | Dentalglas 2 | 61.85 | 61.22 | 60.18 |
| | (B3) | Dentalglas 1 | 12.37 | 12.24 | 12.04 |
| (C) Initiatoren | (C1) | CQ | 0.04 | 0.04 | 0.04 |
| | (C2) | DABE | 0.07 | 0.07 | 0.07 |
| (D) Sonstiges | (D1) | BHT | 0.02 | 0.02 | 0.02 |
| | | Gesamt | 100.00 | 100.00 | 100.00 |
| Viskosität $\eta$(20°C) [$Pa \cdot s$] | | | 1045 | 1233 | 1568 |
| Viskosität $\eta$(50°C) [$Pa \cdot s$] | | | 125 | 137 | 147 |
| $\frac{\eta(50°C)}{\eta(20°C)}$ | | | 0.120 | 0.111 | 0.094 |
| Thermoeffekt $\frac{\eta(20°C)-\eta(50°C)}{\eta(20°C)} \times 100\%$ | | | 88.0% | 88.9% | 90.6% |
| Biegefestigkeit [MPa] | | | 135.3 | 142.1 | 139.9 |

Tabelle 10:

| Beispiel | | | 23 | 24 | 25 |
|---|---|---|---|---|---|
| (A) Monomere | (A4) | DIPENTA | 4.19 | 4.83 | 5.54 |
| | (A2) | BisGMA | 11.01 | 11.28 | 11.64 |
| | (A5) | TEGDMA | 2.27 | 1.79 | 1.29 |
| (B) Füllstoffe | (B1) | Nano-$SiO_2$ 1 | 10.00 | 10.00 | 10.00 |
| | (B2) | Dentalglas 2 | 60.33 | 59.97 | 59.50 |
| | (B3) | Dentalglas 1 | 12.07 | 11.99 | 11.90 |
| (C) Initiatoren | (C1) | CQ | 0.04 | 0.04 | 0.04 |
| | (C2) | DABE | 0.07 | 0.07 | 0.07 |
| (D) Sonstiges | (D1) | BHT | 0.02 | 0.02 | 0.02 |
| | | Gesamt | 100.00 | 100.00 | 100.00 |
| Viskosität η(20°C) [*Pa* · *s*] | | | 941 | 1156 | 1311 |
| Viskosität η(50°C) [*Pa* · *s*] | | | 131 | 139 | 147 |
| $\frac{\eta(50°C)}{\eta(20°C)}$ | | | 0.139 | 0.120 | 0.112 |
| Thermoeffekt $\frac{\eta(20°C)-\eta(50°C)}{\eta(20°C)} \times 100\%$ | | | 86.1% | 88.0% | 88.8% |
| Biegefestigkeit [MPa] | | | 121.4 | 125.3 | 122.1 |

Tabelle 11:

| Beispiel | | | 26 | 27 | 28 |
|---|---|---|---|---|---|
| (A) Monomere | (A4) | TCDDMA | 3.13 | 2.28 | 1.59 |
| | | UDMA | 3.88 | 3.73 | 3.58 |
| | (A2) | BisGMA | 13.58 | 13.88 | 13.91 |
| | (A5) | TEGDMA | 0.97 | 0.83 | 0.79 |
| (B) Füllstoffe | (B1) | Nano-$SiO_2$ 1 | 4.72 | 4.36 | 4.00 |
| | (B2) | Dentalglas 2 | 61.33 | 54.67 | 48.00 |
| | (B3) | Dentalglas 1 | 12.27 | 20.13 | 28.00 |
| (C) Initiatoren | (C1) | CQ | 0.04 | 0.04 | 0.04 |
| | (C2) | DABE | 0.07 | 0.07 | 0.07 |
| (D) Sonstiges | (D1) | BHT | 0.02 | 0.02 | 0.02 |
| | | Gesamt | 100.00 | 100.00 | 100.00 |
| Viskosität η(20°C) [*Pa* · *s*] | | | 1570 | 1588 | 1592 |
| Viskosität η(50°C) [*Pa* · *s*] | | | 149 | 128 | 102 |
| $\frac{\eta(50°C)}{\eta(20°C)}$ | | | 0.095 | 0.081 | 0.064 |

(fortgesetzt)

| Beispiel | | | 26 | 27 | 28 |
|---|---|---|---|---|---|
| Thermoeffekt $\frac{\eta(20°C)-\eta(50°C)}{\eta(20°C)} \times 100\%$ | | | 90.5% | 91.9% | 93.6% |
| Biegefestigkeit [MPa] | | | 141.1 | 137.7 | 140.5 |

Tabelle 12:

| Beispiel | | | 29 | 30 | 31 |
|---|---|---|---|---|---|
| (A) Monomere | (A4) | UDMA | 5.17 | 5.17 | 5.27 |
| | (A2) | BisGMA | 5.82 | | 5.75 |
| | | MDI-HEMA | 5.82 | 11.64 | |
| | | TMXDI-HEMA | | | 5.75 |
| | (A5) | TEGDMA | 1.66 | 1.66 | 1.70 |
| (B) Füllstoffe | (B1) | Nano-$SiO_2$ 1 | 10.00 | 10.00 | 10.00 |
| | (B2) | Dentalglas 2 | 59.50 | 59.50 | 59.50 |
| | (B3) | Dentalglas 1 | 11.90 | 11.90 | 11.90 |
| (C) Initiatoren | (C1) | CQ | 0.04 | 0.04 | 0.04 |
| | (C2) | DABE | 0.07 | 0.07 | 0.07 |
| (D) Sonstiges | (D1) | BHT | 0.02 | 0.02 | 0.02 |
| | | Gesamt | 100.00 | 100.00 | 100.00 |
| Viskosität $\eta$(20°C) [$Pa \cdot s$] | | | 1723 | 1952 | 1984 |
| Viskosität $\eta$(50°C) [$Pa \cdot s$] | | | 147 | 150 | 143 |
| $\frac{\eta(50°C)}{\eta(20°C)}$ | | | 0.085 | 0.077 | 0.072 |
| Thermoeffekt $\frac{\eta(20°C)-\eta(50°C)}{\eta(20°C)} \times 100\%$ | | | 91.5% | 92.3% | 92.8% |
| Biegefestigkeit [MPa] | | | 129 | 133 | 138 |

Tabelle 13:

| Beispiel | | | 32 | 33 | 34 |
|---|---|---|---|---|---|
| (A) Monomere | (A4) | Polysiloxan 1 | 6.17 | 9.57 | 18.12 |
| | (A2) | BisGMA | 11.64 | 9.57 | |
| | (A5) | TEGDMA | 0.66 | | |
| (B) Füllstoffe | (B1) | Nano-$SiO_2$ 1 | 9.00 | 8.99 | 8.99 |
| | (B2) | Dentalglas 2 | 60.33 | 59.78 | 60.63 |
| | (B3) | Dentalglas 1 | 12.07 | 11.96 | 12.13 |
| (C) Initiatoren | (C1) | CQ | 0.04 | 0.04 | 0.04 |
| | (C2) | DABE | 0.07 | 0.07 | 0.07 |

(fortgesetzt)

| Beispiel | | | 32 | 33 | 34 |
|---|---|---|---|---|---|
| (D) Sonstiges | (D1) | BHT | 0.02 | 0.02 | 0.02 |
| | | Gesamt | 100.00 | 100.00 | 100.00 |
| Viskosität η(20°C) [*Pa* · *s*] | | | 1665 | 2029 | 1789 |
| Viskosität η(50°C) [*Pa* · *s*] | | | 149 | 147 | 143 |
| $\frac{\eta(50°C)}{\eta(20°C)}$ | | | 0.089 | 0.072 | 0.080 |
| Thermoeffekt $\frac{\eta(20°C)-\eta(50°C)}{\eta(20°C)} \times 100\%$ | | | 91.1% | 92.8% | 92.0% |
| Biegefestigkeit [MPa] | | | 132.2 | 131.0 | 134.7 |

Tabelle 14:

| Vergleichsbeispiel | | | V1 | V2 | V3 |
|---|---|---|---|---|---|
| (A) Monomere | (A1) | TCDDMA | 12.47 | | 5.92 |
| | (A2) | BisGMA | 5.34 | | |
| | (A3) | UDMA | | 13.67 | 8.89 |
| | | BisEMA | | | |
| (B) Füllstoffe | (B1) | Nano-$SiO_2$ 1 | 13.55 | 17.68 | 13.05 |
| | (B2) | Dentalglas 2 | 57.10 | 57.12 | 60.03 |
| | (B3) | Dentalglas 1 | 11.41 | 11.39 | 11.94 |
| (C) Initiatoren | (C1) | CQ | 0.04 | 0.04 | 0.05 |
| | (C2) | DABE | 0.07 | 0.06 | 0.07 |
| (D) Sonstiges | (D1) | BHT | 0.02 | 0.04 | 0.05 |
| | | Gesamt | 100.00 | 100.00 | 100.00 |
| Viskosität η(20°C) [*Pa* · *s*] | | | 233.9 | 1522.3 | 678.8 |
| Viskosität η(50°C) [*Pa* · *s*] | | | 105.8 | 550.5 | 310.1 |
| $\frac{\eta(50°C)}{\eta(20°C)}$ | | | 0.45 | 0.36 | 0.46 |
| Thermoeffekt $\frac{\eta(20°C)-\eta(50°C)}{\eta(20°C)} \times 100\%$ | | | 54.8% | 63.8% | 54.3% |
| Biegefestigkeit [MPa] | | | 118.8 | 162.9 | 156.7 |

Tabelle 15:

| Vergleichsbeispiel | | | V4 | V5 | V6 |
|---|---|---|---|---|---|
| (A) Monomere | (A1) | TCDDMA | 7.74 | 3.98 | 9.96 |
| | (A2) | BisGMA | | | |
| | (A3) | UDMA | 11.61 | | |
| | | BisEMA | | 10.47 | 4.49 |
| (B) Füllstoffe | (B1) | Nano-$SiO_2$ 1 | 5.75 | 13.14 | 13.14 |
| | (B2) | Dentalglas 2 | 62.25 | 60.30 | 60.30 |
| | (B3) | Dentalglas 1 | 12.44 | 12.04 | 12.04 |
| (C) Initiatoren | (C1) | CQ | 0.06 | 0.02 | 0.02 |
| | (C2) | DABE | 0.09 | 0.03 | 0.03 |
| (D) Sonstiges | (D1) | BHT | 0.06 | 0.02 | 0.02 |
| | | Gesamt | 100.00 | 100.00 | 100.00 |
| Viskosität $\eta$(20°C) [$Pa \cdot s$] | | | 206.7 | 377.0 | 314.8 |
| Viskosität $\eta$(50°C) [$Pa \cdot s$] | | | 95.6 | 242.8 | 189.1 |
| $\frac{\eta(50°C)}{\eta(20°C)}$ | | | 0.46 | 0.64 | 0.60 |
| Thermoeffekt $\frac{\eta(20°C)-\eta(50°C)}{\eta(20°C)} \times 100\%$ | | | 53.7% | 35.6% | 39.9% |
| Biegefestigkeit [MPa] | | | 159.8 | 139.4 | 130.0 |

Nachfolgend werden relevante Aspekte der vorliegenden Erfindung zusammengefasst:

Aspekte:

1. Dentale, lichthärtbare, einkomponentige Kompositzusammensetzung, umfassend:

(A) Monomere,
(B) Füllstoffe, und
(C) Initiatoren,

dadurch gekennzeichnet, dass

- die Viskosität $\eta_{20}$ der Kompositzusammensetzung bei 20°C größer als 400 Pa*s ist und
- die Viskosität $\eta_{50}$ der Kompositzusammensetzung bei 50°C niedriger als 150 Pa*s ist und
- der Quotient $\eta_{50}$ / $\eta_{20}$ aus der Viskosität der Kompositzusammensetzung bei 50°C und der Viskosität der Kompositzusammensetzung bei 20°C kleiner als 0,125 ist,

2. Dentale, lichthärtbare, einkomponentige Kompositzusammensetzung nach Aspekt 1, wobei

- die Viskosität $\eta_{20}$ der Kompositzusammensetzung bei 20°C größer als 800 Pa*s, vorzugsweise größer als 1200 Pa*s ist und/oder
- die Viskosität $\eta_{50}$ der Kompositzusammensetzung bei 50°C niedriger als 120 Pa*s, vorzugsweise niedriger als 90 Pa*s ist und/oder
- der Quotient $\eta_{50}$ / $\eta_{20}$ aus der Viskosität der Kompositzusammensetzung bei 50°C und der Viskosität der Kompositzusammensetzung bei 20°C kleiner als 0,1 ist.

3. Dentale, lichthärtbare, einkomponentige Kompositzusammensetzung nach einem der vorangehenden Aspekte, wobei die Viskosität $\eta_{37}$ der Kompositzusammensetzung bei 37°C größer als 400 Pa*s ist.

4. Dentale, lichthärtbare, einkomponentige Kompositzusammensetzung nach einem der vorangehenden Aspekte, ausgewählt aus der Gruppe bestehend aus dentales Füllungsmaterial, Unterfüllungsmaterial, Befestigungsmaterial und Fissurenversiegler.

5. Dentale, lichthärtbare, einkomponentige Kompositzusammensetzung nach einem der vorangehenden Aspekte, umfassend:

(A) Monomere in einer Menge von 6 bis 35 Gew.-%, bezogen auf die Gesamtmenge der Kompositzusammensetzung, vorzugsweise 10 bis 35 Gew.-%, besonders bevorzugt 10 bis 25 Gew.-%,
(B) Füllstoffe in einer Menge von 65 bis 93 Gew.-%, bezogen auf die Gesamtmenge der Kompositzusammensetzung, vorzugsweise 65 bis 89 Gew.-%, besonders bevorzugt 75 bis 89 Gew.-%,
(C) Initiatoren in einer Menge von 0,001 bis 3 Gew.-%, bezogen auf die Gesamtmenge der Kompositzusammensetzung,
(D) weitere Additive in einer Menge von 0,001 bis 5 Gew.-% bezogen auf die Gesamtmenge der Kompositzusammensetzung.

6. Dentale, lichthärtbare, einkomponentige Kompositzusammensetzung nach einem der vorangehenden Aspekte, wobei Bestandteil (A) eine Mischung umfasst von zumindest

(A-i) einer ersten Monomerensubstanz und
(A-ii) einer zweiten Monomerensubstanz,

wobei

- die Viskosität $\eta_{20}$ der zweiten Monomerensubstanz (A-ii) bei 20 °C größer ist als 100 Pa*s,
- die Viskosität $\eta_{20}$ der ersten Monomerensubstanz (A-i) bei 20 °C größer ist als 100 mPa*s,
- die Viskosität der zweiten Monomerensubstanz (A-ii) bei 20 °C größer ist als die der ersten Monomerensubstanz (A-i) und
- das Massenverhältnis der ersten Monomerensubstanz (A-i) zur zweiten Monomerensubstanz (A-ii) im Bereich von 2:1 bis 1:10 liegt,

wobei die zweite Monomerensubstanz (A-ii) vorzugsweise zumindest 40 Gew.-% 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI) enthält, wobei die Gew.-%-Angabe auf die Gesamtmasse der Monomere (A) bezogen ist.

7. Dentale, lichthärtbare, einkomponentige Kompositzusammensetzung, vorzugsweise nach einem der vorangehenden Aspekte, wobei

die Monomere (A) bestehen aus

(A1) 10 bis 60 Gew.-%, vorzugsweise 20 bis 50 Gew.-%, besonders bevorzugt 25 bis 40 Gew.-%, lichthärtbare bi- oder tricyclische Verbindungen $Q(Y_xZ_e)_b$, wobei gilt

Q bedeutet ein gesättigtes oder olefinisch ungesättigtes bi- oder tricyclisches Strukturelement, jeder Index b ist eine natürliche Zahl, ausgewählt aus der Gruppe der natürlichen Zahlen 1, 2, und 3,
jedes Z bedeutet eine lichthärtbare Gruppe,
jeder Index e ist eine natürliche Zahl, ausgewählt aus der Gruppe der natürlichen Zahlen 1, 2 und 3,
jedes Y bedeutet in der Struktur $Q(Y_xZ_e)_b$ bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und
jeder Indexx ist 0 oder 1,

(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des

Diisocyanatodiphenylmethans (MDI) und/oder lichthärtbare Derivate des Tetramethyl-m-xylylendiisocyanats (TMXDI),
(A3) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A1) oder (A2) zuzuordnen sind,

wobei die Gew.-%-Angaben von (A1), (A2) und (A3) auf die Gesamtmasse der Monomere (A) bezogen sind.

8. Dentale, lichthärtbare, einkomponentige Kompositzusammensetzung nach Aspekt 7, wobei Q ausgewählt ist aus der Gruppe der Strukturelemente bestehend aus Bicyclo[1.1.1]pentan, Bicyclo[2.1.1]hexan, Bicyclo[2.2.1]heptan, Bicyclo[2.2.1]hepten, Bicyclo[3.1.1]heptan, Bicyclo[2.2.2]octan, Bicyclo[4.1.1]octan, Bicyclo[3.2.1]octan, Bicyclo[4.2.1]nonan, Bicyclo[3.3.1]nonan, Bicyclo[5.1.1]nonan, Bicyclo[3.2.2]nonan, Bicyclo[6.1.1]decan, Bicyclo[5.2.1]decan, Bicyclo[4.2.2]decan, Bicyclo[3.3.2]decan, Bicyclo[7.1.1]undecan, Bicyclo[6.2.1]undecan, Bicyclo[5.2.2]undecan, Bicyclo[4.3.2]undecan, Bicyclo[3.3.3]undecan, Bicyclo[8.1.1]dodecan, Bicyclo[7.2.1]dodecan, Bicyclo[6.2.2]dodecan, Bicyclo[5.3.2]dodecan, Bicyclo[4.3.3]dodecan, Bicyclo[4.4.2]dodecan, Bicyclo[5.4.1]dodecan, bicyclische Tridecane, bicyclische Tetradecane, bicyclische Pentadecane, Tricyclo[3.2.1.0$^{2,6}$]octan, Tricyclo[4.2.1.0$^{2,6}$]nonan, Tricyclo[5.2.1.0$^{2,6}$]decan, Tricyclo[6.2.1.0$^{2,6}$]undecan, Tricyclo[7.2.1.0$^{2,6}$]dodecan, Tricyclo[4.2.1.1$^{2,5}$]decan, Tricyclo[4.3.1.1 $^{2,5}$]decan, Tricyclo[4.4.1.1$^{2,5}$]decan, Tricyclo[2.2.1.0$^{2,6}$]heptan, Tricyclo[2.2.2.0$^{2,6}$]octan, Tricyclo[3.2.2.0$^{2,6}$]nonan, Tricyclo[3.3.1.1$^{3,7}$]decan, Tricyclo[3.2.1.1$^{3,7}$]nonan, Tricyclo[4.2.2.2$^{2,5}$]dodecan, Tricyclo[4.3.2.2$^{2,5}$]tridecan, Tricyclo[4.4.2.2$^{2,5}$]tetradecan, Tricyclo[4.2.1.0$^{3,7}$]nonan, Tricyclo[4.4.1.1$^{1,5}$]dodecan, Tricyclo[6.2.1.0$^{2,7}$]undecan, Tricyclo[5.2.2.0$^{2,6}$]undecan, Tricyclo[6.2.2.0$^{2,7}$]dodecan, Tricyclo[4.3.2.0$^{2,5}$]undecan, Tricyclo[4.2.2.0$^{2,5}$]decan und Tricyclo[5.5.1.0$^{3,11}$]tridecan, wobei Q vorzugsweise ausgewählt ist aus der Gruppe der Strukturelemente bestehend aus Bicyclo[2.2.1]heptan, Bicyclo[2.2.1]hept-2-en, Tricyclo[3.3.1.1$^{3,7}$]decan und Tricyclo[5.2.1.0$^{2,6}$]decan und wobei Q besonders vorzugsweise das Tricyclo[5.2.1.0$^{2,6}$]decan ist.

9. Dentale, lichthärtbare, einkomponentige Kompositzusammensetzung nach einem der Aspekte 7 bis 8, wobei die lichthärtbare Gruppe Z ein Strukturelement bedeutet, das ausgewählt ist aus der Gruppe bestehend aus $-O-(C=O)-CH=CH_2$, $-O-(C=O)-C(CH_3)=CH_2$, - $(C=O)-CH=CH_2$, $-(C=O)-C(CH_3)=CH_2$, $-CH=CH_2$, $-C(CH_3)=CH_2$, $-CH_2-CH=CH_2$, $-CH_2-C(CH_3)=CH_2$ und $-O-CH=CH_2$, bevorzugt ausgewählt ist aus der Gruppe bestehend aus $-O-(C=O)-CH=CH_2$ und $-O-(C=O)-C(CH_3)=CH_2$.

10. Dentale, lichthärtbare, einkomponentige Kompositzusammensetzung nach einem der Aspekte 7 bis 9 wobei

die Komponente (A1) das Bis(methacryloyloxymethyl)tricyclo[5.2.1.0$^{2,6}$]decan und/oder das alkoxylierte Bis(methacryloyloxymethyl)tricyclo[5.2.1.0$^{2,6}$]decan umfasst oder daraus besteht, vorzugsweise das Bis(methacryloyloxymethyl)tricyclo[5.2.1.0$^{2,6}$]decan umfasst oder daraus besteht und/oder
die Komponente (A2) Bis-GMA umfasst oder daraus besteht.

11. Dentale, lichthärtbare, einkomponentige Kompositzusammensetzung nach einem der Aspekte 7 bis 10, wobei (A3) ein oder mehrere Di(meth)acrylat-Monomere enthält, gewählt aus der Gruppe bestehend aus Ethylenglykoldi(meth)acrylat, alkoxyliertes Ethylenglykoldi(meth)acrylat, Diethylenglykoldi(meth)acrylat, 1,6-Hexandioldi(meth)acrylat, Triethylenglykoldi(meth)acrylat, 1,12-Dodecandioldi(meth)acrylat, 1,10-Decandioldi-(meth)acrylat, Bisphenol-A-di(meth)acrylat, alkoxyliertes Bisphenol-A-di(meth)acrylat, Polyethylenglykoldi(meth)acrylat, Propylenglykoldi(meth)acrylat, Dipropylenglykoldi(meth)-acrylat, Tripropylenglykoldi(meth)acrylat, Tetrapropylenglykoldi(meth)acrylat, Polypropylen-glykoldi(meth)acrylat, 7,7,9-Trimethyl-3,14-dioxa-4,13-dioxo-5,12-diazahexadecan-1,16-dioxydi(meth)acrylat, Butandioldi(meth)acrylat, Propandioldi(meth)acrylat, Tetraethylen-glykoldi(meth)acrylat, Neopentylglykoldi(meth)acrylat, alkoxyliertes Neopentylglykoldi(meth)acrylat, 2-Hydroxypropyl-1,3-di(meth)acrylat, 3-Hydroxypropyl-1,2-di(meth)acrylat, Pentaerythritoldi(meth)acrylat, alkoxyliertes Pentaerythritoldi(meth)acrylat, Pentaerythritoltri(meth)acrylat, alkoxyliertes Pentaerythritoltri(meth)acrylat, Pentaerythritoltetra(meth)acrylat, alkoxyliertes Pentaerythritoltetra(meth)acrylat, Dipentaerythritoldi(meth)acrylat, alkoxyliertes Dipentaerythritoldi(meth)acrylat, Dipentaerythritoltri(meth)acrylat, alkoxyliertes Dipentaerythritoltri(meth)acrylat, Dipentaerythritoltetra(meth)acrylat, alkoxyliertes Dipentaerythritoltetra(meth)acrylat, Dipentaerythritolpenta(meth)acrylat, alkoxyliertes Dipentaerythritolpenta(meth)acrylat, Dipentaerythritolhexa(meth)acrylat, alkoxyliertes Dipentaerythritolhexa(meth)acrylat, Trimethylolpropantri(meth)acrylat, alkoxyliertes Trimethylolpropantri(meth)acrylat und Cyclohexandimethanoldi(meth)acrylat.

12. Dentale, lichthärtbare, einkomponentige Kompositzusammensetzung nach einem der Aspekte 1 - 6, wobei die

Monomere (A) bestehen aus (A4) 10 bis 60 Gew.-%, vorzugsweise 20 bis 50 Gew.-%, besonders bevorzugt 25 bis 40 Gew.-% eines oder mehrerer Verbindungen ausgewählt aus der Gruppe bestehend aus

- lichthärtbare bi- oder tricyclische Verbindungen $Q(Y_xZ_e)_b$, wobei gilt Q bedeutet ein gesättigtes oder olefinisch ungesättigtes bi- oder tricyclisches Strukturelement,

  jeder Index b ist eine natürliche Zahl, ausgewählt aus der Gruppe der natürlichen Zahlen 1, 2, und 3,
  jedes Z bedeutet eine lichthärtbare Gruppe,
  jeder Index e ist eine natürliche Zahl, ausgewählt aus der Gruppe der natürlichen Zahlen 1, 2 und 3,
  jedes Y bedeutet in der Struktur $Q(Y_xZ_e)_b$ bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und
  jeder Indexx ist 0 oder 1,

- 7,7,9-Trimethyl-3,14-dioxa-4,13-dioxo-5,12-diazahexadecan-1,16-dioxydi(meth)acrylat (UDMA),
- alkoxylierte Bisphenol - A - di(meth)acrylate mit 2 bis 6 Alkoxyeinheiten,
- hydroxylgruppenhaltige Poly(meth)acrylate, ausgewählt aus der Gruppe bestehend aus Dipentaerythritoldi(meth)acrylat, alkoxyliertes Dipentaerythritoldi(meth)acrylat, Dipentaerythritoltri(meth)acrylat, alkoxyliertes Dipentaerythritoltri(meth)acrylat, Dipentaerythritoltetra(meth)acrylat, alkoxyliertes Dipentaerythritoltetra(meth)acrylat, Dipentaerythritolpenta(meth)acrylat, alkoxyliertes Dipentaerythritolpenta(meth)acrylat, Pentaerythritoldi(meth)acrylat, alkoxyliertes Pentaerythritoldi(meth)acrylat, Pentaerythritoltri(meth)acrylat, alkoxyliertes Pentaerythritoltri(meth)acrylat, und
- lichthärtbare, kettenförmige und/oder ringförmige und/oder käfigförmige Polysiloxane, (A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),

  (A5) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A4) oder (A2) zuzuordnen sind,
  wobei die Gew.-%-Angaben von (A4), (A2) und (A5) auf die Gesamtmasse der Monomere (A) bezogen sind.

13. Dentale, lichthärtbare, einkomponentige Kompositzusammensetzung nach einem der Aspekte 1 - 6, wobei die Monomere (A) bestehen aus

(A4) 10 bis 60 Gew.-%, vorzugsweise 20 bis 50 Gew.-%, besonders bevorzugt 25 bis 40 Gew.-% eines oder mehrerer Verbindungen ausgewählt aus der Gruppe bestehend aus

- lichthärtbare bi- oder tricyclische Verbindungen $Q(Y_xZ_e)_b$, wobei gilt Q bedeutet ein gesättigtes oder olefinisch ungesättigtes bi- oder tricyclisches Strukturelement,

  jeder Index b ist eine natürliche Zahl, ausgewählt aus der Gruppe der natürlichen Zahlen 1, 2, und 3,
  jedes Z bedeutet eine lichthärtbare Gruppe,
  jeder Index e ist eine natürliche Zahl, ausgewählt aus der Gruppe der natürlichen Zahlen 1, 2 und 3,
  jedes Y bedeutet in der Struktur $Q(Y_xZ_e)_b$ bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und
  jeder Indexx ist 0 oder 1,

- 7,7,9-Trimethyl-3,14-dioxa-4,13-dioxo-5,12-diazahexadecan-1,16-dioxydi(meth)acrylat (UDMA),
- 7,9,9-Trimethyl-3,14-dioxa-4,13-dioxo-5,12-diazahexadecan-1,16-dioxydi(meth)acrylat
- 7,9-Dimethyl-3,14-dioxa-4,13-dioxo-5,12-diazahexadecan-1,16-dioxydi(meth)acrylat
- 3,14-Dioxa-4,13-dioxo-5,12-diazahexadecan-1,16-dioxydi(meth)acrylat
- 1 ,5,5- Trimethyl-1-[(2-methacryloyloxyethyl)carbamoylmethyl]-3-(2-methacryloyloxyethyl)carbamoylcyclohexan
- 7,7,9,9-Tetramethyl-3,14-dioxa-4,13-dioxo-5,12-diazahexadecan-1,16-dioxydi(meth)acrylat
- 2,7,7,9,15-Pentamethyl-3,14-dioxa-4,13-dioxo-5,12-diazahexadecan-1,16-dioxydi(meth)acrylat
- 2,7,9,9,15-Pentamethyl-3,14-dioxa-4,13-dioxo-5,12-diazahexadecan-1,16-dioxydi(meth)acrylat

- 2,7,9,15-Tetramethyl-3,14-dioxa-4,13-dioxo-5,12-diazahexadecan-1,16-dioxydi(meth)acrylat
- 2,15-Dimethyl-3,14-dioxa-4,13-dioxo-5,12-diazahexadecan-1, 16-dioxydi(meth)acrylat
- 1,5,5-Trimethyl-1-[(1-methacryloyloxypropan-2-yl)carbamoylmethyl]-3-(1-methacryloyloxypropan-2-yl)carbamoylcyclohexan
- 2,7,7,9,9,15-Hexamethyl-3,14-dioxa-4,13-dioxo-5,12-diazahexadecan-1,16-dioxydi(meth)acrylat,
- alkoxylierte Bisphenol -A - di(meth)acrylate mit 2 bis 6 Alkoxyeinheiten,
- hydroxylgruppenhaltige Poly(meth)acrylate, ausgewählt aus der Gruppe bestehend aus Dipentaerythritoldi(meth)acrylat, alkoxyliertes Dipentaerythritoldi(meth)acrylat, Dipentaerythritoltri(meth)acrylat, alkoxyliertes Dipentaerythritoltri(meth)acrylat, Dipentaerythritoltetra(meth)acrylat, alkoxyliertes Dipentaerythritoltetra(meth)acrylat, Dipentaerythritolpenta(meth)acrylat, alkoxyliertes Dipentaerythritolpenta(meth)acrylat, Pentaerythritoldi(meth)acrylat, alkoxyliertes Pentaerythritoldi(meth)acrylat, Pentaerythritoltri(meth)acrylat, alkoxyliertes Pentaerythritoltri(meth)acrylat, und
- lichthärtbare, kettenförmige und/oder ringförmige und/oder käfigförmige Polysiloxane,

(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),
(A5) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A4) oder (A2) zuzuordnen sind, wobei die Gew.-%-Angaben von (A4), (A2) und (A5) auf die Gesamtmasse der Monomere (A) bezogen sind.

14. Dentale, lichthärtbare, einkomponentige Kompositzusammensetzung nach einem der Aspekte 1 bis 5, wobei die Komponente A mehr als 60 Gew.-% die lichthärtbaren, kettenförmigen und/oder ringförmigen und/oder käfigförmigen Polysiloxane umfaßt.

15. Dentale, lichthärtbare, einkomponentige Kompositzusammensetzung nach einem der vorangehenden Aspekte, wobei

die Füllstoffe (B) bestehen aus und/oder herstellbar sind durch Vermischen von (B1) 2 bis 25 Gew.-%, vorzugsweise 3 bis 20 Gew.-%, anorganischem Füllstoff mit einem $D_{50}$-Wert von 1 nm bis 200 nm,
und weiteren Füllstoffbestandteilen, vorzugsweise
(B2) 40 bis 90 Gew.-%, vorzugsweise 50 bis 80 Gew.-%, anorganischem Füllstoff mit einem $D_{50}$-Wert von größer 1 $\mu$m bis 10 $\mu$m,
(B3) 8 bis 50 Gew.-%, vorzugsweise 15 bis 40 Gew.-%, anorganischem Füllstoff in einer Größe mit einem $D_{50}$-Wert von 0,4 $\mu$m bis 1,0 $\mu$m und
(B4) 0 bis 25 Gew.-%, vorzugsweise 0 bis 15 Gew.-%, weitere Füllstoffe, die nicht (B1), (B2) oder (B3) zuzuordnen sind,
wobei die Gew.-%-Angaben von (B1), (B2), (B3) und (B4) auf die Gesamtmasse der Füllstoffe (B) bezogen sind.

16. Dentale, lichthärtbare, einkomponentige Kompositzusammensetzung nach Aspekt 15, wobei

die Partikel des anorganischen Füllstoffs (B1) nicht aggregiert und nicht agglomeriert sind und/oder
der anorganische Füllstoff (B1) in einer Größe mit einem $D_{50}$-Wert unter 100 nm, vorzugsweise unter 70 nm vorliegt
und/oder
der anorganische Füllstoff (B2) in einer Größe mit einem $D_{50}$-Wert von 1,2 $\mu$m bis 5,0 $\mu$m, vorzugsweise von 1,5 $\mu$m bis 4,0 $\mu$m vorliegt
und/oder
der anorganische Füllstoff (B3) in einer Größe mit einem $D_{50}$-Wert von 0,5 $\mu$m bis 0,9 $\mu$m und vorzugsweise von 0,6 $\mu$m bis 0,8 vorliegt.

17. Dentale, lichthärtbare, einkomponentige Kompositzusammensetzung nach einem der Aspekte 15 oder 16, wobei (B1)

- Oxide oder Mischoxide ausgewählt aus der Gruppe bestehend aus den Elementen Silizium, Titan, Yttrium, Barium, Zirkonium, Hafnium, Niob, Tantal, Wolfram, Wismut, Molybdän, Zinn, Zink, Ytterbium, Lanthan, Cer, Aluminium und deren Mischungen, vorzugsweise Kieselsäure und/oder
- Sulfide, Selenide und Telluride von Metallen, Mischmetallen und deren Mischungen und/oder

- Salze der Seltenen Erden, des Scandiums und des Yttriums, vorzugsweise Ytterbiumfluorid und/oder
- Salze des Bariums und des Strontiums, vorzugsweise Bariumsulfat und/oder Strontiumfluorid und/oder
- Mischfluoride zwischen Ytterbiumfluorid und Strontiumfluorid, vorzugsweise das Strontiumfluorid-dotierte Ytterbiumfluorid und/oder das Ytterbiumfluorid dotierte Strontiumfluorid

enthält.

18. Dentale, lichthärtbare, einkomponentige Kompositzusammensetzung nach einem der Aspekte 15 bis 17, wobei die Bestandteile (B2) und (B3) umfassen:

- Materialien auf der Basis von Oxiden oder Mischoxiden aus $SiO_2$, $ZrO_2$, $TiO_2$ und/oder
- Quarz-Glaskeramik oder Glaspulver, Bariumsilikatgläser, Bariumfluorsilikatgläser, Strontiumsilikatgläser, Strontiumborsilikate, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate und/oder
- Metallsalze, vorzugsweise Bariumsulfat oder Calciumfluorid und/oder
- Ytterbiumfluorid.

19. Dentale, lichthärtbare, einkomponentige Kompositzusammensetzung nach einem der Aspekte 15 bis 18,

wobei das Verhältnis der Gesamtmasse von (B2) zu (B3) im Bereich von 1 : 1 bis 12 : 1 liegt, vorzugsweise im Bereich von 1,5 : 1 bis 8 : 1 und/oder
wobei das Verhältnis der mittleren Korngröße von (B2) zur mittleren Korngröße von (B3) im Bereich von 1,5 : 1 bis 10 : 1 liegt, vorzugsweise im Bereich von 2 : 1 bis 5 : 1.

20. Dentale, lichthärtbare, einkomponentige Kompositzusammensetzung nach einem der Aspekte 15 bis 19, wobei (B4)

- verstärkend wirkende Füllstoff-Materialien, vorzugsweise Glasfasern, Polyamid- oder Kohlenstofffasern und/oder
- weitere anorganische Füllstoffe
  und/oder
- Splitter- oder Perlpolymerisate, vorzugsweise Perlpolymerisate von Homo- oder Copolymeren organisch härtbarer Monomere,
  umfasst.

21. Dentale, lichthärtbare, einkomponentige Kompositzusammensetzung nach einem der Aspekte 15 bis 20, wobei die Komponenten (B1) und/oder (B2) und/oder (B3) und/oder (B4) organisch oberflächenmodifiziert, vorzugsweise silanisiert, sind.

22. Dentale, lichthärtbare, einkomponentige Kompositzusammensetzung nach einem der Aspekte 15 bis 21, wobei die Komponenten (B1) und/oder (B2) und/oder (B3) und/oder (B4) durch Verbindungen der allgemeinen Formel X-Sp-V oberflächenmodifiziert sind,
wobei "X" und "V" funktionelle Gruppen bedeuten, die durch einen Linker "Sp" miteinander verbunden sind und wobei

- die funktionelle Gruppe "X" so ausgewählt ist, dass sie unter Komplexbildung eine entsprechende Bindung mit der Oberfläche des Füllstoffpartikels eingehen kann, vorzugsweise eine Gruppe des Typs Silan, Phosphat, Phosphonat, Carboxylat, Dithiophosphat, Dithiophosphonat, Amin oder Amid ist und
- der Linker "Sp" lineare oder verzweigte Alkylketten, Aromaten oder Kombinationen dieser Gruppen aufweist, die jeweils durch Heteroatome wie O, N, S oder P oder durch eine Urethangruppe unterbrochen sein können und
- die funktionelle Gruppe "V" lichthärtbare Gruppen, vorzugsweise (Meth)acrylatgruppen aufweist.

23. Dentale, lichthärtbare, einkomponentige Kompositzusammensetzung nach einem der Aspekte 15 bis 22, wobei die Komponenten (B1) und/oder (B2) und/oder (B3) und/oder (B4) mit einer Verbindung der Formeln (1) oder (2) silanisiert sind

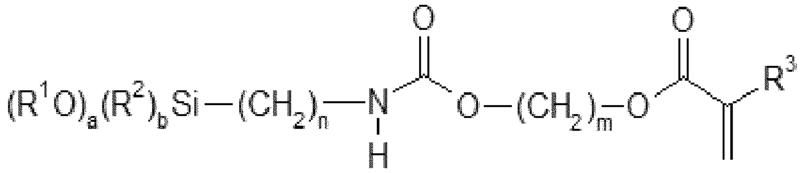

(1)

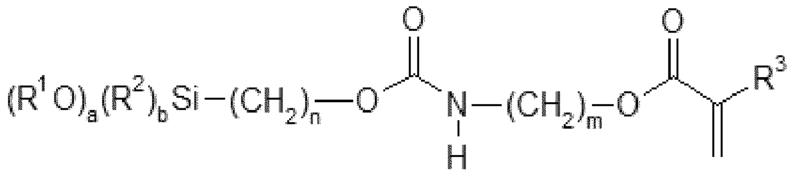

(2)

wobei $R^1$ eine C1- bis C4-Alkylgruppe bedeutet, und
$R^2$ eine C1- bis C8-Alkylgruppe bedeutet, und
$R^3$ ein Wasserstoffatom oder eine Methylgruppe bedeutet, und
a = 1, 2 oder 3, und
b = 3 - a, und
n = 1 bis 8, und
m = 1 bis 8 ist.

24. Gehärtete Kompositzusammensetzung, erhältlich durch Lichthärten einer dentalen, lichthärtbaren, einkomponentigen Kompositzusammensetzung nach einem der vorangehenden Aspekte.

25. Dentale, lichthärtbare, einkomponentige Kompositzusammensetzung nach einem der Aspekte 1 bis 23, zur Anwendung in einem dentalen Therapieverfahren, vorzugsweise zur Anwendung in einem dentalen Therapieverfahren mit folgenden Schritten:

- Erwärmen der Kompositzusammensetzung auf eine Temperatur von 40 °C oder mehr, vorzugsweise eine Temperatur im Bereich von 40 °C bis 80 °C, vorzugsweise in einem Ofen und/oder durch Bestrahlung, vorzugsweise mit IR-Strahlen,
- Kontaktieren der auf eine Temperatur von 40 °C oder mehr, vorzugsweise eine Temperatur im Bereich von 40 °C bis 80 °C, erwärmten Kompositzusammensetzung mit einem zu behandelnden Zahn eines Patienten, vorzugsweise als dentales Füllungsmaterial, Unterfüllungsmaterial, Befestigungsmaterial und Fissurenversiegler.

26. Verwendung einer Kompositzusammensetzung nach einem der Aspekte 1 bis 23, zur Herstellung eines dentalen Erzeugnisses, wobei die Herstellung nicht am menschlichen oder tierischen Körper erfolgt.

27. Vorrichtung zur Applikation einer Kompositzusammensetzung, umfassend einen Hohlraum, der zumindest teilweise mit einer Menge einer Kompositzusammensetzung nach einem der Aspekte 1 bis 23 gefüllt ist
und
eine mit dem Hohlraum verbundene Applikationsspitze mit einer Austrittsöffnung für die Kompositzusammensetzung.

28. Vorrichtung nach Aspekt 27 wobei die Austrittsöffnung eine Austrittsquerschnittsfläche im Bereich von 0,2 bis 3,0 $mm^2$ besitzt, vorzugsweise eine Austrittsquerschnittsfläche von nicht mehr als 2,0 $mm^2$, besonders bevorzugt eine Austrittsquerschnittsfläche von nicht mehr als 1,5 $mm^2$, besitzt.

29. Vorrichtung nach einem der Aspekte 27 oder 28, wobei

die Applikationsspitze eine vorzugsweise aus Metall oder Kunststoff bestehende Kanüle ist und/oder
die Vorrichtung ausgewählt ist aus der Gruppe bestehend aus Compulen und Spritzen.

30. Verfahren zur Herstellung einer Kompositzusammensetzung nach einem der Aspekte 1 bis 23 oder zur Herstellung einer Vorrichtung zur Applikation einer Kompositzusammensetzung nach einem der Aspekte 27 bis 29, mit

folgendem Schritt: Vermischen der Bestandteile

(A) Monomere in einer Menge von 6 bis 35 Gew.-%, bezogen auf die Gesamtmenge der Kompositzusammensetzung, vorzugsweise 10 bis 35 Gew.-%, besonders bevorzugt 10 bis 25 Gew.-%,
(B) Füllstoffe in einer Menge von 65 bis 93 Gew.-%, vorzugsweise 65 bis 89 Gew.-%, besonders bevorzugt 75 bis 89 Gew.-%, bezogen auf die Gesamtmenge der Kompositzusammensetzung,
(C) Initiatoren in einer Menge von 0,001 bis 3 Gew.-% bezogen auf die Gesamtmenge der Kompositzusammensetzung,
(D) weitere Additive in einer Menge von 0,001 bis 5 Gew.-% bezogen auf die Gesamtmenge der Kompositzusammensetzung,

zu der Kompositzusammensetzung,
wobei die Bestandteile so ausgewählt sind, dass die Viskosität $\eta_{20}$ der Kompositzusammensetzung bei 20°C größer als 400 Pa*s ist und die Viskosität $\eta_{50}$ der Kompositzusammensetzung bei 50°C niedriger als 150 Pa*s ist und wobei der Quotient $\eta_{50}$ / $\eta_{20}$ aus der Viskosität der Kompositzusammensetzung bei 50°C und der Viskosität der Kompositzusammensetzung bei 20°C kleiner als 0,125 ist, vorzugsweise kleiner als 0,1 ist.

31. Verfahren nach Aspekt 30, wobei

Bestandteil (A) hergestellt wird mit den folgenden Schritten:
Bereitstellen von zumindest

(A-i) einer ersten Monomerensubstanz und
(A-ii) einer zweiten Monomerensubstanz,
wobei

- die Viskosität $\eta_{20}$ der zweiten Monomerensubstanz (A-ii) bei 20 °C größer ist als 100 Pa*s,
- die Viskosität $\eta_{20}$ der ersten Monomerensubstanz (A-i) bei 20 °C größer ist als 100 mPa*s,
- die Viskosität der zweiten Monomerensubstanz (A-ii) bei 20 °C größer ist als die der ersten Monomerensubstanz (A-i) und
- das Massenverhältnis der ersten Monomerensubstanz (A-i) zur zweiten Monomerensubstanz (A-ii) im Bereich von 2:1 bis 1:10 liegt,

wobei die zweite Monomerensubstanz (A-ii) vorzugsweise zumindest 40 Gew.-% 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI) enthält, wobei die Gew.-%-Angabe auf die Gesamtmasse der Monomere (A) bezogen ist
und
Mischen der bereitgestellten Monomerensubstanzen (A-i) und (A-ii), vor dem und/oder beim Mischen mit den Bestandteilen (B), (C) und (D)

und/oder wobei
Bestandteil (B) hergestellt wird durch Vermischen von

(B1) 2 bis 25 Gew.-%, vorzugsweise 3 bis 20 Gew.-%, anorganischem Füllstoff mit einem $D_{50}$-Wert von 1 nm bis 200 nm,
und weiteren Füllstoffbestandteilen, vorzugsweise (B2) 40 bis 90 Gew.-%, vorzugsweise 50 bis 80 Gew.-%, anorganischem Füllstoff mit einem $D_{50}$-Wert von größer 1 $\mu$m bis 10 $\mu$m,
(B3) 8 bis 50 Gew.-%, vorzugsweise 15 bis 40 Gew.-%, anorganischem Füllstoff in einer Größe mit einem $D_{50}$-Wert von 0,4 $\mu$m bis 1,0 $\mu$m und
(B4) 0 bis 25 Gew.-%, vorzugsweise 0 bis 15 Gew.-%, weitere Füllstoffe, die nicht (B1), (B2) oder (B3) zuzuordnen sind,

wobei die Gew.-%-Angaben von (B1), (B2), (B3) und (B4) auf die Gesamtmasse der Füllstoffe (B) bezogen sind.

32. Verfahren nach einem der Aspekte 30 bis 31, mit folgendem zusätzlichen Schritt zur Herstellung der Vorrichtung zur Applikation einer Kompositzusammensetzung:

- Einfüllen der hergestellten Kompositzusammensetzung nach einem der Aspekte 1 bis 23 in einen Hohlraum einer zuvor nicht gefüllten Vorrichtung zur Applikation einer Kompositzusammensetzung.

33. Verfahren zum Vorbereiten einer dentalen Behandlung eines Patienten, mit folgendem Schritt:
Erwärmen einer Kompositzusammensetzung nach einem der Aspekte 1 bis 23 oder einer Vorrichtung nach einem der Aspekte 27 bis 29, vorzugsweise hergestellt gemäß einem Verfahren nach Aspekt 30 bis 32, auf eine Temperatur von 40 °C oder mehr, vorzugsweise eine Temperatur im Bereich von 40 °C bis 80 °C, vorzugsweise in einem Ofen und/oder durch Bestrahlung, vorzugsweise mit IR-Strahlen.

34. Anwendung einer dentalen, lichthärtbaren, einkomponentigen Kompositzusammensetzung nach einem der Aspekte 1 bis 23 in einem dentalen Therapieverfahren,
vorzugsweise zur Anwendung in einem dentalen Therapieverfahren mit folgenden Schritten:

- Erwärmen der Kompositzusammensetzung auf eine Temperatur von 40 °C oder mehr, vorzugsweise eine Temperatur im Bereich von 40 °C bis 80 °C, vorzugsweise in einem Ofen und/oder durch Bestrahlung, vorzugsweise mit IR-Strahlen,
- Kontaktieren der auf eine Temperatur von 40 °C oder mehr, vorzugsweise eine Temperatur im Bereich von 40 °C bis 80 °C, erwärmten Kompositzusammensetzung mit einem zu behandelnden Zahn eines Patienten, vorzugsweise als dentales Füllungsmaterial, Unterfüllungsmaterial, Befestigungsmaterial und Fissurenversiegler.

## Patentansprüche

**1.** Dentale, lichthärtbare, einkomponentige Kompositzusammensetzung, umfassend:

(A) Monomere,
(B) Füllstoffe, und
(C) Initiatoren,

**dadurch gekennzeichnet, dass**

- die Viskosität $\eta_{20}$ der Kompositzusammensetzung bei 20°C größer als 400 Pa*s ist
und
- die Viskosität $\eta_{50}$ der Kompositzusammensetzung bei 50°C niedriger als 150 Pa*s ist
und
- der Quotient $\eta_{50}$ / $\eta_{20}$ aus der Viskosität der Kompositzusammensetzung bei 50°C und der Viskosität der Kompositzusammensetzung bei 20°C kleiner als 0,125 ist,

**2.** Dentale, lichthärtbare, einkomponentige Kompositzusammensetzung nach Anspruch 1, wobei

- die Viskosität $\eta_{20}$ der Kompositzusammensetzung bei 20°C größer als 800 Pa*s, vorzugsweise größer als 1200 Pa*s ist und/oder
- die Viskosität $\eta_{50}$ der Kompositzusammensetzung bei 50°C niedriger als 120 Pa*s, vorzugsweise niedriger als 90 Pa*s ist und/oder
- der Quotient $\eta_{50}$ / $\eta_{20}$ aus der Viskosität der Kompositzusammensetzung bei 50°C und der Viskosität der Kompositzusammensetzung bei 20°C kleiner als 0,1 ist
und/oder

wobei
die Viskosität $\eta_{37}$ der Kompositzusammensetzung bei 37°C größer als 400 Pa*s ist.

**3.** Dentale, lichthärtbare, einkomponentige Kompositzusammensetzung nach einem der vorangehenden Ansprüche, ausgewählt aus der Gruppe bestehend aus dentales Füllungsmaterial, Unterfüllungsmaterial, Befestigungsmaterial und Fissurenversiegler.

**4.** Dentale, lichthärtbare, einkomponentige Kompositzusammensetzung nach einem der vorangehenden Ansprüche, umfassend:

(A) Monomere in einer Menge von 6 bis 35 Gew.-%, bezogen auf die Gesamtmenge der Kompositzusammensetzung, vorzugsweise 10 bis 35 Gew.-%, besonders bevorzugt 10 bis 25 Gew.-%,
(B) Füllstoffe in einer Menge von 65 bis 93 Gew.-%, bezogen auf die Gesamtmenge der Kompositzusammensetzung, vorzugsweise 65 bis 89 Gew.-%, besonders bevorzugt 75 bis 89 Gew.-%,
(C) Initiatoren in einer Menge von 0,001 bis 3 Gew.-%, bezogen auf die Gesamtmenge der Kompositzusammensetzung,
(D) weitere Additive in einer Menge von 0,001 bis 5 Gew.-%, bezogen auf die Gesamtmenge der Kompositzusammensetzung
und/oder

wobei

Bestandteil (A) eine Mischung umfasst von zumindest
(A-i) einer ersten Monomerensubstanz und
(A-ii) einer zweiten Monomerensubstanz,
wobei

- die Viskosität $\eta_{20}$ der zweiten Monomerensubstanz (A-ii) bei 20 °C größer ist als 100 Pa*s,
- die Viskosität $\eta_{20}$ der ersten Monomerensubstanz (A-i) bei 20 °C größer ist als 100 mPa*s,
- die Viskosität der zweiten Monomerensubstanz (A-ii) bei 20 °C größer ist als die der ersten Monomerensubstanz (A-i) und
- das Massenverhältnis der ersten Monomerensubstanz (A-i) zur zweiten Monomerensubstanz (A-ii) im Bereich von 2:1 bis 1:10 liegt,

wobei die zweite Monomerensubstanz (A-ii) vorzugsweise zumindest 40 Gew.-% 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI) enthält, wobei die Gew.-%-Angabe auf die Gesamtmasse der Monomere (A) bezogen ist.

**5.** Dentale, lichthärtbare, einkomponentige Kompositzusammensetzung nach einem der vorangehenden Ansprüche, wobei

die Monomere (A) bestehen aus
(A1) 10 bis 60 Gew.-%, vorzugsweise 20 bis 50 Gew.-%, besonders bevorzugt 25 bis 40 Gew.-%, lichthärtbare bi- oder tricyclische Verbindungen $Q(Y_xZ_e)_b$, wobei gilt Q bedeutet ein gesättigtes oder olefinisch ungesättigtes bi- oder tricyclisches Strukturelement,
jeder Index b ist eine natürliche Zahl, ausgewählt aus der Gruppe der natürlichen Zahlen 1, 2, und 3,
jedes Z bedeutet eine lichthärtbare Gruppe,
jeder Index e ist eine natürliche Zahl, ausgewählt aus der Gruppe der natürlichen Zahlen 1, 2 und 3,
jedes Y bedeutet in der Struktur $Q(Y_xZ_e)_b$ bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und
jeder Indexx ist 0 oder 1,
(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),
(A3) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A1) oder (A2) zuzuordnen sind,
wobei die Gew.-%-Angaben von (A1), (A2) und (A3) auf die Gesamtmasse der Monomere (A) bezogen sind,
wobei vorzugsweise

Q ausgewählt ist aus der Gruppe der Strukturelemente bestehend aus Bicyclo[1.1.1]pentan, Bicyclo[2.1.1]hexan, Bicyclo[2.2.1]heptan, Bicyclo[2.2.1]hepten, Bicyclo[3.1.1]heptan, Bicyclo[2.2.2]octan, Bicyclo[4.1.1]octan, Bicyclo[3.2.1]octan, Bicyclo[4.2.1]nonan, Bicyclo[3.3.1]nonan, Bicyclo[5.1.1]nonan, Bicyclo[3.2.2]nonan, Bicyclo[6.1.1]decan, Bicyclo[5.2.1]decan, Bicyclo[4.2.2]decan, Bicyclo[3.3.2]decan, Bicyclo[7.1.1]undecan, Bicyclo[6.2.1]undecan, Bicyclo[5.2.2]undecan, Bicyclo[4.3.2]undecan, Bicyclo[3.3.3]undecan, Bicyclo[8.1.1]dodecan, Bicyclo[7.2.1]dodecan, Bicyclo[6.2.2]dodecan-, Bicyclo[5.3.2]dodecan, Bicyclo[4.3.3]dodecan, Bicyclo[4.4.2]dodecan, Bicyclo[5.4.1]dodecan, bicyclische Tridecane, bicyclische Tetradecane, bicyclische Pentadecane, Tricyclo[3.2.1.0$^{2,6}$]octan, Tricyclo[4.2.1.0$^{2,6}$]nonan, Tricyclo[5.2.1.0$^{2,6}$]decan, Tricyclo[6.2.1.0$^{2,6}$]undecan, Tricyclo[7.2.1.0$^{2,6}$]dodecan, Tricyclo[4.2.1.1$^{2,5}$]decan, Tricyclo[4.3.1.1$^{2,5}$]decan, Tricyclo[4.4.1.1$^{2,5}$]decan, Tricyclo[2.2.1.0$^{2,6}$]heptan, Tricyclo[2.2.2.0$^{2,6}$]octan, Tricyclo[3.2.2.0$^{2,6}$]nonan, Tricyclo[3.3.1.1$^{3,7}$]decan, Tricyclo[3.2.1.1$^{3,7}$]nonan, Tricyclo[4.2.2.2$^{2,5}$]dodecan, Tricyclo[4.3.2.2$^{2,5}$]tridecan, Tricyclo[4.4.2.2$^{2,5}$]tetradecan, Tricyclo[4.2.1.0$^{3,7}$]nonan, Tricyclo[4.4.1.1$^{1,5}$]dodecan, Tricyclo[6.2.1.0$^{2,7}$]undecan, Tricyclo[5.2.2.0$^{2,6}$]undecan, Tricyclo[6.2.2.0$^{2,7}$]dodecan, Tricyclo[4.3.2.0$^{2,5}$]undecan, Tricyclo[4.2.2.0$^{2,5}$]decan und Tricyclo[5.5.1.0$^{3,11}$]tridecan, wobei
Q vorzugsweise ausgewählt ist aus der Gruppe der Strukturelemente bestehend aus Bicyclo[2.2.1]heptan, Bicyclo[2.2.1]hept-2-en, Tricyclo[3.3.1.1$^{3,7}$]decan und Tricyclo[5.2.1.0$^{2,6}$]decan und wobei Q besonders vorzugsweise das Tricyclo[5.2.1.0$^{2,6}$]decan ist
und/oder
die lichthärtbare Gruppe Z ein Strukturelement bedeutet, das ausgewählt ist aus der Gruppe bestehend aus $-O-(C=O)-CH=CH_2$, $-O-(C=O)-C(CH_3)=CH_2$, $-(C=O)-CH=CH_2$, - $(C=O)-C(CH_3)=CH_2$, $-CH=CH_2$, $-C(CH_3)=CH_2$, $-CH_2-CH=CH_2$, $-CH_2-C(CH_3)=CH_2$ und - $O-CH=CH_2$, bevorzugt ausgewählt ist aus der Gruppe bestehend aus $-O-(C=O)-CH=CH_2$ und $-O-(C=O)-C(CH_3)=CH_2$
und/oder
die Komponente (A1) das Bis(methacryloyloxymethyl)tricyclo[5.2.1.0$^{2,6}$]decan und/oder das alkoxylierte Bis(methacryloyloxymethyl)tricyclo[5.2.1.0$^{2,6}$]decan umfasst oder daraus besteht, vorzugsweise das Bis(methacryloyloxymethyl)tricyclo[5.2.1.0$^{2,6}$]decan umfasst oder daraus besteht und/oder
die Komponente (A2) Bis-GMA umfasst oder daraus besteht
und/oder
(A3) ein oder mehrere Di(meth)acrylat-Monomere enthält, gewählt aus der Gruppe bestehend aus Ethylenglykoldi(meth)acrylat, alkoxyliertes Ethylenglykoldi(meth)acrylat, Diethylenglykoldi(meth)acrylat, 1,6-Hexandioldi(meth)acrylat, Triethylenglykoldi(meth)acrylat, 1,12-Dodecandioldi(meth)acrylat, 1,10-Decandioldi-(meth)acrylat, Bisphenol-A-di(meth)acrylat, alkoxyliertes Bisphenol-A-di(meth)acrylat, Polyethylenglykoldi(meth)acrylat, Propylenglykoldi(meth)acrylat, Dipropylenglykoldi-(meth)acrylat, Tripropylenglykoldi(meth)acrylat, Tetrapropylenglykoldi(meth)acrylat, Polypropylenglykoldi(meth)acrylat, 7,7,9-Trimethyl-3,14-dioxa-4,13-dioxo-5,12-diazahexadecan-1, 16-dioxydi(meth)acrylat, Butandioldi(meth)acrylat, Propandioldi(meth)acrylat, Tetraethylenglykoldi(meth)acrylat, Neopentylglykol-di(meth)acrylat, alkoxyliertes Neopentylglykoldi(meth)acrylat, 2-Hydroxypropyl-1,3-di(meth)acrylat, 3-Hydroxypropyl-1,2-di(meth)acrylat, Pentaerythritoldi(meth)acrylat, alkoxyliertes Pentaerythritoldi(meth)acrylat, Pentaerythritoltri(meth)acrylat, alkoxyliertes Pentaerythritoltri(meth)acrylat, Pentaerythritoltetra(meth)acrylat, alkoxyliertes Pentaerythritoltetra(meth)acrylat, Dipentaerythritoldi(meth)acrylat, alkoxyliertes Dipentaerythritoldi(meth)acrylat, Dipentaerythritoltri(meth)acrylat, alkoxyliertes Dipentaerythritoltri(meth)acrylat, Dipentaerythritoltetra(meth)acrylat, alkoxyliertes Dipentaerythritoltetra(meth)acrylat, Dipentaerythritolpenta(meth)acrylat, alkoxyliertes Dipentaerythritolpenta(meth)acrylat, Dipentaerythritolhexa(meth)acrylat, alkoxyliertes Dipentaerythritolhexa(meth)acrylat, Trimethylolpropantri(meth)acrylat, alkoxyliertes Trimethylolpropantri(meth)acrylat und Cyclohexandimethanoldi(meth)acrylat.

**6.** Dentale, lichthärtbare, einkomponentige Kompositzusammensetzung nach einem der Ansprüche 1 - 5,

wobei die Monomere (A) bestehen aus

(A4) 10 bis 60 Gew.-%, vorzugsweise 20 bis 50 Gew.-%, besonders bevorzugt 25 bis 40 Gew.-% eines oder mehrerer Verbindungen ausgewählt aus der Gruppe bestehend aus

- lichthärtbare bi- oder tricyclische Verbindungen $Q(Y_xZ_e)_b$, wobei gilt Q bedeutet ein gesättigtes oder olefinisch ungesättigtes bi- oder tricyclisches Strukturelement,

jeder Index b ist eine natürliche Zahl, ausgewählt aus der Gruppe der natürlichen Zahlen 1, 2, und 3,
jedes Z bedeutet eine lichthärtbare Gruppe,
jeder Index e ist eine natürliche Zahl, ausgewählt aus der Gruppe der natürlichen Zahlen 1, 2 und 3,
jedes Y bedeutet in der Struktur $Q(Y_xZ_e)_b$ bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und
jeder Indexx ist 0 oder 1,

- 7,7,9-Trimethyl-3,14-dioxa-4,13-dioxo-5,12-diazahexadecan-1,16-dioxydi(meth)acrylat (UDMA),

- alkoxylierte Bisphenol - A - di(meth)acrylate mit 2 bis 6 Alkoxyeinheiten,
- hydroxylgruppenhaltige Poly(meth)acrylate, ausgewählt aus der Gruppe bestehend aus Dipentaerythritoldi(meth)acrylat, alkoxyliertes Dipentaerythritoldi(meth)acrylat, Dipentaerythritoltri(meth)acrylat, alkoxyliertes Dipentaerythritoltri(meth)acrylat, Dipentaerythritoltetra(meth)acrylat, alkoxyliertes Dipentaerythritoltetra(meth)acrylat, Dipentaerythritolpenta(meth)acrylat, alkoxyliertes Dipentaerythritolpenta(meth)acrylat, Pentaerythritol-di(meth)acrylat, alkoxyliertes Pentaerythritoldi(meth)acrylat, Pentaerythritol-tri(meth)acrylat, alkoxyliertes Pentaerythritoltri(meth)acrylat,
und
- lichthärtbare, kettenförmige und/oder ringförmige und/oder käfigförmige Polysiloxane,

(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI), (A5) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A4) oder (A2) zuzuordnen sind,
wobei die Gew.-%-Angaben von (A4), (A2) und (A5) auf die Gesamtmasse der Monomere (A) bezogen sind und/oder

wobei die Monomere (A) bestehen aus

(A4) 10 bis 60 Gew.-%, vorzugsweise 20 bis 50 Gew.-%, besonders bevorzugt 25 bis 40 Gew.-% eines oder mehrerer Verbindungen ausgewählt aus der Gruppe bestehend aus

- lichthärtbare bi- oder tricyclische Verbindungen $Q(Y_xZ_e)_b$, wobei gilt Q bedeutet ein gesättigtes oder olefinisch ungesättigtes bi- oder tricyclisches Strukturelement,

jeder Index b ist eine natürliche Zahl, ausgewählt aus der Gruppe der natürlichen Zahlen 1, 2, und 3,
jedes Z bedeutet eine lichthärtbare Gruppe,
jeder Index e ist eine natürliche Zahl, ausgewählt aus der Gruppe der natürlichen Zahlen 1, 2 und 3,
jedes Y bedeutet in der Struktur $Q(Y_xZ_e)_b$ bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und
jeder Indexx ist 0 oder 1,

- 7,7,9-Trimethyl-3,14-dioxa-4,13-dioxo-5,12-diazahexadecan-1,16-dioxydi(meth)acrylat (UDMA),
- 7,9,9-Trimethyl-3,14-dioxa-4,13-dioxo-5,12-diazahexadecan-1,16-dioxydi(meth)acrylat
- 7,9-Dimethyl-3,14-dioxa-4,13-dioxo-5,12-diazahexadecan-1,16-dioxydi(meth)acrylat
- 3,14-Dioxa-4,13-dioxo-5,12-diazahexadecan-1,16-dioxydi(meth)acrylat
- 1,5,5-Trimethyl-1-[(2-methacryloyloxyethyl)carbamoylmethyl]-3-(2-methacryloyloxyethyl)carbamoylcyclohexan
- 7,7,9,9-Tetramethyl-3,14-dioxa-4,13-dioxo-5,12-diazahexadecan-1,16-dioxydi(meth)acrylat
- 2,7,7,9,15-Pentamethyl-3,14-dioxa-4,13-dioxo-5,12-diazahexadecan-1,16-dioxydi(meth)acrylat
- 2,7,9,9,15-Pentamethyl-3,14-dioxa-4,13-dioxo-5,12-diazahexadecan-1,16-dioxydi(meth)acrylat
- 2,7,9,15-Tetramethyl-3,14-dioxa-4,13-dioxo-5,12-diazahexadecan-1,16-dioxydi(meth)acrylat
- 2,15-Dimethyl-3,14-dioxa-4,13-dioxo-5,12-diazahexadecan-1,16-dioxydi(meth)acrylat
- 1,5,5-Trimethyl-1-[(1-methacryloyloxypropan-2-yl)carbamoylmethyl]-3-(1-methacryloyloxypropan-2-yl)carbamoylcyclohexan
- 2,7,7,9,9,15-Hexamethyl-3,14-dioxa-4,13-dioxo-5,12-diazahexadecan-1,16-dioxydi(meth)acrylat,
- alkoxylierte Bisphenol - A - di(meth)acrylate mit 2 bis 6 Alkoxyeinheiten,
- hydroxylgruppenhaltige Poly(meth)acrylate, ausgewählt aus der Gruppe bestehend aus Dipentaerythritoldi(meth)acrylat, alkoxyliertes Dipentaerythritoldi(meth)acrylat, Dipentaerythritoltri(meth)acrylat, alkoxyliertes Dipentaerythritoltri(meth)acrylat, Dipentaerythritoltetra(meth)acrylat, alkoxyliertes Dipentaerythritoltetra(meth)acrylat, Dipentaerythritolpenta(meth)acrylat, alkoxyliertes Dipentaerythritolpenta(meth)acrylat, Pentaerythritoldi(meth)acrylat, alkoxyliertes Pentaerythritoldi(meth)acrylat, Pentaerythritoltri(meth)acrylat, alkoxyliertes Pentaerythritoltri(meth)acrylat,
und
- lichthärtbare, kettenförmige und/oder ringförmige und/oder käfigförmige Polysiloxane,

(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI), (A5) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A4) oder (A2) zuzuordnen sind,
wobei die Gew.-%-Angaben von (A4), (A2) und (A5) auf die Gesamtmasse der Monomere (A) bezogen sind.

**7.** Dentale, lichthärtbare, einkomponentige Kompositzusammensetzung nach einem der Ansprüche 1 bis 6, wobei die Komponente A mehr als 60 Gew.-% lichthärtbaren, kettenförmigen und/oder ringförmigen und/oder käfigförmigen Polysiloxanen umfaßt.

**8.** Dentale, lichthärtbare, einkomponentige Kompositzusammensetzung nach einem der vorangehenden Ansprüche, wobei

die Füllstoffe (B) bestehen aus und/oder herstellbar sind durch Vermischen von (B1) 2 bis 25 Gew.-%, vorzugsweise 3 bis 20 Gew.-%, anorganischem Füllstoff mit einem $D_{50}$-Wert von 1 nm bis 200 nm,
und weiteren Füllstoffbestandteilen, vorzugsweise
(B2) 40 bis 90 Gew.-%, vorzugsweise 50 bis 80 Gew.-%, anorganischem Füllstoff mit einem $D_{50}$-Wert von größer 1 µm bis 10 µm,
(B3) 8 bis 50 Gew.-%, vorzugsweise 15 bis 40 Gew.-%, anorganischem Füllstoff in einer Größe mit einem $D_{50}$-Wert von 0,4 µm bis 1,0 µm und
(B4) 0 bis 25 Gew.-%, vorzugsweise 0 bis 15 Gew.-%, weitere Füllstoffe, die nicht (B1), (B2) oder (B3) zuzuordnen sind,
wobei die Gew.-%-Angaben von (B1), (B2), (B3) und (B4) auf die Gesamtmasse der Füllstoffe (B) bezogen sind,
wobei vorzugsweise

die Partikel des anorganischen Füllstoffs (B1) nicht aggregiert und nicht agglomeriert sind und/oder
der anorganische Füllstoff (B1) in einer Größe mit einem $D_{50}$-Wert unter 100 nm, vorzugsweise unter 70 nm vorliegt und/oder
der anorganische Füllstoff (B2) in einer Größe mit einem $D_{50}$-Wert von 1,2 µm bis 5,0 µm, vorzugsweise von 1,5 µm bis 4,0 µm vorliegt
und/oder
der anorganische Füllstoff (B3) in einer Größe mit einem $D_{50}$-Wert von 0,5 µm bis 0,9 µm und vorzugsweise von 0,6 µm bis 0,8 vorliegt
und/oder
wobei vorzugsweise (B1)

- Oxide oder Mischoxide ausgewählt aus der Gruppe bestehend aus den Elementen Silizium, Titan, Yttrium, Barium, Zirkonium, Hafnium, Niob, Tantal, Wolfram, Wismut, Molybdän, Zinn, Zink, Ytterbium, Lanthan, Cer, Aluminium und deren Mischungen, vorzugsweise Kieselsäure und/oder
- Sulfide, Selenide und Telluride von Metallen, Mischmetallen und deren Mischungen und/oder
- Salze der Seltenen Erden, des Scandiums und des Yttriums, vorzugsweise Ytterbiumfluorid und/oder
- Salze des Bariums und des Strontiums, vorzugsweise Bariumsulfat und/oder Strontiumfluorid und/oder
- Mischfluoride zwischen Ytterbiumfluorid und Strontiumfluorid, vorzugsweise das Strontiumfluorid-dotierte Ytterbiumfluorid und/oder das Ytterbiumfluorid dotierte Strontiumfluorid

enthält
und/oder

wobei die Bestandteile (B2) und (B3) vorzugsweise umfassen:

- Materialien auf der Basis von Oxiden oder Mischoxiden aus $SiO_2$, $ZrO_2$, $TiO_2$ und/oder
- Quarz-Glaskeramik oder Glaspulver, Bariumsilikatgläser, Bariumfluorsilikatgläser, Strontiumsilikatgläser, Strontiumborsilikate, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate und/oder

- Metallsalze, vorzugsweise Bariumsulfat oder Calciumfluorid und/oder
- Ytterbiumfluorid

und/oder

wobei vorzugsweise
das Verhältnis der Gesamtmasse von (B2) zu (B3) im Bereich von 1 : 1 bis 12 : 1 liegt, vorzugsweise im Bereich von 1,5 : 1 bis 8 : 1 und/oder
wobei das Verhältnis der mittleren Korngröße von (B2) zur mittleren Korngröße von (B3) im Bereich von 1,5 : 1 bis 10 : 1 liegt, vorzugsweise im Bereich von 2 : 1 bis 5 : 1
und/oder
wobei vorzugsweise (B4)

- verstärkend wirkende Füllstoff-Materialien, vorzugsweise Glasfasern, Polyamid- oder Kohlenstofffasern

und/oder

- weitere anorganische Füllstoffe

und/oder

- Splitter- oder Perlpolymerisate, vorzugsweise Perlpolymerisate von Homo- oder Copolymeren organisch härtbarer Monomere,

umfasst.

**9.** Dentale, lichthärtbare, einkomponentige Kompositzusammensetzung nach Anspruch 8,

wobei die Komponenten (B1) und/oder (B2) und/oder (B3) und/oder (B4) organisch oberflächenmodifiziert, vorzugsweise silanisiert, sind
und/oder
wobei die Komponenten (B1) und/oder (B2) und/oder (B3) und/oder (B4) durch Verbindungen der allgemeinen Formel X-Sp-V oberflächenmodifiziert sind,
wobei "X" und "V" funktionelle Gruppen bedeuten, die durch einen Linker "Sp" miteinander verbunden sind und wobei

- die funktionelle Gruppe "X" so ausgewählt ist, dass sie unter Komplexbildung eine entsprechende Bindung mit der Oberfläche des Füllstoffpartikels eingehen kann, vorzugsweise eine Gruppe des Typs Silan, Phosphat, Phosphonat, Carboxylat, Dithiophosphat, Dithiophosphonat, Amin oder Amid ist und
- der Linker "Sp" lineare oder verzweigte Alkylketten, Aromaten oder Kombinationen dieser Gruppen aufweist, die jeweils durch Heteroatome wie O, N, oder P oder durch eine Urethangruppe unterbrochen sein können und
- die funktionelle Gruppe "V" lichthärtbare Gruppen, vorzugsweise (Meth)acrylatgruppen aufweist

und/oder

wobei die Komponenten (B1) und/oder (B2) und/oder (B3) und/oder (B4) mit einer Verbindung der Formeln (1) oder (2) silanisiert sind

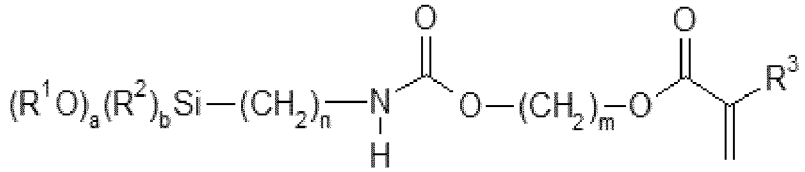

(1)

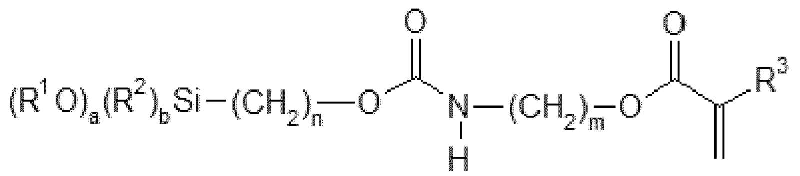

(2)

wobei $R^1$ eine C1- bis C4-Alkylgruppe bedeutet, und
$R^2$ eine C1- bis C8-Alkylgruppe bedeutet, und
$R^3$ ein Wasserstoffatom oder eine Methylgruppe bedeutet, und
a = 1, 2 oder 3, und
b = 3 - a, und
n = 1 bis 8, und
m = 1 bis 8 ist.

**10.** Gehärtete Kompositzusammensetzung, erhältlich durch Lichthärten einer dentalen, lichthärtbaren, einkomponentigen Kompositzusammensetzung nach einem der vorangehenden Ansprüche.

**11.** Dentale, lichthärtbare, einkomponentige Kompositzusammensetzung nach einem der Ansprüche 1 bis 9, zur Anwendung in einem dentalen Therapieverfahren,
vorzugsweise zur Anwendung in einem dentalen Therapieverfahren mit folgenden Schritten:

- Erwärmen der Kompositzusammensetzung auf eine Temperatur von 40 °C oder mehr, vorzugsweise eine Temperatur im Bereich von 40 °C bis 80 °C, vorzugsweise in einem Ofen und/oder durch Bestrahlung, vorzugsweise mit IR-Strahlen,
- Kontaktieren der auf eine Temperatur von 40 °C oder mehr, vorzugsweise eine Temperatur im Bereich von 40 °C bis 80 °C, erwärmten Kompositzusammensetzung mit einem zu behandelnden Zahn eines Patienten, vorzugsweise als dentales Füllungsmaterial, Unterfüllungsmaterial, Befestigungsmaterial und Fissurenversiegler.

**12.** Verwendung einer Kompositzusammensetzung nach einem der Ansprüche 1 bis 9, zur Herstellung eines dentalen Erzeugnisses, wobei die Herstellung nicht am menschlichen oder tierischen Körper erfolgt.

**13.** Vorrichtung zur Applikation einer Kompositzusammensetzung, umfassend

einen Hohlraum, der zumindest teilweise mit einer Menge einer Kompositzusammensetzung nach einem der Ansprüche 1 bis 9 gefüllt ist
und
eine mit dem Hohlraum verbundene Applikationsspitze mit einer Austrittsöffnung für die Kompositzusammensetzung,
wobei vorzugsweise

die Austrittsöffnung eine Austrittsquerschnittsfläche im Bereich von 0,2 bis 3,0 $mm^2$ besitzt, vorzugsweise eine Austrittsquerschnittsfläche von nicht mehr als 2,0 $mm^2$, besonders bevorzugt eine Austrittsquerschnittsfläche von nicht mehr als 1,5 $mm^2$, besitzt und/oder
die Applikationsspitze eine vorzugsweise aus Metall oder Kunststoff bestehende Kanüle ist
und/oder
die Vorrichtung ausgewählt ist aus der Gruppe bestehend aus Compulen und Spritzen.

**14.** Verfahren zur Herstellung einer Kompositzusammensetzung nach einem der Ansprüche 1 bis 9 oder zur Herstellung einer Vorrichtung zur Applikation einer Kompositzusammensetzung nach Anspruch 13, mit folgendem Schritt:

Vermischen der Bestandteile

(A) Monomere in einer Menge von 6 bis 35 Gew.-%, bezogen auf die Gesamtmenge der Kompositzusammensetzung, vorzugsweise 10 bis 35 Gew.-%, besonders bevorzugt 10 bis 25 Gew.-%,
(B) Füllstoffe in einer Menge von 65 bis 93 Gew.-%, vorzugsweise 65 bis 89 Gew.-%, besonders bevorzugt

75 bis 89 Gew.-%, bezogen auf die Gesamtmenge der Kompositzusammensetzung,
(C) Initiatoren in einer Menge von 0,001 bis 3 Gew.-% bezogen auf die Gesamtmenge der Kompositzusammensetzung,
(D) weitere Additive in einer Menge von 0,001 bis 5 Gew.-% bezogen auf die Gesamtmenge der Kompositzusammensetzung,

zu der Kompositzusammensetzung,
wobei die Bestandteile so ausgewählt sind, dass die Viskosität $\eta_{20}$ der Kompositzusammensetzung bei 20°C größer als 400 Pa*s ist und die Viskosität $\eta_{50}$ der Kompositzusammensetzung bei 50°C niedriger als 150 Pa*s ist und wobei der Quotient $\eta_{50}$ / $\eta_{20}$ aus der Viskosität der Kompositzusammensetzung bei 50°C und der Viskosität der Kompositzusammensetzung bei 20°C kleiner als 0,125 ist, vorzugsweise kleiner als 0,1 ist,
wobei vorzugsweise

Bestandteil (A) hergestellt wird mit den folgenden Schritten:

Bereitstellen von zumindest
(A-i) einer ersten Monomerensubstanz und
(A-ii) einer zweiten Monomerensubstanz,
wobei

- die Viskosität $\eta_{20}$ der zweiten Monomerensubstanz (A-ii) bei 20 °C größer ist als 100 Pa*s,
- die Viskosität $\eta_{20}$ der ersten Monomerensubstanz (A-i) bei 20 °C größer ist als 100 mPa*s,
- die Viskosität der zweiten Monomerensubstanz (A-ii) bei 20 °C größer ist als die der ersten Monomerensubstanz (A-i)
und
- das Massenverhältnis der ersten Monomerensubstanz (A-i) zur zweiten Monomerensubstanz (A-ii) im Bereich von 2:1 bis 1:10 liegt,

wobei die zweite Monomerensubstanz (A-ii) vorzugsweise zumindest 40 Gew.-% 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI) enthält,
wobei die Gew.-%-Angabe auf die Gesamtmasse der Monomere (A) bezogen ist
und
Mischen der bereitgestellten Monomerensubstanzen (A-i) und (A-ii), vor dem und/oder beim Mischen mit den Bestandteilen (B), (C) und (D)
und/oder wobei

Bestandteil (B) hergestellt wird durch Vermischen von

(B1) 2 bis 25 Gew.-%, vorzugsweise 3 bis 20 Gew.-%, anorganischem Füllstoff mit einem $D_{50}$-Wert von 1 nm bis 200 nm,
und weiteren Füllstoffbestandteilen, vorzugsweise (B2) 40 bis 90 Gew.-%, vorzugsweise 50 bis 80 Gew.-%, anorganischem Füllstoff mit einem $D_{50}$-Wert von größer 1 $\mu$m bis 10 $\mu$m,
(B3) 8 bis 50 Gew.-%, vorzugsweise 15 bis 40 Gew.-%, anorganischem Füllstoff in einer Größe mit einem $D_{50}$-Wert von 0,4 $\mu$m bis 1,0 $\mu$m und
(B4) 0 bis 25 Gew.-%, vorzugsweise 0 bis 15 Gew.-%, weitere Füllstoffe, die nicht (B1), (B2) oder (B3) zuzuordnen sind,

wobei die Gew.-%-Angaben von (B1), (B2), (B3) und (B4) auf die Gesamtmasse der Füllstoffe (B) bezogen sind,
vorzugsweise mit folgendem zusätzlichen Schritt zur Herstellung der Vorrichtung zur Applikation einer Kompositzusammensetzung:

- Einfüllen der hergestellten Kompositzusammensetzung nach einem der Ansprüche 1 bis 9 in einen Hohlraum einer zuvor nicht gefüllten Vorrichtung zur Applikation einer Kompositzusammensetzung.

**15.** Verfahren zum Vorbereiten einer dentalen Behandlung eines Patienten, mit folgendem Schritt:

Erwärmen einer Kompositzusammensetzung nach einem der Ansprüche 1 bis 9 oder einer Vorrichtung nach Anspruch 13, vorzugsweise hergestellt gemäß einem Verfahren nach Anspruch 14, auf eine Temperatur von 40 °C oder mehr, vorzugsweise eine Temperatur im Bereich von 40 °C bis 80 °C, vorzugsweise in einem Ofen und/oder durch Bestrahlung, vorzugsweise mit IR-Strahlen.

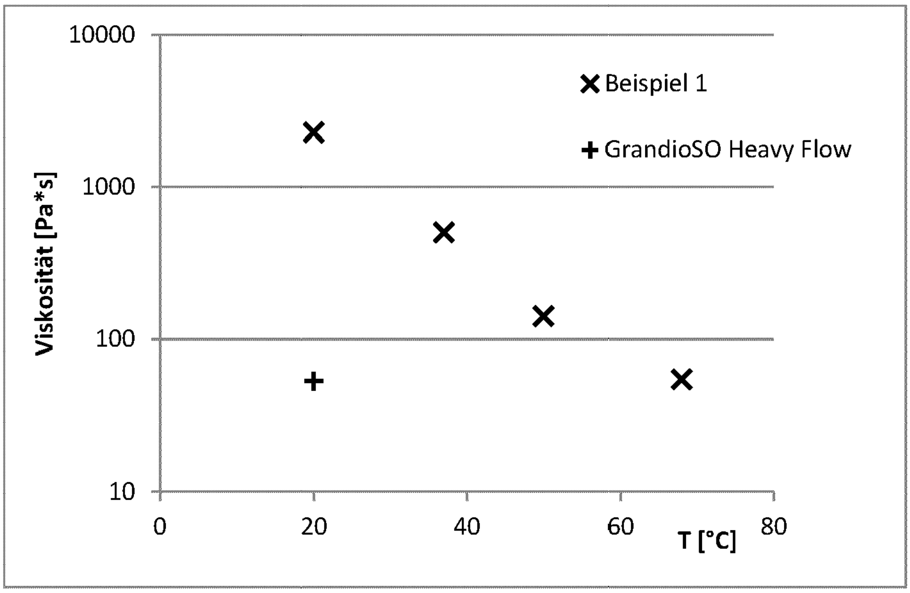
10000
1000
100
10
Viskosität [Pa*s]
0
20
40
60
80
T [°C]
Beispiel 1
GrandioSO Heavy Flow

Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6236020 B1, von J. Friedmann
- US 6320162 B1
- US 6312254 B1
- US 6616448 B2
- US 7015423 B2
- US 7097452 B2
- EP 1151728 B1
- EP 2016962 A
- EP 2016962 B
- JP 7206740 A
- EP 1112995 B1
- EP 0049631 B1
- DE 10352260 B3
- EP 0023686 B1
- US 3066112 A
- US 5319054 A
- US 5440003 A
- WO 2018071920 A1
- DE 19803979 A1
- US 3290350 A
- DE 4416857 C1
- DE 19860364 C2
- EP 1874847 B1
- EP 1685182 B1
- WO 2013041723 A1
- WO 2013053693 A1
- DE 102014210432
- WO 2005011621 A1
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- EP 1905415 A
- US 4772530 A
- US 4954414 A
- US 4874450 A
- US 5055372 A
- US 5057393 A
- EP 0783880 B1
- DE 10119831 A1
- EP 1563821 A1
- DE 2816823
- DE 2931926
- DE 2931925
- US 4131729 A
- DE 2200021
- DE 2406557
- DE 3522005
- DE 3522006
- DE 3703120
- DE 102005021332 A1
- DE 102007034457 A1
- EP 2436366 B1


### In der Beschreibung aufgeführte Nicht-Patentliteratur


- **J. FRIEDMAN.** Thermally assisted polymerization of composite resins. *Contemp. Esthet. Restor. Pract.,* 2003, vol. 7, 46
- **H.E. STRASSLER ; R.D. TRUSHKOWSKY.** Predictable restoration of Class 2 preparations with composite resin. *Dent. Today,* 2004, vol. 23, 93-99
- **J.S. BLALOCK ; R.G. HOLMES ; F.A. RUEGGEBERG.** Effect of temperature on uncured composite film thickness. *J. Prothet. Dent.,* 2006, vol. 96, 424-432
- **M. TRUJILLO ; S.M. NEWMANN ; J.W. STANSBURY.** Use of near-IR to monitor the influence of external heating on dental composite photopolymerization. *Dent. Mater.,* 2004, vol. 20, 766-777

- **U. LOHBAUER ; S. ZINELIS ; C. RAHIOTIS ; A. PETSCHELT ; G. ELIADES.** The effect of resin composite pre-heating on monomer conversion and polymerization shrinkage. *Dent. Mater.,* 2009, vol. 25, 514-519
- **F.C. CALHEIROS ; M. DARONCH ; F.A. RUEGGEBERG ; R.R. BRAGA.** Effect of temperature on composite polymerization stress and degree of conversion. *Dent. Mater.,* 2014, vol. 30, 613-618
- **N.R. FROES-SALGADO ; L.M. SILVA ; Y. KAWANO ; C. FRANCCI ; A. REIS ; A.D. LOGUERCIO.** Composite pre-heating: Effects on marginal adaptation, degree of conversion and mechanical properties. *Dent. Mater.,* 2010, vol. 26, 908-9015
- **J.S. BLALOCK et al.** Effect of temperature on unpolymerized composite resin film thickness. *J. Prosthet. Dent.,* 2006, vol. 96 (6), 424-423
- **J. DA COSTA et al.** Effect of heat on the flow of commercial composites. *Am. J. Dent.,* 2009, vol. 22 (2), 92-96
- **M. GOULART et al.** Effect of pre-heating composites on film thickness. *Journal of Research in Dentistry,* 2013, vol. 1 (4), 274-280
- **S. DEB et al.** Pre-warming of dental composites. *Dental Materials,* 2011, vol. 27, e51-e59
- **S. LUCEY et al.** Effect of pre-heating on the viscosity and microhardness of a resin composite. *Journal of Oral Rehabilitation,* 2010, vol. 37, 278-282
- *Journal of Organometallic Chemistry,* 1993, vol. 447, 153-157
- **N. MOSZNER et al.** Synthesis and polymerisation of new multifunctional urethane methacrylates. *Die Angewandte Makromolekulare Chemie,* 1999, vol. 265, 31-35
- **N. MOSZNER et al.** A partially aromatic urethane dimethacrylate as a new substitute for Bis-GMA in restorative composites. *Dental Materials,* 2008, vol. 24, 694-699
- *CHEMICAL ABSTRACTS,* 865234-08-8
- *CHEMICAL ABSTRACTS,* 34100-36-2
- *CHEMICAL ABSTRACTS,* 42405-01-6
- *CHEMICAL ABSTRACTS,* 865234-10-2
- *CHEMICAL ABSTRACTS,* 105883-40-7
- *CHEMICAL ABSTRACTS,* 52723-94-1
- *CHEMICAL ABSTRACTS,* 76701-94-5
- **VON DEBORAH HUCK-JONES ; RENATE HESSEMANN.** Chemische Identität einzelner Partikel. *Nachrichten aus der Chemie,* September 2014, vol. 62, 886-887
- **J.-P. FOUASSIER.** Photoinitiation, Photopolymerization and Photocuring. Hanser Publishers, 1995
- **J.F. RABEK.** Radiation Curing in Polymer Science and Technology. Elsevier Applied Science, 1993, vol. II